# EUROPEAN PATENT APPLICATION

(11) **EP 1 632 573 A2**
(43) Date of publication of application: **08.03.2006**
(21) Application number: 05015946.6
(22) Date of filing: 29.01.1999
(51) Int. Cl.: C12N 15/12, C12N 5/16, C07K 14/705, G01N 33/53

(54) **Modulation of sodium channels in dorsal root ganglia**

(30) Priority: 29.01.1998 US 72990 P; 20.11.1998 US 109402 P
(62) Divisional of application: 99904457.1
(71) Applicant: Yale University, New Haven, CT 06520 (US)
(72) Inventor: Dib-Hajj, Sulayman, East Lyme CT 06333 (US); Waxman, Stephen, New Haven CT 06515 (US)
(74) Representative: Thomson, Paul Anthony

(57) **Abstract**

An isolated nucleic acid molecule selected from the group consisting of a nucleic acid molecule comprising the nucleotide sequence shown in SEQ ID NO: 4 (Figure 7A), an isolated nucleic acid molecule comprising a nucleotide sequence that encodes a protein with the amino acid sequence shown in SEQ ID NO: 5 (Figure 7B), and a nucleic acid molecule that encodes a protein capable of producing a sodium current and which hybridizes to the sequence shown in SEQ ID NO: 4 (Figure 7A) under highly stringent conditions.

## Description

### FIELD OF THE INVENTION

The present invention relates to a novel tetrodotoxin resistant sodium channel and related nucleotides, as well as screening assays for identifying agents useful in treating acute or chronic pain or other hyperexcitability states. This application is related to U.S. Provisional Application 60/072,990, filed January 29, 1998, U.S. Provisional Application 60/109,402 entitled "Modulation of Sodium Channels in Dorsal Root Ganglia", filed November 20, 1998 and to U.S. Provision Application 60/109,666, entitled "Differential Role of GDNF and NGF in the Maintenance of Two TTX-Resistant Sodium Channels in Adult DRG Neurons," filed on November 20, 1998, all of which are herein incorporated by reference.

### BACKGROUND

### A. Sodium Channels

Voltage-gated sodium channels are a class of specialized protein molecules that act as molecular batteries permitting excitable cells (neurons and muscle fibers) to produce and propagate electrical impulses. Voltage-gated Na⁺ channels from rat brain are composed of three subunits, the pore-forming a subunit (260 KDa) and two auxillary subunits, β1 (36 KDa) and β2 (33 KDa) that may modulate the properties of the α-subunit; the α subunit is sufficient to form a functional channel that generates a Na current flow across the membrane [references 1,2 as cited below]. Nine distinct a subunits have been identified in vertebrates and are encoded by members of an expanding gene family [3 and references therein, 4-6] and respective orthologues of a number of them have been cloned from various mammalian species including humans. Specific a subunits are expressed in a tissue- and developmentally-specific manner [7,8]. Aberrant expression patterns or mutations of voltage-gated sodium channel α-subunits underlie a number of human and animal disorders [9-13].

Voltage-gated sodium channel α-subunits consist of four domains (D1-4) of varying internal homology but of similar predicted structure, connected by three intracellular loops (L1-3). The four domains fold to form a channel that opens to both the cytoplasm and the extracellular space via a pore. The pore opens and closes depending upon the physiological state of the cell membrane.

Each domain consists of six transmembrane segments (S1-6) that allow the protein to weave through the membrane with intra- and extracellular linkers. The linkers of S5-S6 segments of the four domains contain sequences that line the pore of the channel, and a highly conserved subset of amino acids that acts as a filter to selectively allow sodium ions to traverse the channel pore into the cytoplasm, thus generating an electric current. The amphiphatic S4 segment, in each of the four domains, rich in basic residues repeated every third amino acid, acts as a voltage sensor and undergoes a conformational change as a result of the change in the voltage difference across the cell membrane. This in turn triggers the conformational change of the protein to open its pore to the extracellular Na⁺ ion gradient.

In most of the known voltage-gated sodium channel α-subunits, the channels close and change into an inoperable state quickly (inactivate), within a few milliseconds, after opening of the pore (activation); SNS-type channels, on the other hand, inactivate slowly and require a greater voltage change to activate. L3, the loop that links domains D3 and D4, contains a tripeptide which acts as an intracellular plug that closes the pore after activation, thus inducing the channel to enter the inactive state. After inactivation, these channels further undergo conformational change to restore their resting state and become available for activation. This period is referred to as recovery from inactivation (repriming). Different channels reprime at different rates, and repriming in SNS is relatively rapid.

Based on amino acid similarities, the voltage-gated sodium channel family has been further subdivided into two subfamilies [14]. Eight of the nine cloned channels belong to subfamily 1. They share many structural features, particularly in their S4 transmembrane segments. However, some of them have been shown to have distinct kinetic properties of inactivation and repriming. Only a single channel of subfamily 2, also referred to as atypical channels, has been identified in human, rat and mouse tissues. This subfamily is primarily characterized by reduced numbers of basic residues in its S4 segments, and thus is predicted to have different voltage-dependence compared to subfamily 1. The physiological function of subfamily 2 channels is currently unknown because its electrophysiological properties have not yet been elucidated.

The blocking of voltage-gated sodium channels by tetrodotoxin, a neurotoxin, has served to functionally classify these channels into sensitive (TTX-S) and resistant (TTX-R) phenotypes. Two mammalian TTX-R channels have so far been identified, one specific to the cardiac muscle and to very limited areas of the central nervous system (CNS) and the second, SNS, is restricted to peripheral neurons (PNS) of the dorsal root ganglia (DRG) and trigeminal ganglia. Specific amino acid residues that confer resistance or sensitivity to TTX have been localized to the ion selectivity filter of the channel pore. The SNS channel is also described in International Patent Application WO 97/01577.

### B. Role of Sodium Channels in Disease States

Because different Na⁺ channel α-subunit isotypes exhibit different kinetics and voltage-dependence, the firing properties of excitable cells depend on the precise mixture of channel types that they express. Mutants of the cardiac and skeletal muscle α-subunit have been shown to cause a number of muscle disorders. Some examples are as follows: A change of a single basic amino acid residue in the S4 of the skeletal muscle channel is sufficient to change the kinetic properties of this channel and induce a disease state in many patients. A tripeptide deletion in L3 of the cardiac channel, proximal to the inactivation gate, induces a cardiac disorder called Long QT syndrome. A single amino acid change in the S5-S6 linker of domain 1 of Scn8a, the region lining the pore of the channel, causes the mouse mutant "jolting". The total loss of this channel by a different mutation causes motor end plate "med" disease in mice. This mutation is characterized by loss of motor neuron stimulation of the innervated muscle.

### C. Sodium Channels and Pain

Axonal injury (injury to nerve fibers, also called axons) can produce chronic pain (termed neuropathic pain). A number of studies have demonstrated altered excitability of the neuronal cell body and dendrites after axonal injury [15-17], and there is evidence for a change in Na⁺ channel density over the neuronal cell body and dendrites following axonal injury [18-20]. The expression of abnormal mixtures of different types of sodium channels in a neuronal cell can also lead to abnormal firing [13], and can contribute to hyperexcitability, paresthesia or pain.

Recent studies from our group on rat sensory DRG neurons have demonstrated a dramatic change in the expression profile of TTX-R and TTX-S currents and in a number of mRNA transcripts that could encode the channels responsible for these currents in DRG neurons following various insults [21-23]. We have, for example, shown an attenuation of the slowly inactivating, TTX-R current and simultaneous enhancement of the rapidly inactivating, TTX-S Na⁺ currents in identified sensory cutaneous afferent neurons following axotomy [21]. We also have shown a loss of TTX-S, slowly repriming current and TTX-R current and a gain in TTX-S, rapidly repriming current in nociceptive (pain) neurons following axotomy [22], down-regulation of SNS transcripts and a simultaneous up-regulation of α-III Transcripts [23]. Also associated with axotomy is a moderate elevation in the levels of αI and αII mRNAs [24]. These changes in the sodium channel profile appear to contribute to abnormal firing that underlies neuropathic pain that patients suffer following axonal injury.

Inflammation, which is also associated with pain (termed inflammatory pain), also causes alteration in the sodium current profile in nociceptive DRG neurons. Inflammatory modulators up-regulate TTX-R current in small C-type nociceptive DRG neurons in culture [25,26]. The rapid action of these modulators suggests that their action include posttranslational modification of existing TTX-R channels. We have now determined that inflammation also increases a TTX-R Na⁺ current and up-regulates SNS transcripts in C-type DRG neurons [58]. This data suggests that changes in the sodium current profile contribute to inflammation evoked-pain.

### D. Therapies for Chronic Pain:

A variety of classes of drugs (anticonvulsants such as phenytoin and carbamazepine; anti-arrhythmics such as mexitine; local anesthetics such as lidocaine) act on Na⁺ channels. Since the various Na⁺ channels produce sodium currents with different properties, selective blockade or activation (or other modulation) of specific channel subtypes is expected to be of significant therapeutic value. Moreover, the selective expression of certain α-subunit isoforms (PN1, SNS, NaN) in specific types of neurons provides a means for selectively altering their behavior.

Nociceptive neurons of the DRG are the major source of the PNS TTX-R Na⁺ current. Thus, the Na⁺ channels producing TTX-R currents provide a relatively specific target for the manipulation of pain-producing neurons. The molecular structure of one TTX-R channel in these DRG neurons, SNS, has been identified but, prior to our research, it has not been determined whether there are other TTX-R channels in these neurons. If such channels could be identified, they would be ideal candidates as target molecules that are preferentially expressed in nociceptive neurons, and whose modulation would attenuate pain transmission.

### SUMMARY OF THE INVENTION

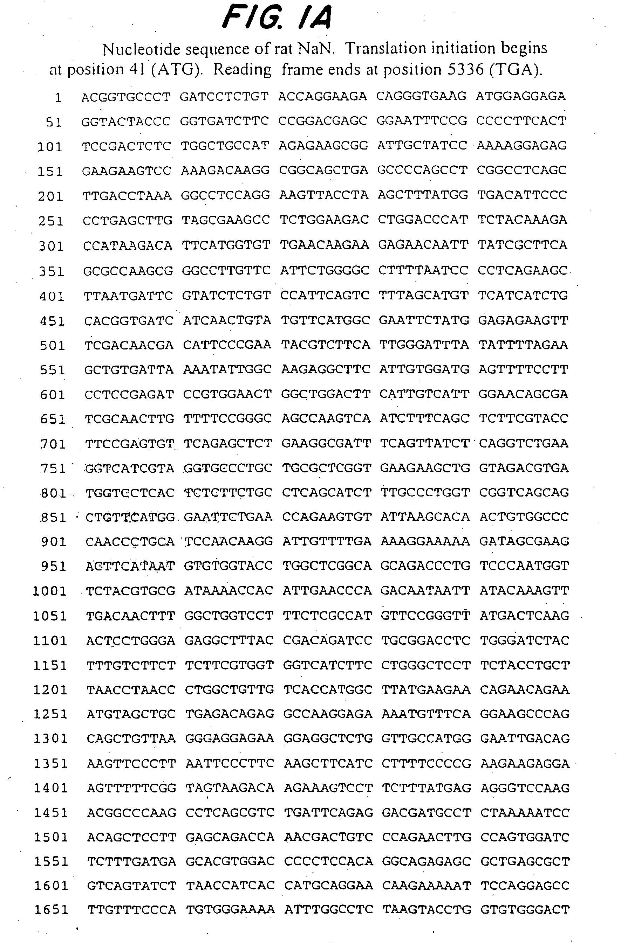

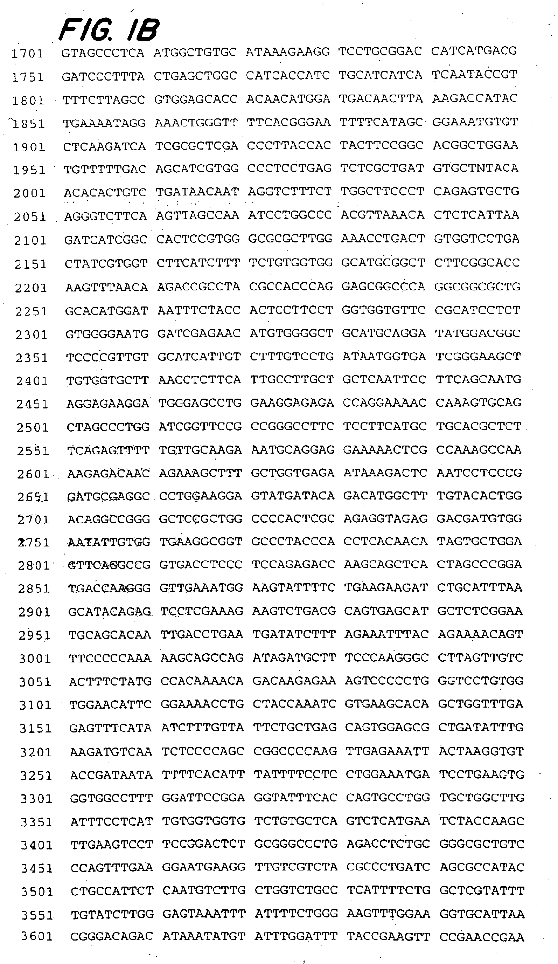

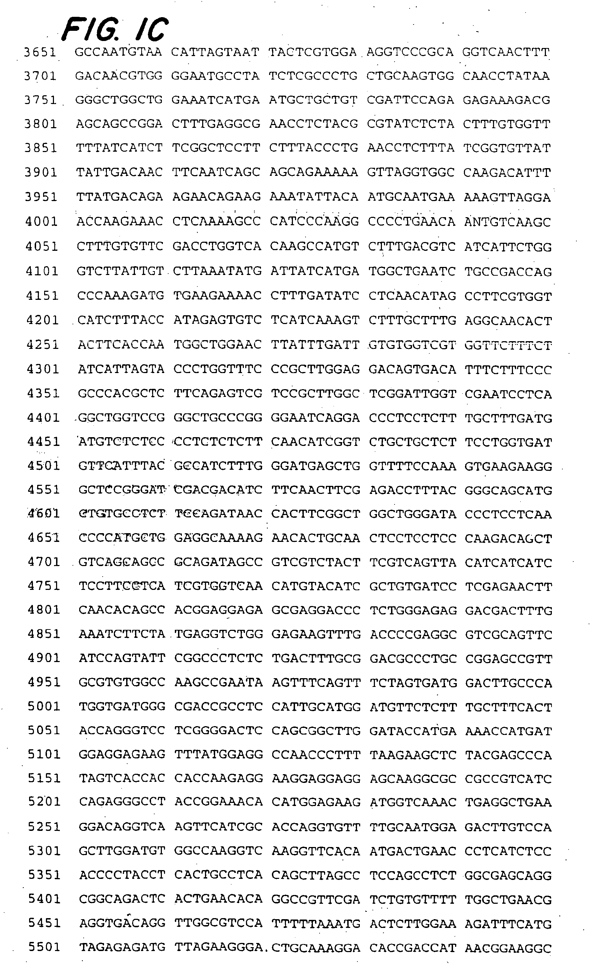

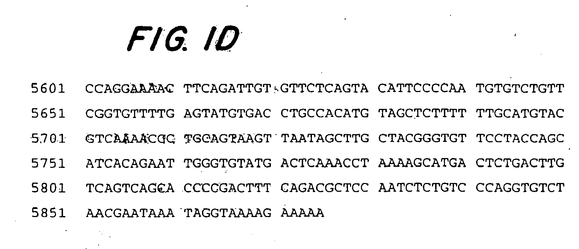

The present invention includes an isolated nucleic acid which encodes a voltage gated Na⁺ channel that is preferentially expressed in dorsal root ganglia or trigeminal ganglia (the NaN channel). (In our preceding U.S. Provisional Application 60/072,990, this NaN channel was referred to by its previous name "NaX.") In a preferred embodiment, the isolated nucleic acid comprises the sequence shown in Fig. 1, Fig. 7A, Fig. 8A, allelic variants of said sequences or nucleic acids that hybridize to the foregoing sequences under stringent conditions.

In another embodiment, the invention includes an expression vector comprising an isolated nucleic acid which encodes the voltage gated Na⁺ channel that is preferentially expressed in dorsal root ganglia or trigeminal ganglia either alone or with appropriate regulatory and expression control elements. In a preferred embodiment, the expression vector comprises an isolated nucleic acid having the sequence shown in Fig. 1, Fig. 7A, Fig. 8A, allelic variants of said sequences or nucleic acids that hybridize to the foregoing sequences under stringent conditions.

The present invention further includes a host cell transformed with an expression vector comprising an isolated nucleic acid which encodes a voltage gated Na⁺ channel that is preferentially expressed in dorsal root ganglia or trigeminal ganglia with appropriate regulatory and expression control elements. In a preferred embodiment, the expression vector comprises an isolated nucleic acid having the sequence shown in Fig. 1, Fig. 7A, Fig. 8A, allelic variants of said sequences or nucleic acids that hybridize to the foregoing sequences under stringent conditions.

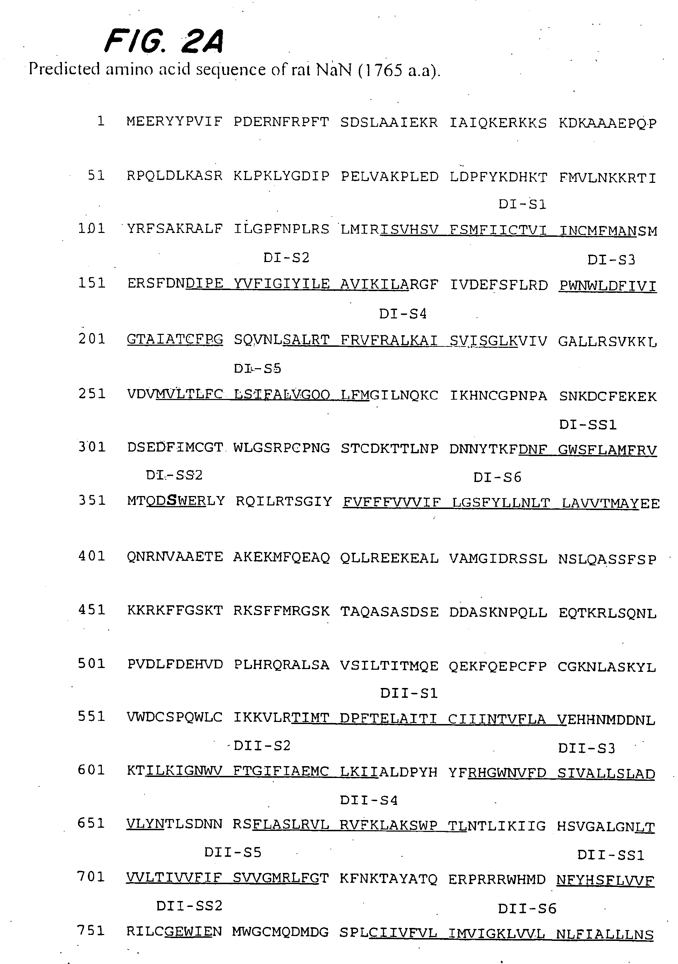

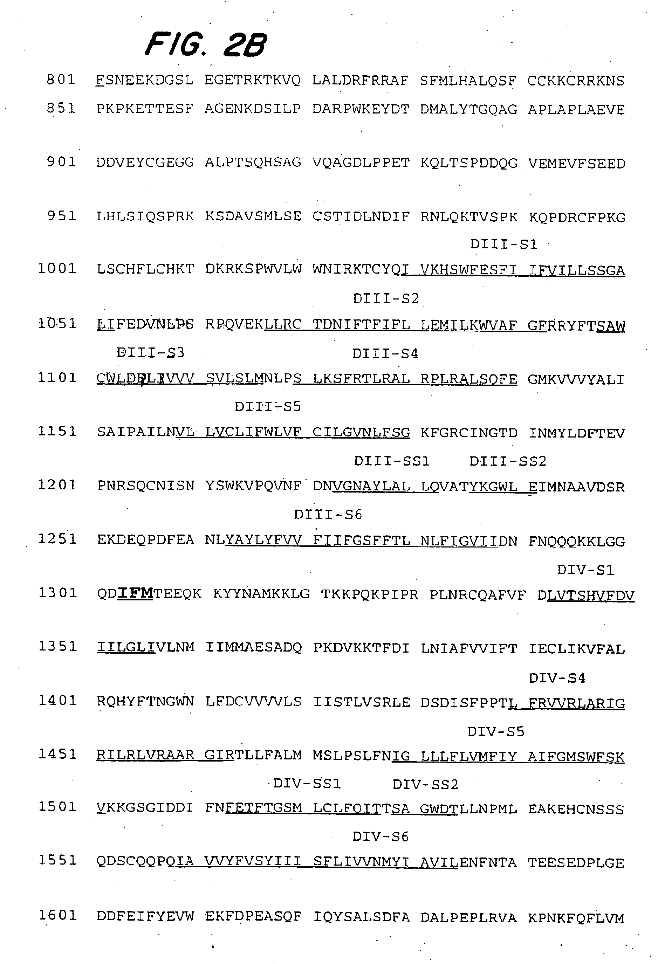

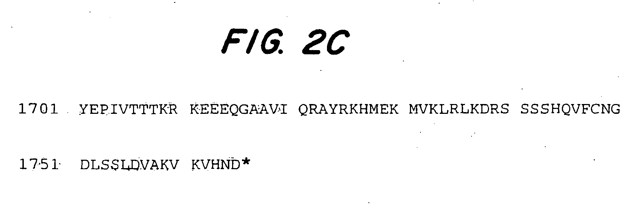

The present invention also includes an isolated voltage gated Na⁺ channel that is preferentially expressed in dorsal root ganglia or trigeminal ganglia. In a preferred embodiment, the channel has the amino acid sequence of Figs. 2, 7B or 8B or is encoded by a nucleic acid having the sequence shown in Figs. 1, 7A or 8A, allelic variants of said sequences or nucleic acids that hybridize to the foregoing sequences under stringent conditions. Peptide fragments of the channel are also included.

Another aspect of the invention is a method to identify an agent that modulates the activity of the NaN channel, comprising the steps of bringing the agent into contact with a cell that expresses the Na⁺ channel on its surface and measuring depolarization, or any resultant changes in the sodium current. The measuring step may be accomplished with voltage clamp measurements, by measuring depolarization, the level of intracellular sodium or by measuring sodium influx.

Another aspect of the invention is a method to identify an agent that modulates the transcription or translation of mRNA encoding the NaN channel. The method comprises the steps of bringing the agent into contact with a cell that expresses the Na⁺ channel on its surface and measuring the resultant level of expression of the Na⁺ channel.

The invention also includes a method to treat pain, paraesthesia and hyperexcitability phenomena in an animal or human subject by administering an effective amount of an agent capable of modulating, such as by inhibiting or enhancing, Na⁺ current flow through NaN channels in DRG or trigeminal neurons. The method may include administering an effective amount of an agent capable of modulating the transcription or translation of mRNA encoding the NaN channel.

Another aspect of the invention is an isolated nucleic acid that is antisense to the nucleic acids described above. In a preferred embodiment, the antisense nucleic acids are of sufficient length to modulate the expression of NaN channel mRNA in a cell containing the mRNA.

Another aspect of the invention is a scintigraphic method to image the loci of pain generation or provide a measure the level of pain associated with DRG or trigeminal neuron mediated hyperexcitability in an animal or human subject by administering labeled monoclonal antibodies or other labeled ligands specific for the NaN Na⁺ channel.

Another aspect of the invention is a method to identify tissues, cells and cell types that express the NaN sodium channel. This method comprises the step of detecting NaN on the cell surface, or en route to the cell surface, or the presence of NaN encoding mRNA.

The present invention further includes a method of producing a transformed cell that expresses an exogenous NaN encoding nucleic acid, comprising the step of transforming the cell with an expression vector comprising an isolated nucleic acid having the sequence shown in Figs. 1, 7A or 8A, allelic variants of said sequences or nucleic acids that hybridize to the foregoing sequences under stringent conditions, together with appropriate regulatory and expression control elements. The invention also includes a method of producing recombinant NaN protein, comprising the step of culturing the transformed host under conditions in which the NaN sodium channel or protein is expressed, and recovering the NaN protein.

The invention also includes an isolated antibody specific for the *NaN* channel or polypeptide fragment thereof. The isolated antibody may be labeled.

Another aspect of the invention includes a therapeutic composition comprising an effective amount of an agent capable of decreasing rapidly repriming sodium current flow in axotomized, inflamed or otherwise injured DRG neurons or in normal DRG neurons that are being driven to fire at high frequency. The invention also includes a method to treat acute pain or acute or chronic neuropathic or inflammatory pain and hyperexcitability phenomena in an animal or a human patient by administering the therapeutic composition.

The present invention also includes a method to screen candidate compounds for use in treating pain and hyperexcitability phenomena by testing their ability to alter the expression or activity of an NaN channel mRNA or protein in axotomized, inflamed or otherwise injured DRG neurons.

### BRIEF DESCRIPTION OF THE DRAWING FIGURES

Fig. 1 shows the sequence of the rat *NaN* cDNA.
Fig. 2 shows the putative amino acid sequence of the rat *NaN* cDNA. Predicted transmembrance segments of domains I - IV are underlined. The amino acid serine "S" in DI-SS2, implicated in the TTX-R phenotype, is in bold face type.
Fig. 3 presents a schematic diagram of predicted secondary structure of the *NaN α-*subunit.
Fig. 4 shows the results of RT-PCR analysis for *α-NaN* in extracts of various tissues using *NaN*-specific primers. *NaN* is abundantly expressed in dorsal root and trigeminal ganglia. Low levels of *NaN* are detected in cerebral hemisphere and retina tissues. No detectable *NaN* signal is seen in cerebellum, optic nerve, spinal cord, sciatic nerve, superior cervical ganglia, skeletal muscle, cardiac muscle, adrenal gland, uterus, liver and kidney.
Fig. 5 shows the tissue distribution of *α-NaN* by in *situ* hybridization. A. Trigeminal ganglion neurons show moderate-to-high hybridization signal. B. Dorsal root ganglion neurons show moderate-to-high hybridization signal in small neurons. Hybridization signal is attenuated in large neurons (arrow). C. Sense probe shows no signal in DRG neurons. D., E., and F. No hybridization signal is seen in spinal cord, cerebellum and liver. All tissues are from adult Sprague-Dawley rat. Scale bars = 50 micrometer.
Fig. 6 shows the predicted lengths of domain I amplification products of rat α-subunits and their subunit-specific restriction enzyme profile.
Figs. 7A-7B set forth the nucleotide and amino acid sequences of the murine NaN.
Fig. 8A-Fig.8B. Fig.8A is a partial nucleotide sequence of the human *NaN*. Fig. 8B is a partial amino acid sequence of the human NaN protein.

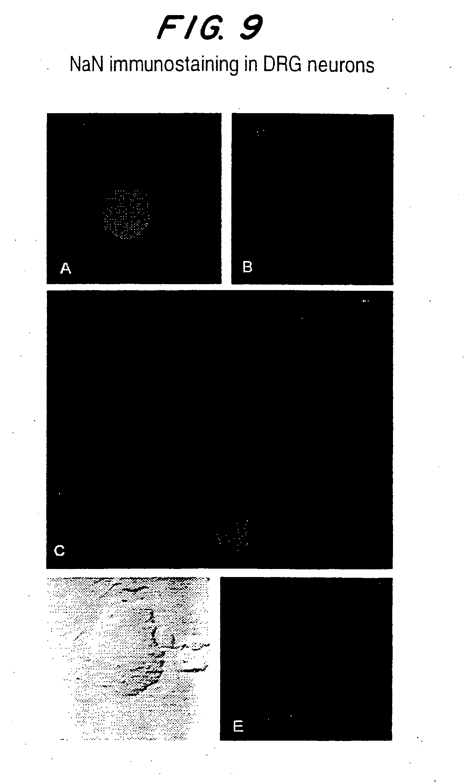

Fig. 9 shows cultures of DRG neurons obtained from L4/5 ganglia of adult rats that were reacted with antibody to NaN and then processed for immunofluorescent localization. a.,b. NaN immunostaining is prominent within the cell bodies of DRG neurons. c. NaN is present in the neuritic outgrowths, as well as the cell bodies, of DRG neurons. d., d'. Nomarski (d.) and fluorescent (d'.) images of a neuron that does not express NaN protein.

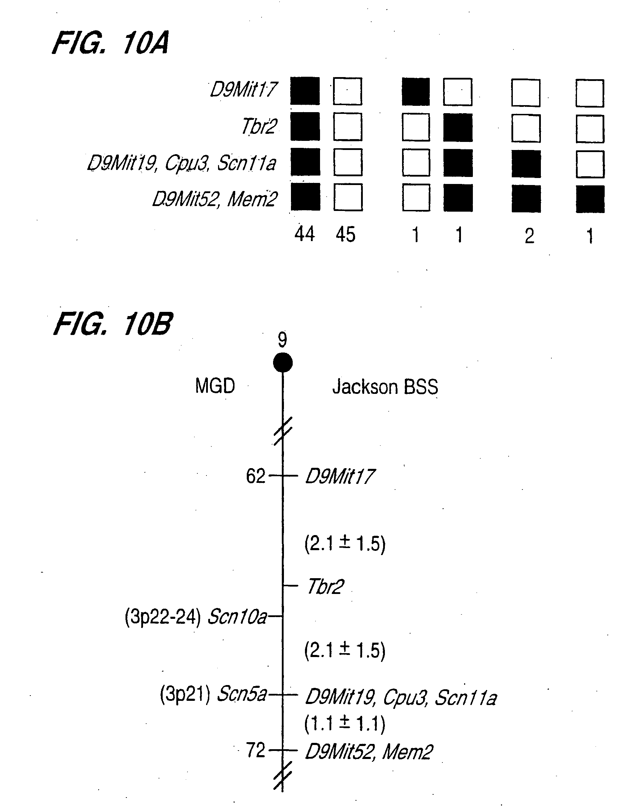

Fig. 10 shows the location of Scn1 la and related genes on distal mouse chromosome 9. (A) Haplotypes from the Jackson BSS backcross. Black boxes represent C57BL/6J alleles and white boxes represent SPRET/Ei alleles. The number of animals with each haplotype is given below each column. Missing data was inferred from adjacent data when typing was ambiguous. (B) Map of distal chromosome 9 based on data in (A). Positions of *Scn5a* and *Scn10a* from the MGD consensus map and the locations of the human orthologs are indicated. Numbers are cM positions on the consensus map (http://www.informatics.jax.org/bin/ccr/index).

### DETAILED DESCRIPTION

The present invention relates to a novel gene that we have discovered, called *NaN. NaN* encodes a previously unidentified protein, referred to herein as NaN, that belongs to the α-subunit voltage-gated sodium channel protein family and that produces a TTX-R sodium current. Such channels underlie the generation and propagation of impulses in excitable cells like neurons and muscle fibers. *NaN* is a novel sodium channel, with a sequence distinct from other, previously identified, channels. The preferential expression of *NaN* on sensory, but not other neurons, makes it a very useful target for diagnostic and/or therapeutic uses in relation to acute and/or chronic pain pathologies.

### Definitions:

This specification uses several technical terms and phrases which are intended to have the following meanings:

The phrase "modulate" or "alter" refers to up- or down-regulating the level or activity of a particular receptor, ligand or current flow. For example an agent might modulate Na⁺ current flow by inhibiting (decreasing) or enhancing. (increasing) Na⁺ current flow. Similarly, an agent might modulate the level of expression of the NaN sodium channel or the activity of the NaN channels that are expressed.

The phrase "sodium current" or "Na⁺ current" means the flow of sodium ions across a cell membrane, often through channels (specialized protein molecules) that are specifically permeable to certain ions, in this case sodium ions.

The phrase "voltage gated" means that the ion channel opens when the cell membrane is in a particular voltage range. Voltage-sensitive sodium channels open when the membrane is depolarized. They then permit Na⁺ ions to flow into the cell, producing further depolarization. This permits the cell to generate electrical impulses (also known as "action potentials").

The phrase "rapidly repriming" means that the currents recover from inactivation more rapidly than do such currents in most other voltage gated sodium channel family members.

The terms "TTX-R" and "TTX-S" means that the flow of current through a cell membrane is, respectively, resistant or sensitive to tetrodotoxin (a neurotoxin produced in certain species) at a concentration of about 100 nM.

The phrase "peripheral nervous system (PNS)" means the part of the nervous system outside of the brain and spinal cord, *i*.*e*., the spinal roots and associated ganglia such as dorsal root ganglia (DRG) and trigeminal ganglia, and the peripheral nerves.

The phrase "inhibits Na⁺ current flow" means that an agent has decreased such current flow relative to a control cell not exposed to that agent. A preferred inhibitor will selectively inhibit such current flow, without affecting the current flow of other sodium channels; or it will inhibit Na⁺ current in the channel of interest to a much larger extent than in other channels.

The phrase "enhances Na⁺ current flow" means that an agent has increased such current flow relative to a control cell not exposed to that agent. A preferred agent will selectively increase such current flow, without affecting the current flow of other sodium channels; or it will increase Na⁺ current in the channel of interest to a much larger extent than in other channels.

The phrase "specifically hybridizes" refers to nucleic acids which hybridize under highly stringent or moderately stringent conditions to the nucleic acids encoding the NaN sodium channel, such as the DNA sequence of Figs. 1, 7A or 8A.

The phrase "isolated nucleic acid" refers to nucleic acids that have been separated from or substantially purified relative to contaminant nucleic acids encoding other polypeptides. "Nucleic acids" refers to all forms of DNA and RNA, including cDNA molecules and antisense RNA molecules.

The phrase "RT-PCR" refers to the process of reverse transcription of RNA (RT) using the enzyme reverse transcriptase, followed by the amplification of certain cDNA templates using the polymerase chain reaction (PCR); PCR requires generic or gene-specific primers and thermostable DNA polymerase, for example, *Taq* DNA polymerase.

The phrase "preferentially expressed" means that voltage gated Na⁺ channel is expressed in the defined tissues in detectably greater quantities than in other tissues. For instance, a voltage gated Na⁺ channel that is preferentially expressed in dorsal root ganglia or trigeminal ganglia is found in detectably greater quantities in dorsal root ganglia or trigeminal ganglia when compared to other tissues or cell types. The quantity of the voltage gated Na⁺ channel may be detected by any available means, including the detection of specific RNA levels and detection of the channel protein with specific antibodies.

### Characterization of the NaN Sodium Channel:

The present invention relates to a previously unidentified, voltage-gated sodium channel α-subunit (NaN), predicted to be TTX-R, voltage-gated, and preferentially expressed in sensory neurons innervating the body (dorsal root ganglia or DRG) and the face (trigeminal ganglia). The predicted open reading frame (ORF), the part of the sequence coding for the NaN protein molecule, has been determined with the putative amino acid sequence from different species (rat, mouse, human) presented in Figs. 2, 7B and 8B.

All of the relevant landmark sequences of voltage-gated sodium channels are present in NaN at the predicted positions, indicating that *NaN* belongs to the sodium channel family. But NaN is distinct from all other previously identified Na channels, sharing a sequence identity of less than 53% with each one of them. *NaN* is distinct from *SNS*, the only other TTX-R Na⁺ channel subunit that has been identified, until our discovery, in PNS. We have identified and cloned *NaN* without using any primers or probes that are based upon or specific to *SNS*. Moreover, *NaN* and *SNS* share only 47% similarity of their predicted open reading frame (ORF), comparable to the limited similarity of *NaN* to all subfamily 1 members.

The low sequence similarity to existing α-subunits clearly identifies *NaN* as a novel gene, not simply a variant of an existing channel. Sequence variations compared to the other voltage-gated channels indicate that NaN may be the prototype of a novel and previously unidentified, third class of TTX-R channels that may possess distinct properties compared to *SNS.* NaN and *SNS,* which are present in nociceptive DRG and trigeminal neurons, may respond to pharmacological interventions in different ways. The preferential expression of NaN in sensory DRG and trigeminal neurons provides a target for selectively modifying the behavior of these nerve cells while not affecting other nerve cells in the brain and spinal cord. A further elucidation of the properties of NaN channels will be important to understand more fully the effects of drugs designed to modulate the function of the "TTX-R" currents which are characteristic of DRG nociceptive neurons and which contribute to the transmission of pain messages, and to abnormal firing patterns after nerve injury and in other painful conditions.

### NaN Nucleic Acids:

Nucleic acid molecules of the invention include the nucleotide sequences set forth in Fig. 1, Fig. 7A, Fig. 8A as well as nucleotide sequences that encode the amino acid sequences of Fig. 2, Fig. 7B and Fig. 8B. Nucleic acids of the claimed invention also include nucleic acids which specifically hybridize to nucleic acids comprising the nucleotide sequences set forth in Fig. 1, Fig. 7A or 8A or nucleotide sequences which encode the amino acid sequences of Fig. 2, Fig. 7B or Fig.8B. A nucleic acid which specifically hybridizes to a nucleic acid comprising that sequence remains stably bound to said nucleic acid under highly stringent or moderately stringent conditions. Stringent and moderately stringent conditions are those commonly defined and available, such as those defined by Sambrook *et al.* [59] or Ausubel *et al*. [60]. The precise level of stringency is not important, rather, conditions should be selected that provide a clear, detectable signal when specific hybridization has occurred.

Hybridization is a function of sequence identity (homology), G+C content of the sequence, buffer salt content, sequence length and duplex melt temperature (T[m]) among other variables. See, Maniatis *et al*.[62]. With similar sequence lengths, the buffer salt concentration and temperature provide useful variables for assessing sequence identity (homology) by hybridization techniques. For example, where there is at least 90 percent homology, hybridization is commonly carried out at 68° C in a buffer salt such as 6XSCC diluted from 20XSSC. See Sambrook *et al.* [59]. The buffer salt utilized for final Southern blot washes can be used at a low concentration, e.g., 0.1XSSC and at a relatively high temperature, e.g., 68° C, and two sequences will form a hybrid duplex (hybridize). Use of the above hybridization and washing conditions together are defined as conditions of high stringency or highly stringent conditions. Moderately stringent conditions can be utilized for hybridization where two sequences share at least about 80 percent homology. Here, hybridization is carried out using 6XSSC at a temperature of about 50-55° C. A final wash salt concentration of about 1-3XSSC and at a temperature of about 60-68° C are used. These hybridization and washing conditions define moderately stringent conditions.

In particular, specific hybridization occurs under conditions in which a high degree of complementarity exists between a nucleic acid comprising the sequence of an isolated sequence and another nucleic acid. With specific hybridization, complementarity will generally be at least about 70%, 75%, 80%, 85%, preferably about 90-100%, or most preferably about 95-100%.

As used herein, homology or identity is determined by **BLAST** (Basic Local Alignment Search Tool) analysis using the algorithm employed by the programs **blastp, blastn, blastx, tblastn** and **tblastx** (Karlin *et al.* Proc. Natl. Acad. Sci. USA 87: 2264-2268 (1990) and Altschul, S. F. J. Mol. Evol. 36: 290-300(1993), both of which are herein incorporated by reference) which are tailored for sequence similarity searching. The approach used by the **BLAST** program is to first consider similar segments between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (Nature Genetics 6: 119-129 (1994)) which is herein incorporated by reference. The search parameters for **histogram**, **descriptions**, **alignments**, **expect** (i.e., the statistical significance threshold for reporting matches against database sequences), **cutoff**, **matrix** and **filter** are at the default settings. The default scoring matrix used by **blastp**, **blastx**, **tblastn**, and **tblastx** is the **BLOSUM62** matrix (Henikoff *et al.* Proc. Natl. Acad. Sci. USA 89: 10915-10919 (1992), herein incorporated by reference). For **blastn**, the scoring matrix is set by the ratios of **M** (i.e., the reward score for a pair of matching residues) to **N** (i.e., the penalty score for mismatching residues), wherein the default values for **M** and **N** are 5 and -4, respectively.

The nucleic acids of the present invention can be used in a variety of ways in accordance with the present invention. For example, they can be used as nucleic acid probes to screen other cDNA and genomic DNA libraries so as to select by hybridization other DNA sequences that encode homologous *NaN* sequences. Contemplated nucleic acid probes could be RNA or DNA labeled with radioactive nucleotides or by non-radioactive methods (for example, biotin). Screening may be done at various stringencies (through manipulation of the hybridization Tm, usually using a combination of ionic strength, temperature and/or presence of formamide) to isolate close or distantly related homologs. The nucleic acids may also be used to generate primers to amplify cDNA or genomic DNA using polymerase chain reaction (PCR) techniques. The nucleic acid sequences of the present invention can also be used to identify adjacent sequences in the genome, for example, flanking sequences and regulatory elements of *NaN.* The nucleic acids may also be used to generate antisense primers or constructs that could be used to modulate the level of gene expression of NaN. The amino acid sequence may be used to design and produce antibodies specific to NaN that could be used to localize NaN to specific cells and to modulate the function of NaN channels expressed on the surface of cells.

### Vectors and Transformed Host Cells:

The present invention also comprises recombinant vectors containing and capable of replicating and directing the expression of nucleic acids encoding a NaN sodium channel in a compatible host cell. For example, the insertion of a DNA in accordance with the present invention into a vector using enzymes such as T4 DNA ligase, may be performed by any conventional means. Such an insertion is easily accomplished when both the DNA and the desired vector have been cut with the same restriction enzyme or enzymes, since complementary DNA termini are thereby produced. If this cannot be accomplished, it may be necessary to modify the cut ends that are produced by digesting back single-stranded DNA to produce blunt ends, or by achieving the same result by filling in the single-stranded termini with an appropriate DNA polymerase. In this way, blunt-end ligation may be carried out. Alternatively, any site desired may be produced by ligating nucleotide sequences (linkers) onto the DNA termini. Such linkers may comprise specific oligonucleotide sequences that encode restriction site recognition sequences.

Any available vectors and the appropriate compatible host cells may be used [59, 60]. Commercially available vectors, for instance, those available from New England Biolabs Inc., Promega Corp., Stratagene Inc. or other commercial sources are included.

The transformation of appropriate cell hosts with an rDNA (recombinant DNA) molecule of the present invention is accomplished by well known methods that typically depend on the type of vector used and host system employed. Frog oocytes can be injected with RNA and will express channels, but in general, expression in a mammalian cell line (such as HEK293 or CHO cells) is preferred. With regard to transformation of prokaryotic host cells, electroporation and salt treatment methods are typically employed, see, for example, Cohen *et al*.[61]; and [62]. With regard to transformation of vertebrate cells with vectors containing rDNAs, electroporation, cationic lipid or salt treatment methods are typically employed [63, 64].

Successfully transformed cells, *i*.*e*., cells that contain an rDNA molecule of the present invention, can be identified by well known techniques. For example, cells resulting from the introduction of an rDNA of the present invention can be cloned to produce single colonies. Cells from those colonies can be harvested, lysed and their DNA content examined for the presence of the rDNA using conventional methods [65, 66] or the proteins produced from the cell assayed via an immunological method. If tags such as green fluorescent protein are employed in the construction of the recombinant DNA, the transfected cells may also be detected *in vivo* by the fluorescence of such molecules by cell sorting.

For transient expression of recombinant channels, transformed host cells for the measurement of Na⁺ current or intracellular Na⁺ levels are typically prepared by co-transfecting constructs into cells such as HEK293 cells with a fluorescent reporter plasmid (such as pGreen Lantern-1, Life Technologies, Inc.) using the calcium-phosphate precipitation technique [27]. HEK293 cells are typically grown in high glucose DMEM (Life Technologies, Inc) supplemented with 10% fetal calf serum (Life Technologies, Inc). After 48 hrs, cells with green fluorescence are selected for recording [28].

For preparation of cell lines continuously expressing recombinant channels, the *NaN* construct is cloned into other vectors that carry a selectable marker in mammalian cells. Transfections are carried out using the calcium phosphate precipitation technique [27]. Human embryonic kidney (HEK-293), chinese hamster ovary (CHO) cells, derivatives of either or other suitable cell lines are grown under standard tissue culture conditions in Dulbeccos's modified Eagle's medium supplemented with 10% fetal bovine serum. The calcium phosphate-DNA mixture is added to the cell culture medium and left for 15-20 hr, after which time the cells are washed with fresh medium. After 48 hrs, antibiotic (G418, Geneticin, Life Technologies) is added to select for cells which have acquired neomycin resistance. After 2-3 weeks in G418, 10-20 isolated cell colonies are harvested using sterile 10ml pipette tips. Colonies are grown for another 4-7 days, split and subsequently tested for channel expression using whole-cell patch-clamp recording techniques and RT-PCR.

### Method of Measuring Na⁺Current Flow:

Na⁺ currents are measured using patch clamp methods [29], as described by Rizzo *et al*. [30] and Dib-Hajj *et al*. [28]. For these recordings data are acquired on a MacIntosh Quadra 950 or similar computer, using a program such as Pulse (v 7.52, HEKA, German). Fire polished electrodes typically (0.8-1.5 MW) are fabricated from capillary glass using a Sutter P-87 puller or a similar instrument. In the most rigorous analyses, cells are usually only considered for analysis if initial seal resistance is <5 Gohm, they have high leakage currents (holding current <0.1 nA at -80 mV), membrane blebs, and an access resistance <5 Mohm. Access resistance is usually monitored throughout the experiment and data are not used if resistance changes occur. Voltage errors are minimized using series resistance compensation and the capacitance artifact is canceled using computer controlled amplifier circuitry or other similar methods. For comparisons of the voltage dependence of activation and inactivation, cells with a maximum voltage error of ±10mV after compensation are used.. Linear leak subtraction is usually used for voltage clamp recordings. Membrane currents are typically filtered at 5 KHz and sampled at 20 KHz. The pipette solution contains a standard solution such as: 140 mM CsF, 2 mM MgCl₂, 1 mM EGTA, and 10 mM Na-HEPES (pH 7.3). The standard bathing solution is usually 140 nM NaC1, 3 mM KC1, 2 mM MgC1₂, 1 mM CaC1₂, 10 mM HEPES, and 10 mM glucose (pH 7.3).

Voltage clamp studies on transformed cells or DRG neurons, using methods such as intracellular patch-clamp recordings, can provide a quantitative measure of the sodium current density (and thus the number of sodium channels in a cell), and channel physiological properties. These techniques, which measure the currents that flow through ion channels such as sodium channels, are described in *Rizzo et al.* [21]. Alternatively, the blockage or enhancement of sodium channel function can be measured using optical imaging with sodium-sensitive dyes or with isotopically labeled Na. These methods which are described in Rose, *et al.,* (*J. Neurophysiology,* 1997 in press) [67] and by Kimelberg and Walz [31], measure the increase in intracellular concentration of sodium ions that occurs when sodium channels are open.

### Measurement of Intracellular Sodium ([Na⁺]ᵢ)

The effects of various agents on cells that express Na⁺ can be determined using ratiometric imaging of [Na⁺]ᵢ using SBFI or other similar ion-sensitive dyes. In this method, as described by Sontheimer *et al*. [32], cytosolic-free Na⁺ is measured using an indicator for Na⁺, such as SBFI (sodium-binding benzofuran isophthalate; [33]) or a similar dye. Cells are first loaded with the membrane-permeable acetoxymethyl ester form of the dye (which is dissolved in dimethyl sulfoxide (DMSO) at a stock concentration of 10 mM). Recordings are obtained on the stage of a microscope using a ratiometric imaging setup (*e.g*., from Georgia Instruments). Excitation light is provided at appropriate wavelengths (*e.g*., 340:385 nm). Excitation light is passed to the cells through a dichroic reflector (400 nm) and emitted light above 450 nm is collected. Fluorescence signals are amplified, *e.g*., by an image intensifier (GenIISyS) and collected with a CCD camera, or similar device, interfaced to a frame grabber. To account for fluorescence rundown, the fluorescence ratio 340:385 is used to assay cytosolic-free Na⁺.

For calibration of SBFI's fluorescence, cells are perfused with calibration solutions containing known Na⁺ concentrations (typically 0 and 30 mM, or 0, 30, and 50 mM [Na⁺]), and with ionophones such as gramicidin and monensin (see above) after each experiment. As reported by Rose and Ransom [34], the 345/390 nm fluorescence ratio of intracellular SBFI changes monotonically with changes in [Na⁺]ᵢ. Experiments are typically repeated on multiple (typically at least 4) different coverslips, providing statistically significant measurements of intracellular sodium in control cells, and in cells exposed to various concentrations of agents that may block, inhibit or enhance Na⁺.

### Method to Measure Na⁺ Influx via Measuring ²²Na or ⁸⁶Rb.

²²Na is a gamma emitter and can be used to measure Na⁺ flux [31], and ⁸⁶Rb⁺ can be used to measure Na⁺/K⁺-ATPase activity [32]. ⁸⁶Rb⁺ ions are taken up by the Na⁺/K⁺-ATPase-like K⁺ ions, but have the advantage of a much longer half-life than ⁴²K⁺ [35]. Thus, measurement of the unidirectional ouabain-sensitive ⁸⁶Rb⁺ uptake provides a quantitative method for assaying Na⁺/K⁺-ATPase activity which provides another indicator of the electrical firing of nerve cells. Following incubation of cells expressing *NaN* with the isotope ²²Na⁺, the cellular content of the isotope is measured by liquid scintillation counting or a similar method, and cell protein is determined using a method such as the bicinchoninic acid protein assay [36] following the modifications described by Goldschmidt and Kimelberg [37] for cultured cells. ²²Na and ⁸⁶Rb⁺ fluxes are determined in the presence and absence of agents that may block, inhibit, or enhance NaN. This permits determination of the actions of these agents on NaN.

### Method to Identify Agents that Modulate NaN-Mediated Current:

Several approaches can be used to identify agents that are able to modulate (i.e., block or augment) the Na⁺ current through the NaN sodium channel. In general, to identify such agents, a model cultured cell line that expresses the NaN sodium channel is utilized, and one or more conventional assays are used to measure Na⁺ current. Such conventional assays include, for example, patch clamp methods, the ratiometric imaging of [Na⁺]_{*i*}*,* and the use of ²²Na and ⁸⁶Rb as described above.

In one embodiment of the present invention, to evaluate the activity of a candidate compound to modulate Na⁺ current, an agent is brought into contact with a suitable transformed host cell that expresses *NaN.* After mixing or appropriate incubation time, the Na⁺ current is measured to determine if the agent inhibited or enhanced Na⁺ current flow.

Agents that inhibit or enhance Na⁺ current are thereby identified. A skilled artisan can readily employ a variety of art-recognized techniques for determining whether a particular agent modulates the Na⁺ current flow.

Because Na⁺ is preferentially expressed in pain-signaling cells, one can also design agents that block, inhibit, or enhance Na⁺ channel function by measuring the response of laboratory animals, treated with these agents, to acute or chronic pain. In one embodiment of this aspect of the invention, laboratory animals such as rats are treated with an agent for instance, an agent that blocks or inhibits (or is thought to block or inhibit) NaN. The response to various painful stimuli are then measured using tests such as the tail-flick test and limb withdrawal reflex, and are compared to untreated controls. These methods are described in Chapter 15 of Reference [38]. In another embodiment of this aspect of the invention, laboratory animals such as rats are subjected to localized injection of pain-producing inflammatory agents such as formalin [39], Freunds adjuvant [40] or carageenan, or are subjected to nerve constriction [41,42] or nerve transection [43] which produce persistent pain. The response to various normal and painful stimuli are then measured, for example, by measuring the latency to withdrawal from a warm or hot stimulus [38] so as to compare control animals and animals treated with agents that are thought to modify NaN.

The preferred inhibitors and enhancers of NaN preferably will be selective for the NaN Na⁺ channel. They may be totally specific (like tetrodotoxin, TTX, which inhibits sodium channels but does not bind to or directly effect any other channels or receptors), or relatively specific (such as lidocaine which binds to and blocks several types of ion channels, but has a predilection for sodium channels). Total specificity is not required for an inhibitor or enhancer to be efficacious. The ratio of its effect on sodium channels vs. other channels and receptors, may often determine its effect and effects on several channels, in addition to the targeted one, may be efficacious [44].

It is contemplated that modulating agents of the present invention can be, as examples, peptides, small molecules, naturally occurring and other toxins and vitamin derivatives, as well as carbohydrates. A skilled artisan can readily recognize that there is no limit as to the structural nature of the modulating agents of the present invention. Screening of libraries of molecules may reveal agents that modulate NaN or current flow through it. Similarly, naturally occurring toxins (such as those produced by certain fish, amphibians and invertebrates) can be screened. Such agents can be routinely identified by exposing a transformed host cell or other cell which expresses a sodium channel to these agents and measuring any resultant changes in Na⁺ current

### Recombinant Protein Expression, Synthesis and Purification:

Recombinant NaN proteins can be expressed, for example, in *E. coli* strains HB101, DH5a or the protease deficient strain such as CAG-456 and purified by conventional techniques.

The peptide agents of the invention can be prepared using standard solid phase (or solution phase) peptide synthesis methods, as is known in the art. In addition, the DNA encoding these peptides may be synthesized using commercially available oligonucleotide synthesis instrumentation and produced recombinantly using standard recombinant production systems. The production using solid phase peptide synthesis is necessitated if non-gene-encoded amino acids are to be included.

### Antibodies and Immunodetection:

Another class of agents of the present invention are antibodies immunoreactive with the Na⁺ channel. These antibodies may block, inhibit, or enhance the Na⁺ current flow through the channel. Antibodies can be obtained by immunization of suitable mammalian subjects with peptides, containing as antigenic regions, those portions of NaN, particularly (but not necessarily) those that are exposed extracellularly on the cell surface. Such immunological agents also can be used in competitive binding studies to identify second generation inhibitory agents. The antibodies may also be useful in imaging studies, once appropriately labeled by conventional techniques.

### Production of Transgenic Animals:

Transgenic animals containing and mutant, knock-out or modified *NaN* genes are also included in the invention. Transgenic animals wherein both *NaN* and the SNS/PN3 gene are modified, disrupted or in some form modified are also included in the present invention. Transgenic animals are genetically modified animals into which recombinant, exogenous or cloned genetic material has been experimentally transferred. Such genetic material is often referred to as a "transgene". The nucleic acid sequence of the transgene, in this case a form of *NaN*, may be integrated either at a locus of a genome where that particular nucleic acid sequence is not otherwise normally found or at the normal locus for the transgene. The transgene may consist of nucleic acid sequences derived from the genome of the same species or of a different species than the species of the target animal.

The term "germ cell line transgenic animal" refers to a transgenic animal in which the genetic alteration or genetic information was introduced into a germ line cell, thereby conferring the ability of the transgenic animal to transfer the genetic information to offspring. If such offspring in fact possess some or all of that alteration or genetic information, then they too are transgenic animals.

The alteration or genetic information may be foreign to the species of animal to which the recipient belongs, foreign only to the particular individual recipient, or may be genetic information already possessed by the recipient. In the last case, the altered or introduced gene may be expressed differently than the native gene.

Transgenic animals can be produced by a variety of different methods including transfection, electroporation, microinjection, gene targeting in embryonic stem cells and recombinant viral and retroviral infection (*see, e*.*g*., U.S. Patent No. 4,736,866; U.S. Patent No. 5,602,307; Mullins *et al.* (1993) Hypertension 22(4):630-633; Brenin *et al*. (1997) Surg. Oncol. 6(2)99-110; Tuan (ed.), *Recombinant Gene Expression Protocols,* Methods in Molecular Biology No. 62, Humana Press (1997)).

A number of recombinant or transgenic mice have been produced, including those which express an activated oncogene sequence (U.S. Patent No. 4,736,866); express simian SV 40 T-antigen (U.S. Patent No. 5,728,915); lack the expression of interferon regulatory factor 1 (IRF-1) (U.S. Patent No. 5,731,490); exhibit dopaminergic dysfunction (U.S. Patent No. 5,723,719); express at least one human gene which participates in blood pressure control (U.S. Patent No. 5,731,489); display greater similarity to the conditions existing in naturally occurring Alzheimer's disease (U.S. Patent No. 5,720,936); have a reduced capacity to mediate cellular adhesion (U.S. Patent No. 5,602,307); possess a bovine growth hormone gene (Clutter *et al.* (1996) Genetics 143(4):1753-1760); or, are capable of generating a fully human antibody response (McCarthy (1997) The Lancet 349(9049):405).

While mice and rats remain the animals of choice for most transgenic experimentation, in some instances it is preferable or even necessary to use alternative animal species. Transgenic procedures have been successfully utilized in a variety of non-murine animals, including sheep, goats, pigs, dogs, cats, monkeys, chimpanzees, hamsters, rabbits, cows and guinea pigs (*see, e.g.,* Kim *et al.* (1997) Mol. Reprod. Dev. 46(4):515-526; Houdebine (1995) Reprod. Nutr. Dev. 35(6):609-617; Petters (1994) Reprod. Fertil. Dev. 6(5):643-645; Schnieke *et al.* (1997) Science 278(5346):2130-2133; and Ainoah (1997) J. Animal Science 75(2):578-585).

The method of introduction of nucleic acid fragments into recombination competent mammalian cells can be by any method which favors co-transformation of multiple nucleic acid molecules. Detailed procedures for producing transgenic animals are readily available to one skilled in the art, including the disclosures in U.S. Patent No. 5,489,743 and U.S. Patent No. 5,602,307.

The specific examples presented below are illustrative only and are not intended to limit the scope of the invention.

### EXAMPLES

### Example 1: Cloning and Characterization of the Rat NaN Coding Sequence

### a. RNA Preparation

Dorsal root ganglia (DRG) from the lumber region (L4-L5) were dissected from adult Sprague-Dawley rats and total cellular RNA was isolated by the single step guanidinum isothiocyanate-acid phenol procedure [45]. For analytical applications, DRG tissues were dissected from a few animals at a time. The quality and relative yield of the RNA was assessed by electrophoresis in a 1% agarose gel. Because of the limited starting material (4 DRGs weigh on average 10 mg), quantifying the RNA yield was not attempted. PolyA+ RNA was purified from about 300 µg of total DRG RNA (28 animals) using the PolyATract isolation system according to the manufacturers recommendations (Promega). Half of the purified RNA was used for the preparation of Marathon cDNA (see below) without further quantification.

### b. Reverse Transcription

For analytical applications, first strand cDNA was synthesized essentially as previously described [46]. Briefly, total RNA was reverse transcribed in a 25 µl final volume using 1µM random hexamer (Boehringer Mannheim) and 500 units SuperScript II reverse transcriptase (Life Technologies) in the presence of 100 units of RNase Inhibitor (Boehringer Mannheim). The reaction buffer consisted of 50 mM Tris-HCl (pH 8.3), 75 mM KCl, 3 mM MgCl₂, 10 mM DTT and 125 µM dNTP. The reaction was allowed to proceed at 37°C for 90 min., 42°C for 30 min, then terminated by heating to 65°C for 10 min.

### c. First-Strand cDNA Synthesis

The Marathon cDNA synthesis protocol was followed according to the manufacturer's instruction as summarized below (all buffers and enzymes are purchased from the manufacturer (Clontech):

Combine the following reagents in a sterile 0.5-ml microcentrifuge tube: 1 µg (1-4 µl) PolyA⁺ RNA sample, 1 µl cDNA Synthesis Primer (10 µM) and sterile H₂O to a final volume of 5 µl. Mix contents and spin the tube briefly in a microcentrifuge. Incubate the mixture at 70°C for 2 min., then immediately quench the tube on ice for 2 min. Touch-spin the tube briefly to collect the condensation. Add the following to each reaction tube: 2 µl 5X First-Strand Buffer, 1 µl dNTP Mix (10 mM), 1 µl [α-³²P]dCTP (1 µCi/µl), 1 µl AMV Reverse Transcriptase (20 units/µl) for a 10 µl volume. The radiolabeled dCTP is optional (used to determine yield of cDNA) and is replaced by sterile H₂O if not used. Mix the contents of the tube by gently pipetting and touch-spin the tube to collect the contents at the bottom. Incubate the mixture at 42°C for 1 hr in an air incubator to reduce condensation and enhance the yield of the first strand cDNA. Place the tube on ice to terminate first-strand synthesis.

### d. Second-Strand cDNA Synthesis

Combine the following components in the reaction tube from above: 48.4 µl Sterile H₂O, 16 µl 5X Second-Strand Buffer, 1.6 µl dNTP Mix (10 mM), 4 µl 20X Second-Strand Enzyme Cocktail for an 80 µl total volume. Mix the contents thoroughly with gentle pipetting and spin the tube briefly in a microcentrifuge. Incubate the mixture at 16°C for 1.5 hr. then add 2 µl (10 units) of T4 DNA Polymerase, mix thoroughly with gentle pipetting and incubate the mixture at 16°C for 45 min. Add 4 µl of the EDTA/Glycogen mix to terminate second-strand synthesis. Extract the mixture with an equal volume of buffer-saturated (pH 7.5) phenol:chloroform:isoamyl alcohol (25:24:1). Mix the contents thoroughly by vortexing and spin the tube in a microcentrifuge at maximum speed (up to 14,000 rpm or 13000xg), 4°C for 10 min. to separate layers. Carefully transfer the top aqueous layer to a clean 0.5-ml tube. Extract the aqueous layer with 100 µl of chloroform:isoamyl alcohol (24:1), vortex, and spin the tube as before to separate the layers. Collect the top layer into a clean 0.5-ml microcentrifuge tube. Ethanol precipitate the double-stranded cDNA by adding one-half volume of 4 M Ammonium Acetate and 2.5 volumes of roomtemperature 95% ethanol. Mix thoroughly by vortexing and spin the tube immediately in a microcentrifuge at top speed, room temperature for 20 min. Remove the supernatant carefully and wash the pellet with 300 µl of 80% ethanol. Spin the tube as before for 10 min. and carefully remove the supernatant. Air dry the pellet for up to 10 min. and dissolve the cDNA in 10 µl of sterile H₂O and store at -20°C. Analyze the yield and size of cDNA by running 2 µl of the cDNA solution on a 1.2% agarose/EtBr gel with suitable DNA size markers (for example the 1 Kbp ladder, Gibco/BRL). If EtBr staining does not show a signal and [α-³²P]dCTP was included in the reaction, dry the agarose gel on a vacuum gel drying system and expose an x-ray film to the gel overnight at -70°C.

### e. Adaptor Ligation

Combine these reagents in a 0.5-ml microcentrifuge test tube, at room temperature, and in the following order: 5 µl ds cDNA, 2 µl Marathon cDNA Adaptor (10 µM), 2 µl 5X DNA Ligation Buffer, 1 µl T4 DNA Ligase (1 unit/µl) for a 10 µl final volume. Mix the contents thoroughly with gentle pipetting and spin the tube briefly in a microcentrifuge. Incubate at either: 16°C overnight; or room temperature (19-23°C) for 3-4 hr. Inactivate the ligase enzyme by heating the mixture at 70°C for 5 min. Dilute 1 µl of this reaction mixture with 250 µl of Tricine-EDTA buffer and use for RACE protocols. Store the undiluted adaptor-ligated cDNA at -20°C for future use.

### f. PCR

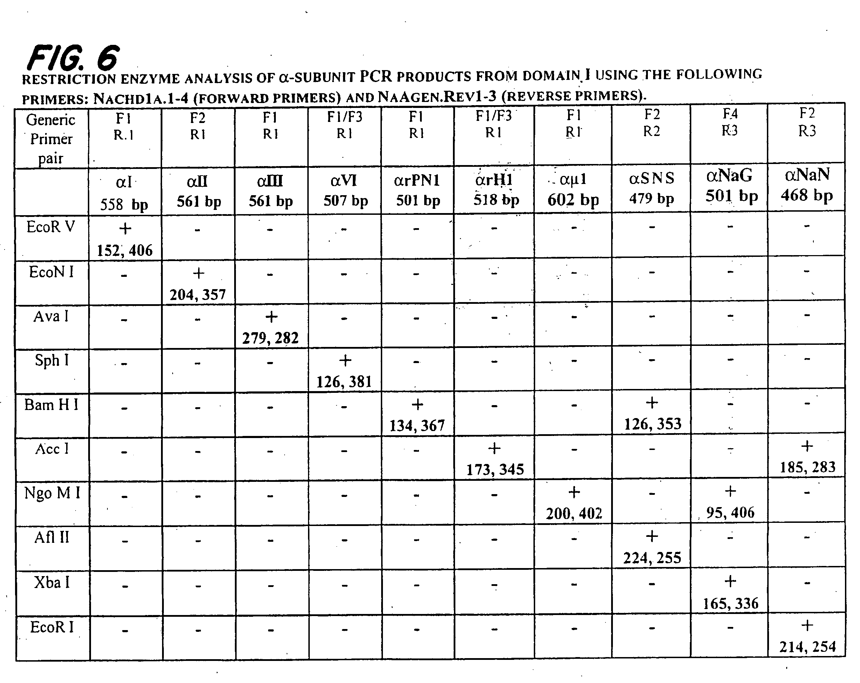

For the initial discovery of *NaN*, we used generic primers designed against highly conserved sequences in domain 1 (D1) of α-subunits I, II and III and later added more primers to accommodate the new α-subunits that were discovered. Thus, we used generic primers that recognize conserved sequences in all known Na⁺ channels. The middle of the amplified region shows significant sequence and length polymorphism (Fig. 6) and [47,48]. Due to codon degeneracy, 4 forward primers (F1-F4) and 3 reverse primers (R1-R3) were designed to ensure efficient priming from all templates that might have been present in the cDNA pool (Table 1); however, any of these primers may bind to multiple templates depending on the stringency of the reaction. Forward primer F1 matches subunits αI, αIII; aNa6; αPN1; αµ1, αrH1 and aSNS/PN3. Sequences of individual subunits show 1 or 2 mismatches to this primer: T to C at position 16 and A to G at position 18 (αNa6); C to R at position 6 (αµ1); A to G at position 18 (αrH1) and T to C at position 3 (aSNS). Forward primer F2 matches subunit αII. Forward primer F3 perfectly matches aNa6 and also matches αrH1 with a single mismatch of C to T at position 16. Reverse primer R1 matches subunits αI, αII, αIII, αNa6, αPN1, αµ1 and αrH1. This primer has mismatches compared to 4 subunits: G to A at position 3, A to G at position 4 and T to G at position 7 (aI); T to C at position 1 and A to G at position 19 (αPN1); G to A at position 3 and A to G at position 7 (αµ1); an extra G after position 3, GC to CT at positions 14-15, and A to T at position 21 (αrH1). Reverse primer R2 matches subunit αSNS/PN3.

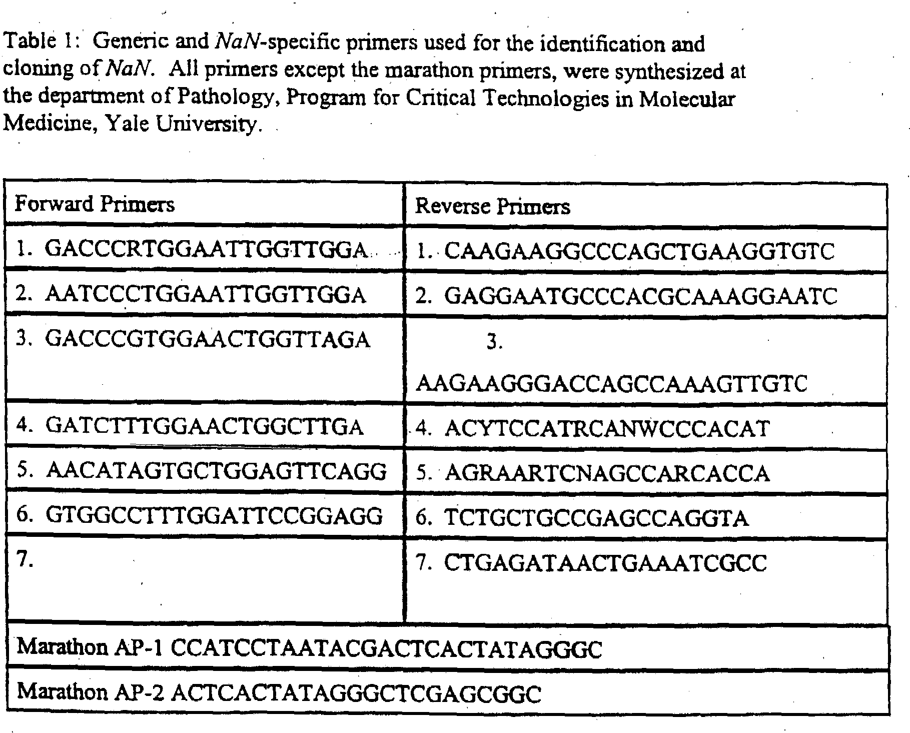

We used the respective mouse atypical sodium channel mNa. 2.3 sequence to design forward primer F4 and reverse primer R3 to amplify the analogous sequence from αNaG, the presumed rat homolog of mNa.2.3 [14]. The amplified sequence was cloned into the *Srf*I site of the vector pCR-SCRIPT (Stratagene). The nucleotide sequence of this fragment shows 88% identity to the respective sequence of mNa.2.3 (Dib-Hajj and Waxman, unpublished [68]). The restriction enzyme *Xba* I was found to be unique to this subunit. Recently, the sequence of a full length cDNA clone of putative sodium channel, NaG-like (SCL-I 1:Y09164), subunit was published [5]. The published sequence is 99% identical to our sequence and confirms the size and restriction enzyme polymorphism of the NaG PCR product.

The predicted lengths of amplified products and subunit-specific restriction enzyme recognition sites are shown in Fig. 6. All subunit sequences are based on Genbank database (accession numbers: aI: X03638; αII: X03639; αIII: Y00766; αNa6: L39018; αhNE-Na: X82835; αµl M26643; αrH1 M27902 and αSNS X92184; mNa 2.3 L36719).

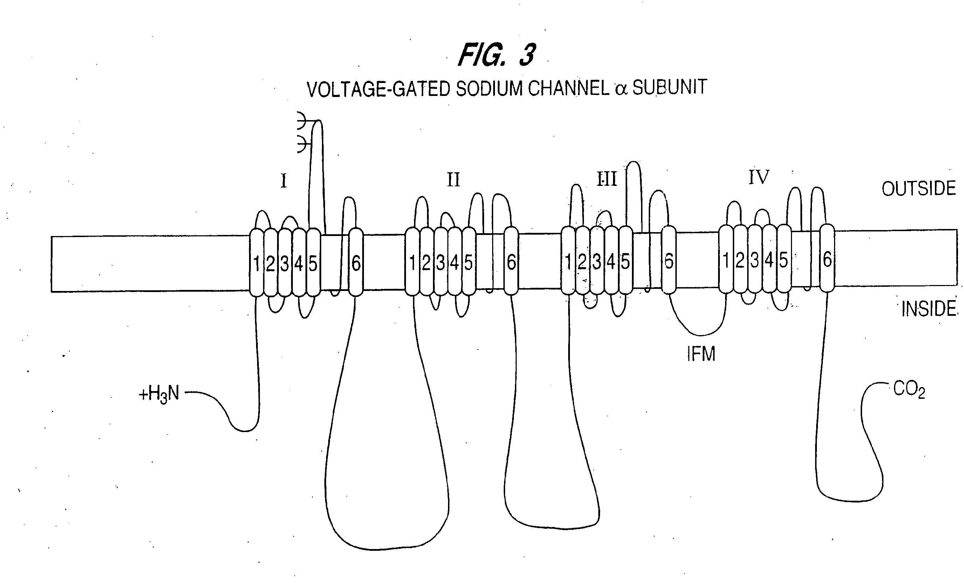

Subsequently, amplification of NaN sequences 3' terminal to the aforementioned fragment was achieved using NaN-specific primers and two generic reverse primers, R4 and R5. The sequence of the R4 primer was based on the amino acid sequence MWV/DCMEV located just N-terminal to domain II S6 segment (see schematic diagram of Fig. 3 of voltage-gated sodium channel α-subunits for reference). The sequence of the R5 primer is based on the amino acid sequence AWCWLDFL which forms the N-terminal portion of domain III S3 segment.

Amplification was typically performed in 60 µ1 volume using 1 µl of the first strand cDNA, 0.8mM of each primer and 1.75 units of Expand Long Template DNA polymerase enzyme mixture (Boehringer Mannheim). Compared to conventional and thermostable DNA polymerases, Expand Long Template enzyme mixture increases the yield of the PCR products without an increase in non-specific amplification [49,50]. The PCR reaction buffer consisted of 50 mM Tris-HCl (pH 9.2), 16 mM (NH4)₂SO₄, 2.25 mM MgCl₂, 2% (v/v) DMSO and 0.1% Tween 20. As described previously [46], amplification was carried out in two stages using a programmable thermal cycler (PTC-200, MJ Research, Cambridge, MA.). First, a denaturation step at 94°C for 4 min, an annealing step at 60°C for 2 min and an elongation step at 72°C for 90 sec. Second, a denaturation step at 94°C for 1 min, an annealing step at 60°C for 1 min and an elongation step at 72°C for 90 sec. The second stage was repeated 33 times for a total of 35 cycles, with the elongation step in the last cycle extended to 10 min.

Primary RACE amplification was performed in 50 µl final volume using 4 µl diluted DRG marathon cDNA template, 0.2 µM marathon AP-1 and NaN-specific primers, 3.5 U Expand Long Template enzyme mixture. Extension period was adjusted at 1 min/800 bp based on the expected product. 5' and 3' RACE amplification was performed using primer pairs marathon AP-1/*NaN*-specific R6 and *NaN*-specific F5/marathon AP-1, respectively. The PCR reaction buffer consisted of 50 mM Tris-HCl (pH 9.2), 16 mM (NH4)₂SO₄, 3.0 mM MgCl₂, 2% (v/v) DMSO and 0.1% Tween 20. Amplification in three stages was performed in a programmable thermal cycler (PTC-200, MJ Research, Cambridge, MA.). An initial denaturation step at 92°C was carried out for 2 min. This was followed by 35 cycles consisting of denaturation at 92°C for 20 sec, annealing step at 60°C for 1 min, and an elongation step at 68°C. Finally, an elongation step at 68°C was carried out for 5 min. Nested amplification was performed using 2 µl of a 1/500 diluted primary RACE product in a final volume of 50 µl under similar conditions to the primary RACE reactions. Primer pairs AP-2/NaN-specific R7 and NaN-specific F6/marathon AP-2 were used for nested 5' and 3' RACE, respectively. Secondary RACE products were band isolated from 1% agarose gels and purified using Qiaex gel extraction kit (Qiagen Inc.).

A schematic diagram of the putative structure of *NaN* is shown in Fig.3. The length of the intracellular loops is highly variable both in sequence and length among the various subunits. The exception is the loop between domains III and IV.

### Example 2: Determination of the Putative Rat Amino Acid Sequence for the NaN Channel

*NaN*-related clones and secondary RACE fragments were sequenced at the W. M. Keck Foundation Biotechnology Resource Lab, DNA sequencing group at Yale University. Sequence analysis including determination of the predicted amino acid sequence was performed using commercial softwares, Lasergene (DNASTAR) and GCG, Inc. The putative amino acid sequence of NaN is shown in Fig. 2. Predicted transmembrane segments of domains I - IV are underlined.

### Example 3: Determination of the Murine NaN Sequence

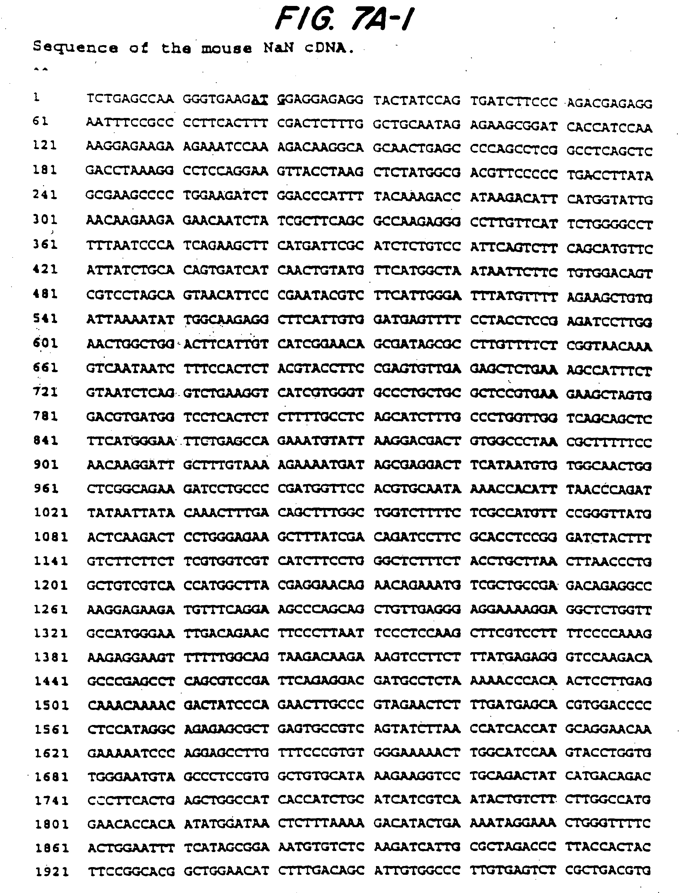

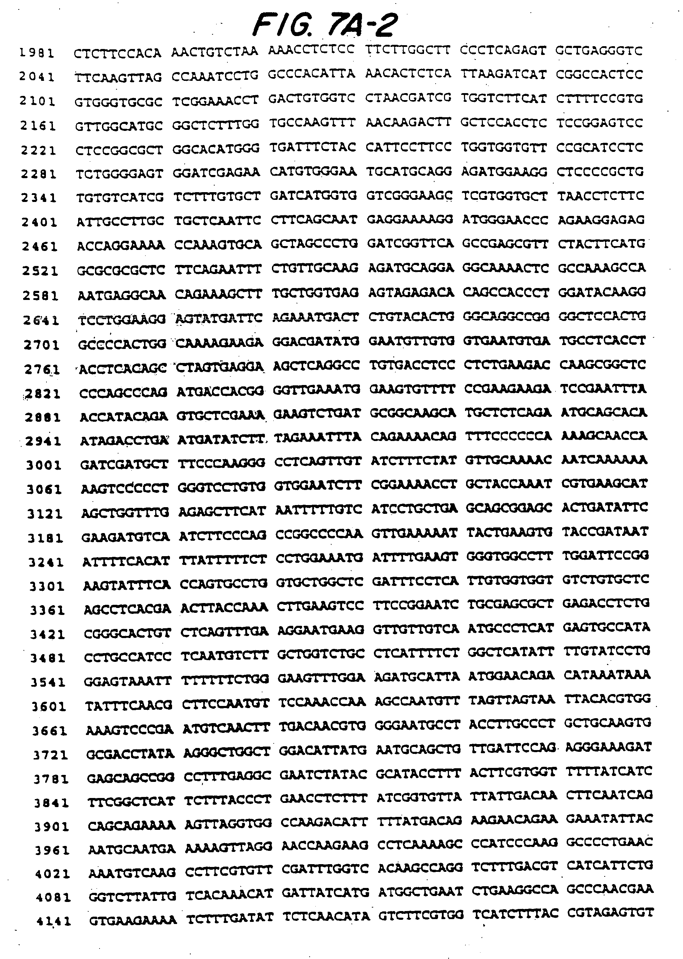

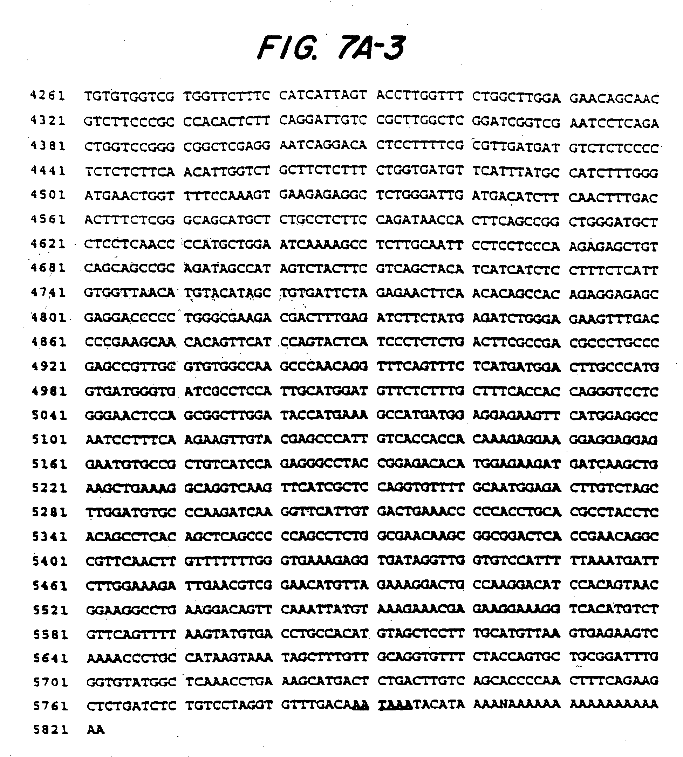

Total RNA extraction from trigeminal ganglia of mice, purification of polyA+ RNA, and Marathon cDNA construction were done as previously described for the rat. The initial amplification was performed using rat *NaN* primers. The forward primer corresponds to nucleotides 765-787 of the rat sequence (5' CCCTGCTGCGCTCGGTGAAGAAG 3'), and the reverse primer corresponds to nucleotides 1156-1137 (negative strand) of the rat sequence (5' GACAAAGTAGATCCCAGAGG 3'). The amplification produced a fragment of the expected size. The sequence of this fragment demonstrated high similarity to rat *NaN*. Other fragments were amplified using different rat primers and primers designed based on the new mouse *NaN* sequence that was being produced. Finally, longer fragments were amplified using mouse Marathon cDNA template and mouse *NaN*-specific primers in combination with adaptor primers that were introduced during the Marathon cDNA synthesis. These fragments were sequenced using primer walking and assembled into Figure 7A.

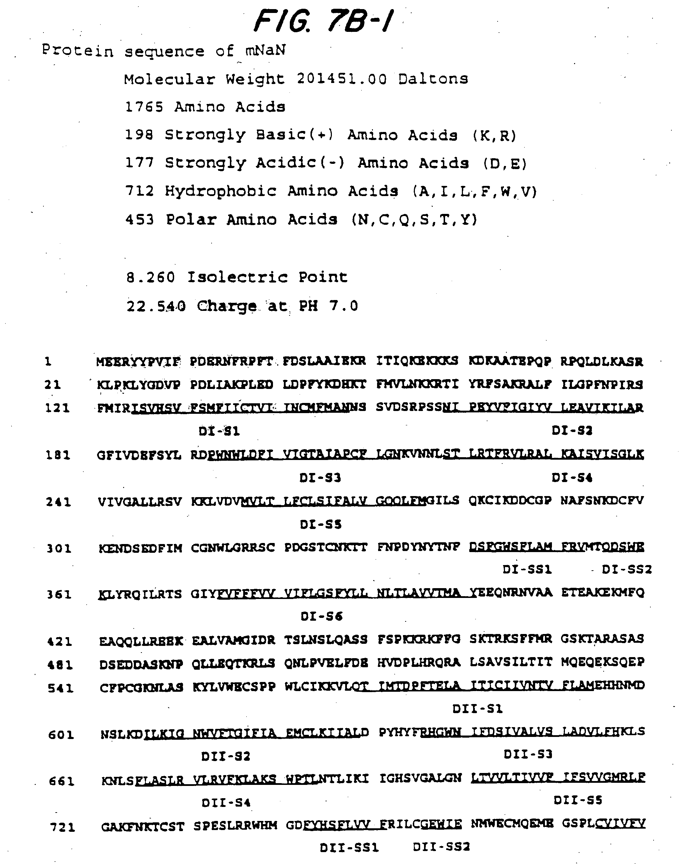

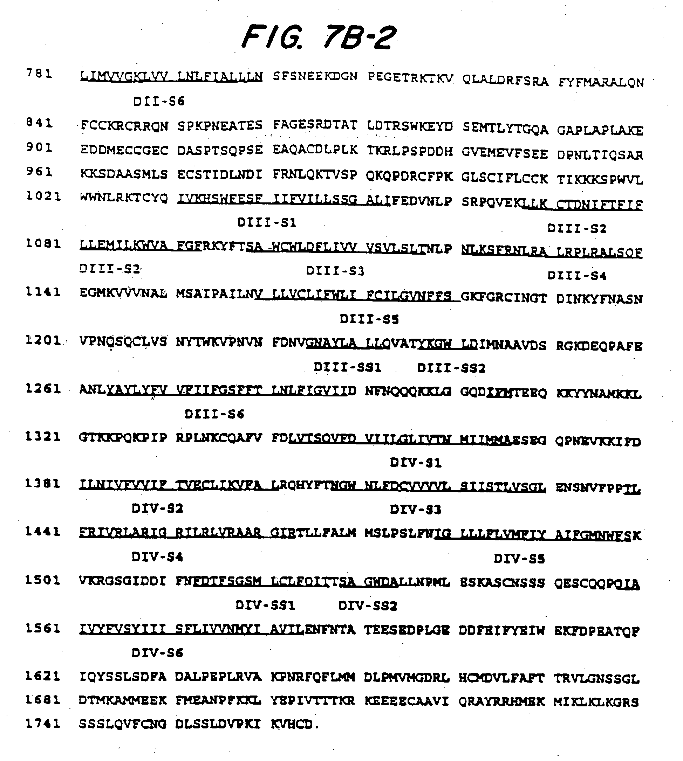

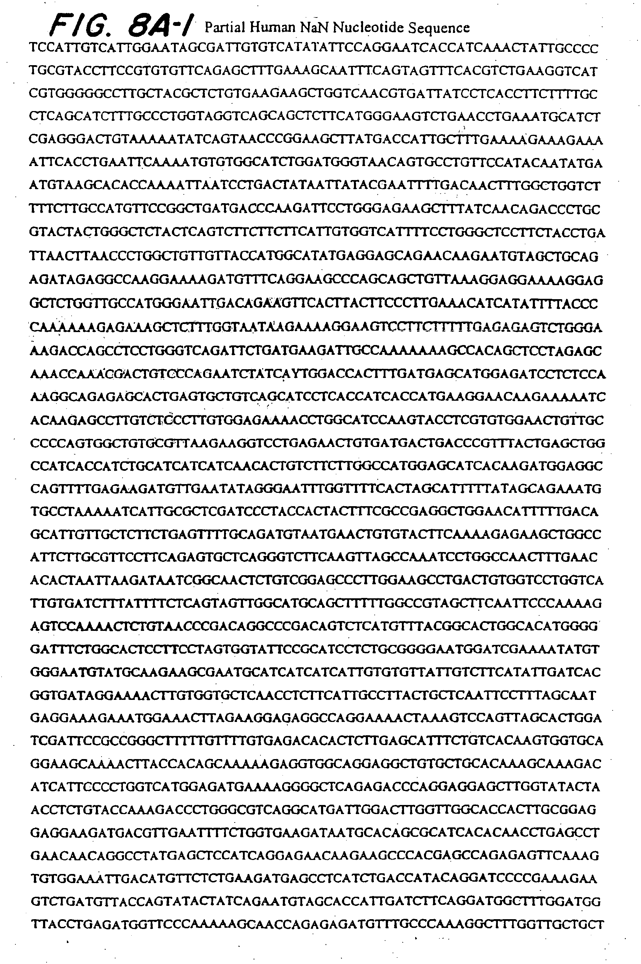

Mouse *NaN* nucleotide sequence, like rat *NaN,* lacks the out-of-frame ATG at the -8 position relative to the translation initiation codon ATG at position 41 (Fig.7A). Translation termination codon TGA is at position 5314. A polyadenylation signal (AATAAA) is present at position 5789 and a putative 23 nucleotide polyA tail is present beginning at position 5800. The sequence encodes an ORF of 1765 a.a. (Fig. 7B), which is 90% similar to rat NaN. The gene encoding NaN has been named Scn11a.

### Chromosomal localization of mouse Nan

A genetic polymorphism between strains C57BL/6J and SPRET/Ei was identified by SSCP analysis of a 274 bp fragment from the 3'UTR of *Scn11a.* Genotyping of 94 animals from the BSS backcross panel (Rowe *et al.,* 1994) demonstrated linkage of *Scn11 a* with markers on distal chromosome 9 (Fig. 10). No recombinants were observed between *Scn11a* and the microsatellite marker *D9Mit19*. Comparison of our data with the MGD consensus map of mouse chromosome 9 revealed close linkage of *Scn11a* with the two other TTX-R voltage-gated sodium channels, *Scn5a* (George *et al*., 1995; Klocke *et al*., 1992) and *Scn10a* (Kozak and Sangameswaran, 1996; Souslova *et al.,* 1997).

### Example 4: Determination of a Partial Human NaN Sequence

Human DRG tissue was obtained from a transplant donor. Total RNA extraction and cDNA synthesis were performed as described previously.

Forward primer corresponds to sequence 310-294 (minus strand) of EST AA446878. The sequence of the primer is 5' CTCAGTAGTTGGCATGC 3'. Reverse primer corresponds to sequence 270-247 (minus strand) of EST AA88521 1. The sequence of the primer is 5'GGAAAGAAGCACGACCACACAGTC 3'. Amplification was performed as previously described. PCR amplification was successful and a 2.1 Kbp fragment was obtained. This fragment was gel purified and sent for sequencing by primer walking, similar to what is done for mouse *NaN.* The sequence of the ESTs is extended in both directions; the additional sequence shows highest similarity to rat and mouse *NaN,* compared to the rest of the subunits.

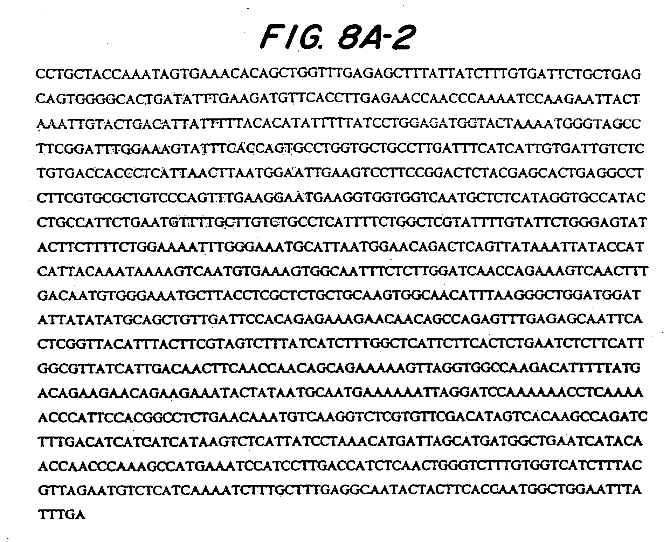

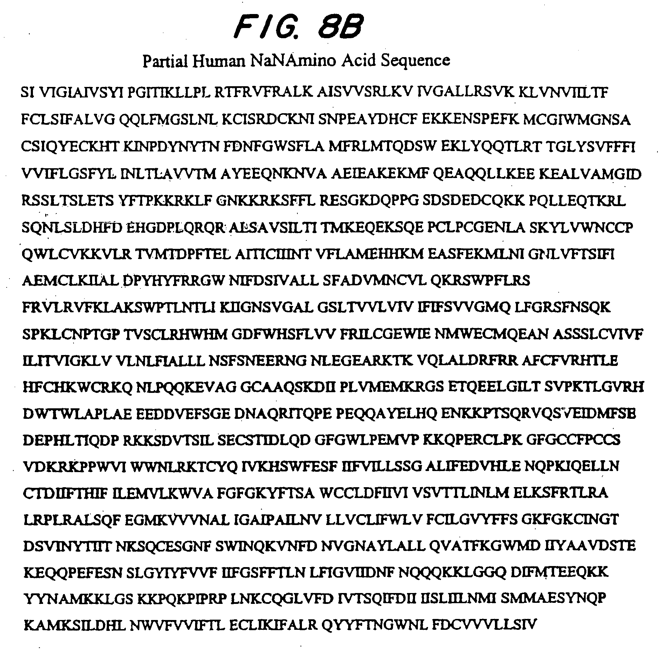

The sequence of a human 2. 1 kbp fragment was obtained using the PCR forward and reverse primers for sequencing from both ends of the fragment. Two additional primers were used to cover the rest of the sequence. The sequence was then extended in the 5' direction using forward primer 1 (above) and human NaN reverse primer (5'-GTGCCGTAAACATGAGACTGTCG3') near the 5' end of the 2.1 kb fragment. The partial amino acid sequence is set forth in Figure 8B.

The partial ORF of the human *NaN* consists 1241 amino acids. The sequence is 64% identical to the corresponding sequence of rat *NaN* (73% similar, allowing for conservative substitutions) using the advanced BLAST program at NIH. Using the Clustal method of alignment (Lasergene software, DNAStar, Inc.) the human NaN is 68% and 69% similar to mouse and rat NaN, respectively. The respective mouse and rat sequences are 88% similar.

### Example 5: Isolation of an Alternative Splicing Variant of Rat NaN

A rat NaN cDNA that encodes a C-terminal truncated version of the full-length rat NaN in Figures 1 and 2 was isolated by sequencing the insert of a rat cDNA clone. The variant NaN cDNA encodes an NaN protein lacking the 387 C-terminal amino acids of the full length NaN and containing a novel 94 amino acid stretch at the C-terminal end. The new sequence arises from the use of a cryptic donor splice site in exon 23 and a novel exon 23', which is located in intron 23. The novel C terminal amino acids are: AAGQAMRKQG DILGPNIHQF SQSSETPFLG CPQQRTCVSF VRPQRVLRVP WFPAWRTVTF LSRPRSSESS AWLGLVESSG WSGLPGESGP SSLL. The N-terminal amino acids of the truncated variant are identical to amino acids 1-1378 of the full length rat NaN of Figure 2. The alternative exon and the splicing pattern was confirmed by comparing the cDNA sequence and the genomic sequence in the respective region.

### Example 6: Methods to Isolate Other NaN Sequences

### a. Isolation of NaN sequences from genomic DNA

The genomic structure of 3 voltage-gated Na⁺ channel α-subunits have already been determined [51-54]. These genes bear remarkable similarity in their organization and provide a predictable map of most of the exon/intron boundaries. Based on the available rat, mouse and human cDNA sequence of *NaN*, disclosed herein, PCR primers are designed to amplify *NaN* homologous sequences from other species using standard PCR protocols.

Alternatively, commercially available genomic DNA libraries are screened with *NaN*-specific probes (based on the rat, mouse, or more preferably, the human sequence) using standard library screening procedures [59, 60]. This strategy yields genomic DNA isolates that can then be sequenced and the exon/intron boundaries determined by homology to the rat, mouse or human cDNA sequence.

### b. Isolation of full length NaN sequences from human autopsy or biopsy/surgical tissues

### b.1. Isolation of human ganglia total RNA

A full length *NaN* human cDNA homologue is isolated from human dorsal root ganglia or trigeminal ganglia or other cranial ganglia from post-mortem human material, foetuses or biopsy or surgical tissues. Total ribonucleic acid (RNA) is isolated from these tissues by extraction in guanidinium isothiocyanate [69] as described in Example 1.

### b.2 Determination of the full length transcript size of the human homologue of the rat NaN sodium channel cDNA.

The method of determining transcript size is as described in Example 9.

### Example 7: Production of human DRG cDNA library

A cDNA library from human DRG or trigeminal ganglia polyA+ RNA was prepared in Example 4 using standard molecular biology techniques [59, 60].

PolyA+ mRNA is hybridized to an oligo(dT) primer and the RNA is copied by reverse transcriptase into single strand cDNA. Then, the RNA in the RNA-DNA hybrid is fragmented by RNase H as *E*. *coli* DNA polymerase I synthesizes the second-strand fragment. The ends of the double stranded cDNA are repaired, linkers carrying specific restriction enzyme site (for example, Eco RI) are ligated to the ends using *E. coli* DNA ligase. The pool of the cDNA insert is then ligated into one of a variety of bacteriophage vectors that are commercially available like Lambda-Zap (Stratagene). The procedures are summarized in more detail as follows:

### a. First strand cDNA Synthesis

Dissolve 10 µg poly(A) + RNA at a concentration of 1 µg/µl in sterile water. Heat the RNA for 2-5 min. at 65°C-70°C, then quench immediately on ice. In a separate tube add in the following order (180 µl total) : 20 µ1 5 mM dNTPs (500 uM final each), 40 µl 5x RT buffer (1x final), 10 µl 200 mM DTT (10 mM final), 20 µl 0.5 mg/ml oligo (dT)12-18 (50 µg/ml final), 60 µl H₂O, 10 µl (10 U) RNasin (50 U/ml final). Mix by vortexing, briefly microcentrifuge, and add the mixture to the tube containing the RNA. Add 20 µl (200 U) AMV or MMLV reverse transcriptase for a final concentration of 1000 U/ml in 200 µl. Mix by pipetting up and down several times and remove 10 µl to a separate tube containing 1 µl of α³²P dCTP. Typically, incubate both tubes at room temperature for 5 min., then place both tubes at 42°C for 1.5 hr. This radiolabeled aliquot is removed to determine incorporation and permit an estimation of recovery; this reaction is stopped by adding 1 µl of 0.5 M EDTA, pH 8.0, and stored frozen at -20°C. The radiolabeled reaction will be used later to estimate the yield and average size of the cDNA inserts. The main reaction is stopped by adding 4 µl of 0.5 M EDTA, pH 8.0, and 200 µl buffered phenol. The mixture is vortexed well, microcentrifuged at room temperature for 1 min. to separate phases, and the upper aqueous layer is transferred to a fresh tube. Back extract the phenol layer with 1X TE buffer (10 mM Tris, 1 mM EDTA, pH 7.5) and pool the aqueous layers from the two extractions. This back extraction of the phenol layer improves the yield. The cDNA is ethanol precipitated using 7.5 M ammonium acetate (final concentration 2.0 to 2.5 M) and 95% ethanol. Place in dry ice/ethanol bath 15 min., warm to 4°C, and microcentrifuge at 10 min. at full speed, 4°C, to pellet nucleic acids. The small, yellow-white pellet is then washed with ice-cold 70% ethanol, and microcentrifuged for 3 min. at full speed, 4°C. Again, remove the supernatant, then briefly dry the pellet.

### b. Second strand synthesis

Typically, the pellet from the first-strand synthesis is resuspended in 284 µl water and these reagents are added in the following order (400 µl total): 4 µl 5 mM dNTPs (50 uM final each), 80 µl 5x second-strand buffer (1x final), 12 µl 5 mM β-NAD (150 uM final), 2 µl 10 uCi/µl α-³²P dCTP (50 uCi/ml final). Mix by vortexing, briefly microcentrifuge, and add: 4 µl (4 U) RNase H (10 U/ml final), 4 µl (20 U) *E*. c*oli* DNA ligase (50 U/ml final), and 10 µl (100 U) *E. coli* DNA polymerase I (250 U/ml final). Mix by pipetting up and down, briefly microcentrifuge, and incubate 12 to 16 hr at 14°C. After second-strand synthesis, remove 4 µl of the reaction to determine the yield from the incorporation of the radiolabel into acid-insoluble material. Extract the second-strand synthesis reaction with 400 µl buffered phenol and back extract the phenol phase with 200 µl TE buffer, pH 7.5, as described above. The double stranded cDNA is then ethanol precipitated as described above.

To complete the second-strand synthesis the double-stranded cDNA ends are rendered blunt using a mixture of enzymes. Resuspend the pellet in 42 µl water then add these reagents in the following order (80 µl total) : 5 µl 5 mM dNTPs (310 uM final each), 16 µl 5x TA buffer (1x final), 1 µl 5 mM β-NAD (62 uM final). Mix by vortexing, microcentrifuge briefly, and add: 4 µl of 2 µg/ml RNase A (100 ng/ml final), 4 µl (4 U) RNase H (50 U/ml final), 4 µl (20 U) *E*. *coli* DNA ligase (250 U/ml final), and 4 µl (8 U) T4 DNA polymerase (140 U/ml final). Mix as above and incubate 45 min at 37°C. Add 120 µl TE buffer, pH 7.5, and 1 µl of 10 mg/ml tRNA. Extract with 200 µl buffered phenol and back extract the phenol layer with 100 µl TE buffer as described above. Pool the two aqueous layers and ethanol precipitate as described above.

### c. Addition of linkers to double stranded cDNA

Combine these reagents in a 0.5-ml microcentrifuge test tube, at room temperature, and in the following order: 100 ng ds cDNA, 2 µl linkers/adaptors (10 µM), 2 µl 5X DNA Ligation Buffer; 1 µl T4 DNA Ligase ( unit/µl) for a 10 µl final volume. Mix the contents thoroughly with gentle pipetting and spin the tube briefly in a microcentrifuge. Incubate at either: 16°C overnight; or room temperature (19-23°C) for 3-4 hr. Inactivate the ligase enzyme by heating the mixture at 70°C for 5 min. This cDNA is typically digested by *Eco* RI to prepare the cohesive ends of the cDNA for ligation into the vector and to cleave linker concatemers. Typically this reaction consists of the 10 µl of the cDNA, 2 µl of 10X *Eco* RI buffer (depending on the company of source), 2 µl of *Eco* RI (10 units/µl) and sterile H₂O to a final volume of 20 µl. The mixture is incubated at 37°C for 2-4 hrs.

### d. Size fractionation of cDNA

Size exclusion columns are typically used to remove linker molecules and short cDNA fragments (350 bp). For example, a 1-ml Sepharose CL-4B column is prepared in a plastic column plugged with a small piece of sterilized glass wool (a 5 ml plastic pipet will work fine). The column is equilibrated with 0.1 M sodium chloride in 1x TE (10mM Tris, 1 mM EDTA, pH 7.5). The cDNA is then loaded onto the column and 200 µl fractions are collected. 2 µl aliquots of each fraction are analyzed by gel electrophoresis and autoradiography to determine the peak of cDNA elution. Typically, fractions containing the first half of the peak are pooled and purified by ethanol precipitation and resuspending in 10 µl distilled water.

### e. Cloning of cDNA into bacteriophage vector

Bacteriophage vectors designed for the cloning and propagation of cDNA are provided ready-digested with *Eco* RI and with phosphatased ends from commercial sources (*e*.*g*., lambda gt10 from Stratagene). The prepared cDNA is ligated into lambda vectors following manufacturer's instructions. Ligated vector/cDNA molecules are packaged into phage particles using packaging extracts available commercially.

### Example 8: Screening of Human cDNA Library

### a. Labeling of cDNA fragments (probes) for library screening

An RNA probe is used that recognizes nucleotide sequences that are specific to *NaN,* such as 1371-1751 of NaN. Other nucleotide sequences can be developed on the basis of the NaN sequence (Fig. 2, 7 and 8) such as nucleotides 765-1160 of the human nucleotide sequence. A *Hind* III/*Bam* HI fragment of *NaN* was inserted in pBluescript (SK+) vector (Stratagene). The sequence of the resulting construct was verified by sequencing. The orientation of the insert is such that the 5' and 3' ends of the construct delineated by the *Hind* III and *Bam* HI restriction enzyme sites, respectively, are proximal to T7 and T3 RNA polymerase promoters, respectively. Digoxigenin-labeled Sense (linearized at the *Hind* III site and transcribed by T7 RNA polymerase) and antisense (linearized at the *Bam* HI site and transcribed by T3 RNA polymerase) transcripts were prepared in vitro using MEGAscript transcription kit (Ambion) according to manufacturer specifications. Briefly, 1 µg linearized template was transcribed with the respective RNA polymerase in a 20 µl final volume containing the following reagents: 1X enzyme mixture containing the respective RNA polymerase and RNase inhibitor and reaction buffer (Ambion), 7.5 mM ATP, GTP and CTP nucleotides, 5.625 mM UTP and 1.725 mM Dig-11UTP (Boehringer Mannheim). In vitro transcription was carried out at 37°C for 3 hrs in a water bath. DNA template was removed by adding 1 µl of RNase-free DNase I (2U/µl) to each reaction and incubating further at 37°C for 15 min. The reaction was then stopped by adding 30 µl nuclease-free H₂) and 25 µl of LiCl precipitation solution (7.5 M Lithium Chloride, 50 mM EDTA).

The mixture was incubated at -20°C for 30 min. The RNA transcripts were pelleted in a microfuge at 13000xg, 4°C for 15 min. The supernatant was removed and the pellet washed once with 100 µl of 75% ethanol. The mixture was re-centrifuged at 13000xg, room temperature for 5 min. The pellet was then air-dried in a closed chamber and subsequently dissolved in 100 ml of RNase-free H₂O. The transcript yield and integrity were determined by comparison to a control DIG-labeled RNA on agarose-formaldehyde gel as described in the DIG/Genius kit according to manufacturer recommendations (Boehringer Mannheim). Alternatively, a skilled artisan can design radioactive probes for autoradiographic analysis.

Other regions of the rat, mouse or human *NaN* sodium channel cDNA, like 3' untranslated sequences, can also be used as probes in a similar fashion for cDNA library screening or Northern blot analysis. Specifically, a probe is made using commercially available kits, such as the Pharmacia oligo labeling kit, or Genius kit (Boehringer Mannheim).

### b. cDNA library screening

Recombinant plaques containing full length human homologues of the *NaN* sodium channel are detected using moderate stringency hybridization washes (50-60°C, 5 x SSC, 30 minutes), using non-radioactive (see above) or radiolabeled DNA or cRNA *NaN*-specific probes derived from the 3' untranslated or other regions as described above. Libraries are screened using standard protocols [59, 60] involving the production of nitrocellulose or nylon membrane filters carrying recombinant phages. The recombinant DNA is then hybridized to *NaN*-specific probes (see above). Moderate stringency washes are carried out.

Plaques which are positive on duplicate filters (i.e., not artefacts or background) are selected for further purification. One or more rounds of screening after dilution to separate the phage are typically performed. Resulting plaques are then purified, DNA is extracted and the insert sizes of these clones characterized. The clones are cross-hybridized to each other using standard techniques [59] and distinct positive clones identified.

Typically, overlapping clones that encode the channel are isolated. Standard cloning techniques are then used to produce a full length cDNA construct that contains any 5' untranslated sequence, the start codon ATG, the coding sequence, a stop codon and any 3' untranslated sequence, a poly A consensus sequence and possibly a poly A run. If overlapping clones do not produce sufficient fragments to assemble a full length cDNA clone, alternative methods like RACE (PCR-based) could be used to generate the missing pieces or a full length clone.

### c. Characterization of the human homologue full-length clone

A *NaN*-specific cDNA sequence from the full-length clone is used as a probe in Northern blot analysis to determine the messenger RNA size in human tissue for comparison with the rat and mouse messenger RNA size. Confirmation of biological activity of the cloned cDNA is carried out using methods similar to those described for the rat *NaN*.

### Example 9: Polymerase chain reaction (PCR) approaches to clone the full length human NaN sodium channels using DNA sequence derived from rat

Total RNA and poly A+ RNA is isolated from human dorsal root ganglia or trigerninal ganglia or other cranial ganglia from post-mortem human material or foetuses or biopsy/surgical tissues as described above. Preparation of cDNA and PCR-based methods are then used as described previously in Example 1.

Using degenerate PCR primers derived from the rat *NaN*-specific coding sequence (see Fig. 2), the cDNA is amplified using the polymerase chain reaction [69]. A skilled artisan could utilize the many variables which can be manipulated in a PCR reaction to derive the homologous sequences required. These include, but are not limited to, varying cycle and step temperatures, cycle and step times, number of cycles, thermostable polymerase, and Mg²⁺ concentration. A greater specificity can be achieved using nested primers derived from further conserved sequences from the *NaN* sodium channel.

Amplification is typically performed in 60 µl volume using 1 µl of the first strand cDNA, 0.8 mM of each primer and 1.75 units of Expand Long Template DNA polymerase enzyme mixture (Boehringer Mannheim). Compared to conventional and thermostable DNA polymerases, Expand Long Template enzyme mixture increases the yield of the PCR products without an increase in non-specific amplification [49,50]. The PCR reaction buffer consists of 50 mM Tris-HCl (pH 9.2), 16 mM (NH4)₂SO₄, 2.25 mM MgCl₂, 2% (v/v) DMSO and 0.1 % Tween 20. As described previously [46], amplification is carried out in two stages using a programmable thermal cycler (PTC-200, MJ Research, Cambridge, MA.). First, a denaturation step at 94°C for 4 min, an annealing step at 60°C for 2 min and an elongation step at 72°C for 90 sec. Second, a denaturation step at 94°C for 1 min, an annealing step at 60°C for 1 min and an elongation step at 72°C for 90 sec. The second stage is repeated 33 times for a total of 35 cycles, with the elongation step in the last cycle extended to 10 min. In addition, control reactions are performed alongside the samples. These should be: 1) all components without cDNA, (negative control) and 2) all reaction components with primers for constitutively expressed product, e.g, GAPDH.

The products of the PCR reactions are examined on 1-1.6% (w/v) agarose gels. Bands on the gel (visualized by staining with ethidium bromide and viewing under UV light) representing amplification products of the approximate predicted size are then cut from the gel and the DNA purified.

The resulting DNA may be sequenced directly or is ligated into suitable vectors such as, but not limited to, pCR II (Invitrogen) or pGEMT (Promega). Clones are then sequenced to identify those containing sequence with similarity to the rat, mouse or partial human *NaN* sodium channel sequence.

### Example 10: Clone analxsis

Candidate clones from Example 9 are further characterized by conventional techniques. The biological activity of expression products is also confirmed using conventional techniques.

### Example 11: Isolation of full length NaN sequences from human fetal tissues

Commercially available human fetal cDNA libraries and/or cDNA pools are screened with *NaN*-specific primers (by PCR) or probes (library screening) using PCR standard PCR protocols and standard library screening procedures as described above.

### Example 12: Northern Blot of rat DRG or Trigeminal Neurons with Fragments of Rat NaN

10-30 µg total DRG and/or RNA from DRG or trigeminal (for positive tissues) and cerebral hemisphere, cerebellum and liver (for negative tissues) is electrophoresed in denaturing 1% agarose-formaldehyde gel or agarose-glyoxal gel, and then is transferred to a nylon membrane as described in achieved in multiple steps, as detailed in standard molecular biology manuals [59, 60]. Radiolabeled (specific activity of >10⁸ dpm/ug) or Digixoginen-labeled RNA probes are typically used for Northern analysis. An antisense RNA probe (see Example 20, which describes *in situ* hybridization with a *NaN*-specific probe) is created by *in vitro* synthesis from a sense DNA fragment. The membrane carrying the immobilized RNA in wetted with 6x SSC, and the membrane is placed RNA-side-up in a hybridization tube. One ml formamide prehybridization/hybridization solution per 10 cm² of membrane is added. Prehybridization and hybridization are usually carried out in glass tubes in a commercial hybridization oven. The tubes are place in a hybridization oven and incubated, with rotation, at 60°C for 15 min to 1 hr. The desired volume of probe is pipeted into the hybridization tube, and the incubation is continued with rotation overnight at 60°C. The probe concentration in the hybridization solution should be 10 ng/ml if the specific activity is 10⁸ dpm/ug or 2 ng/ml if the specific activity is 10⁹ dpm/ug (use 2-10 ng/ml ofDigixogenin labeled probe).

The hybridization solution is poured off and an equal volume of 2x SSC/0.1% SDS is added. Incubation with rotation for 5 min at room temperature is carried out. The wash solution is changed, and this step is repeated. To reduce background, it may be beneficial to double the volume of the wash solutions. The wash solution is replaced with an equal volume of 0.2x SSC/0.1% SDS and the tube is incubated for 5 min with rotation at room temperature. The wash solution is changed and this step is repeated (this is a low-stringency wash). For moderate or high stringency conditions, further washes are done with wash solutions pre-warmed to moderate (42°C) or high (68°C) temperatures. The final wash solution is removed and the membrane rinsed in 2x SSC at room temperature. Autoradiography is then performed for up to 1 week. Alternatively, signal is detected using chemiluminescence technology (Amersham) if non-radioactive probes are used. The transcript size is calculated from the signal from the gel in comparison with gel molecular weight standard markers.

### Example 13: Tissue specific distribution of NaN by RT-PCR

*NaN*-specific forward (5' CCCTGCTGCGCTCGGTGAAGAA 3') and reverse primer (5' GACAAAGTAGATCCCAGAGG 3'), were used in RT-PCR assays using cDNA template prepared from multiple rat. These primers amplify *NaN* sequence between nucleotides 765 and 1156 (392 bp) and are *NaN*-specific as judged by lack of similarity to sequences in the database (using programs like BLASTN from the National Library of Medicine). Amplification was typically performed in a 60 µl volume using 1 µl of the first strand of cDNA, 0.8 µM of each primer and 1.75 units of Expand Long Template DNA polymerase enzyme mixture (Bochringer Mannheim). Compared to conventional and thermostable DNA polymerases, Expand Long Template enzyme mixture increases the yield of the PCR products without an increase in non-specific amplification [49, 50]. The PCR reaction buffer consisted of 50 mM Tris-HCl (pH 9.2), 16mM (NH4)₂SO₄, 2.25 mM MgCl₂, 2% (v/v) DMSO and 0.1% Tween 20. As described previously [71], amplification was carried out in two stages using a programmable thermal cycler (PTC-200, MJ Research, Cambridge, MA.). First, a denaturation step is performed at 94°C for 4 min., followed by an annealing step at 60°C for 2 min, and then an elongation step at 72°C for 90 sec. Second, a denaturation step is performed at 94°C for 1 min, followed by an annealing step at 60°C for 1 min, and then an elongation step at 72°C for 90 sec. The second stage was repeated 33 times for a total of 25-35 cycles, with the elongation step in the last cycle extended to 10 min.

Glyceraldehyde-3-phosphate dehydrogenase (GAPDH) was used as an internal control to ensure that a lack of *NaN* signals in different tissues was not due to degraded templates or presence of PCR inhibitors. Rat GAPDH sequences were co-amplified using primers which amplify a 66 bp product that corresponds to nucleotides 328-994 (accession number: M17701). The amplified product spans multiple exon/intron splice sites, based on the structure of the human gene [72]. Dnase I treatment was routinely performed prior to reverse transcription to prevent amplification of GAPDH sequences from genomic processed pseudogenes that may have contaminated the total RNA preparation [73].

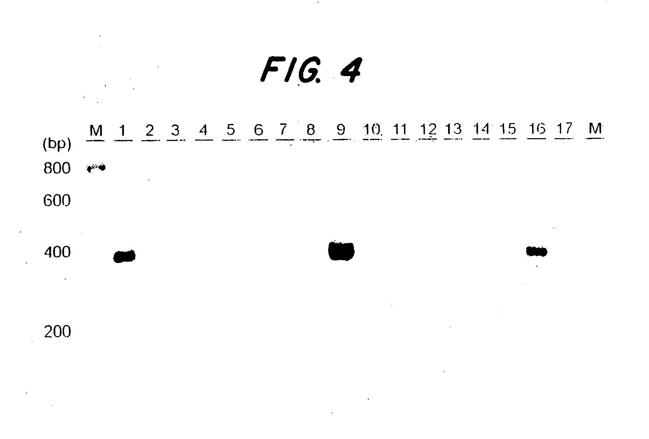

*NaN* is primarily and preferentially expressed in DRG and trigeminal ganglia neurons. Figure 4 shows the results of screening by RT-PCR for the expression of *NaN* in various neuronal and non-neuronal tissues. Lanes 1, 2, 4, 9 and 16 show a single amplification product co-migrating with the 400 bp marker, consistent with *NaN*-specific product of 392 bp. Lanes 1 and 16, 2, 4 and 9 contain products using DRG, cerebral hemisphere, retina, and trigeminal ganglia, respectively. Using this assay, *NaN* was not detected in cerebellum, optic nerve, spinal cord, sciatic nerve, superior cervical ganglia, skeletal muscle, cardiac muscle, adrenal gland, uterus, liver or kidney (lanes 3, 5-8, and 10-15, respectively). The attenuated *NaN* signal in cerebral hemisphere and retina, and the absence of this signal in the remaining tissues is not due to degraded RNA or the presence of PCR inhibitors in the cDNA templates as comparable GAPDH amplification products were obtained in a parallel set of PCR reaction (data not shown).

### Example 14: Transformation of a Host Cell with the NaN Coding Sequence

Transformed host cells for the measurement of Na⁺ current or intracellular Na⁺ levels are usually prepared by co-transfecting constructs into cells such as HEK293 cells with a fluorescent reporter plasmid (pGreen Lantern-1, Life Technologies, Inc.) using the calcium-phosphate precipitation technique [27]. HEK293 cells are typically grown in high glucose DMEM (Life Technologies, Inc.) supplemented with 10% fetal calf serum (Life Technologies, Inc). After 48 hrs, cells with green fluorescence are selected for recording [28].

For preparation of cell lines continuously expressing recombinant channels, the *NaN* construct is cloned into other vectors that carry a selectable marker in mammalian cells. Transfections are carried out using the calcium phosphate precipitation technique [27]. Human embryonic kidney (HEK-293), chinese hamster ovary (CHO) cells, or other suitable cell lines are grown under standard tissue culture conditions in Dulbeccos's modified Eagle's medium supplemented with 10% fetal bovine serum. The calcium phosphate-DNA mixture is added to the cell culture medium and left for 15-20 hr, after which time the cells are washed with fresh medium. After 48 hrs, antibiotic (G418, Geneticin, Life Technologies) is added to select for cells which have acquired neomycin resistance. After 2-3 weeks in G418, 10-20 isolated cell colonies are harvested using sterile 10ml pipette tips. Colonies are grown for another 4-7 days, split and subsequently tested for channel expression using whole-cell patch-clamp recording techniques and RT-PCR.

### Example 15: Production ofNaN specific Antibodies

Antibodies specific to the rat, mouse or human NaN are produced with an immunogenic NaN-specific peptide by raising polyclonal antibodies in rabbits. In one example, the peptide **CGPNPASNKDCFEKEKDSED** (rat amino acids 285-304) was selected because it fits the criteria for immunogenecity and surface accessibility. This peptide sequence does not match any peptide in the public databases. The underlined cysteine (C) residue was changed to Alanine (A) to prevent disulfide bond formation. This amino acid change is not expected to significantly affect the specificity of the antibodies.

Peptide synthesis, rabbit immunization, and affinity purification of the antipeptide antibodies were performed using standard procedures. Purified antibodies were then tested on DRG neurons in culture. Immunostaining procedures using these antibodies before and after blocking with excess peptide were performed according to standard procedures.

DRG neurons after 16-24 h in culture were processed for immunocytochemical detection of NaN protein as follows. Coverslips were washed with complete saline solution (137 mM NaCl, 5.3 mM KCI, 1 ITIM M902 25 mM sorbitol, 10 mM HEPES, 3 mM CaC12 pH 7.2), fixed with 4% paraformaldehyde in 0. 14 M phosphate buffer for 10 min at 4°C, washed with three 5-min with phosphate-buffered saline (PBS), and blocked with PBS containing 20% normal goat serum, 1% bovine serum albumin and 0. 1 % Triton X- 100 for 15 minutes. The coverslips were incubated in anti-NaN antibody (1:100 dilution) at 4°C overnight. Following overnight incubation, coverslips were washed extensively in PBS and then incubated with goat anti-rabbit IgG-conjugated to Cy3 (1:3000; Amersharn) for 2 h at room temperature. The coverslips were rinsed with PBS and mounted onto glass slides with Aqua-poly-mount. The neurons were examined with a Leitz Aristoplan light microscope equipped with epifluorescence and images were captured with a Dage DC330T color camera and Scion CG-7 color PCI frame grabber (see Figure 7).

### Example 16: NaN expression is altered in a neuropathic pain model

The CCI model of neuropathic pain (Bennett and Xie) was used to study the plasticity of sodium channel expression in DRG neurons. Twenty two adult, femal Sprague-Dawley rats, weighing 240-260g, were anesthetized with pentobarbital sodium (50 mg/kg ip) and the right sciatic nerve exposed at the mid-thigh. Four chromic gut (4-0) ligatures were tied loosely around the nerve as described by Bennett and Xie (1988) *Pain 33, 87-107.* The incision site was closed in layers and a bacteriostatic agent administered intramuscularly.

Previous studies have shown that transection of the sciatic nerve induces dramatic changes in sodium currents of axotomized DRG neurons, which is paralleled by significant changes to transcripts of various sodium channels expressed in these neurons. Sodium currents that are TTX-R and the transcripts of two TTX-R sodium channels (SNS/PN3 and NaN) are significantly attenuated while a rapidly repriming silent TTX-S current emerges and the transcript of α-III sodium channel, which produces a TTX-S current, is up-regulated. We have discovered that CCI-induced changes in DRG neurons, 14 days post-surgery, mirror those of axotomy. Transcripts of NaN and SNS, the two sensory neuron-specific TTX-R channels, are significantly down-regulated as is the TTX-R sodium current, while transcripts of the TTX-S α-III sodium channel are up-regulated, in small diameter DRG neurons. These changes may be partly responsible for making DRG neurons hyperexcitable, that contributes to the hyperalgesia that results from this injury.

### Example 17: Assays for agents which modulate the activity of the NaN channel using patch clamp methods

Cells lines expressing the cloned Na⁺ channel are used to assay for agents which modulate the activity of the NaN channel, *e.g*., agents which block or inhibit the channel or enhance channel opening. Since the channel activation is voltage dependent, depolarizing conditions may be used for observation of baseline activity that is modified by the agent to be tested. Depolarization may be achieved by any means available, for example, by raising the extracellular potassium ion concentration to about 20 to 40 nM, or by repeated electrical pulses.

The agent to be tested is incubated with HEK 293 or other transformed cells that express the Na⁺ channel [28]. After incubation for a sufficient period of time, the agent induced changes in Na⁺ channel activity can be measured by patch-clamp methods [29]. Data for these measurements are acquired on a Macintosh Quadra 950, or similar computer, using a program such as Pulse (v 7.52, HEKA, German). Fire-polished electrodes (0.8-1.5 MW) are fabricated from capillary glass using a Sutter P-87 puller or a similar instrument. Cells are usually only considered for analysis if initial seal resistance is <5 Gohm, they have high leakage currents (holding current <0.1 nA at -80 mV), membrane blebs, and an access resistance <5 Mohm. Access resistance is monitored and data is not used if resistance changes occur. Voltage errors are minimized using series resistance compensation and the capacitance artifact will be canceled as necessary using computer-controlled amplifier circuitry or other similar methods.

For comparisons of the voltage dependence of activation and inactivation, cells with a maximum voltage error of <10 mV after compensation are usually used. Linear leak subtraction is used for voltage clamp recordings. Membrane currents are typically filtered at 5 KHz and sampled at 20 KHz. The pipette solution contains a standard solution such as: 140 mM CsF, 2 mM MgCl₂, 1 mM EGTA, and 10 mM Na-HEPES (pH 7.3). The standard bathing solution is a standard solution such as 140 mM NaCl, 3 mM KCl, 2 mM MgCl₂, 1 mM CaCl₂, 10 mM HEPES, and 10 mM glucose (pH 7.3).

Tetrodotoxin (TTX)-resistant and TTX-sensitive Na⁺ currents are measured by exposure to appropriate concentrations of TTX and/or by pre-pulse protocols which distinguish between TTX-sensitive and TTX-resistant currents on the basis of their distinct steady-state inactivation properties [22,55].

Data are collected using standard pulse protocols and are analyzed to measure sodium current properties that include voltage-dependence, steady-state characteristics, kinetics, and re-priming. Measurements of current amplitude and cell capacitance provides an estimate of Na⁺ current density, thereby permitting comparisons of channel density under different conditions [22,30]. Cells are studied in the current clamp mode to study patterns of spontaneous and evoked action potential generation, threshold for firing, frequency response characteristics, and response to de- and hyperpolarization, and other aspects of electrogenesis [55]. These measurements are carried out both in control cells expressing *NaN* and in cells expressing *NaN* that also have been exposed to the agent to be tested.

### Example 18: Assays for agents which modulate the activity of the NaN channel by the measurement of Intracellular Sodium [Na⁺]

The agent to be tested is incubated with cells exhibiting NaN channel activity. After incubation for a sufficient period of time, the agent induced changes in Na⁺ channel are measured by ratiometric imaging of [Na⁺], using SBFI. In this method, cytosolic-free Na⁺ is measured using an indicator for Na⁺, such as SBFI (sodium-binding benzofuran isophthalate; [33]) or a similar dye. Cells are first loaded with the membrane-permeable acetoxymethyl ester form of SBFI (SBFI/AM) or a similar dye (usually dissolved in dimethyl sulfoxide (DMSO) at a stock concentration of 10 mM). Recordings are obtained on the stage of a microscope using a commercially available ratiometric imaging setup (*e*.*g*., from Georgia Instruments). Excitation light is provided at appropriate wavelengths (*e.g*., 340:385 nm). Excitation light is passed to the cells through a dichroic reflector (400 nm) and emitted light above 450 nm was collected. Fluorescence signals are amplified, *e*.*g*., by an image intensifier (GenIISyS) and collected with a CCD camera, or similar device, interfaced to a frame grabber. To account for fluorescence rundown, the fluorescence ratio 340:385 is used to assay cytosolic-free Na⁺.

For calibration of SBFI's fluorescence, cells are perfused with calibration solutions containing known Na⁺ concentrations (typically 0 and 30 mM, or 0, 30, and 50 mM [Na⁺], and gramicidin and monensin. As reported by Rose and Ransom [34], the 345/390 nm fluorescence ratio of intracellular SBFI changes monotonically with changes in [Na⁺ ]ᵢ. Experiments are repeated on multiple (typically at least 4) different coverslips, providing statistically significant measurements of intracellular sodium in control cells, and in cells exposed to various concentrations of agents that may block, inhibit or enhance the activity of the channel. Use of this method is illustrated in *Sontheimer et al.* [32].

### Example 19: Assays for agents which modulate the activity of the NaN channel by scintigraphic imaging

Cells lines expressing the cloned Na⁺ channel are used to assay for agents which modulate the activity of the NaN channel, *e*.*g*., agents which block the channel or enhance channel opening. For example, the agent to be tested is incubated with HEK 293 or other transformed cells that express the Na⁺ channel [28]. After incubation for a sufficient period of time, the agent induced changes in Na⁺⁺ channel activity are detected by the measurement of Na⁺ influx by isotopic methods. ²²Na is a gamma emitter and can be used to measure Na⁺ flux [31] and ⁸⁶Rb⁺ can be used to measure Na⁺/K⁺ ATPase activity which provides a measure of Na channel activity [32] ⁸⁶Rb⁺ ions are taken up by the Na⁺/K+ATPase like K+ ions, but have the advantage of a much longer half-life than ⁴²K⁺ [35]. Thus, measurement of the unidirectional ouabain-sensitive ⁸⁶Rb⁺ uptake provides a quantitative method for assaying Na⁺/K⁺-ATPase activity which follows action potentials.

Following incubation of cell expressing *NaN* to the isotope, the cellular content of the isotope is measured by liquid scintillation counting or a similar method, and cell protein is determined using a method such as the bicinchoninic acid protein assay [36] following the modifications [37] for cultured cells. ²²Na and ⁸⁶Rb⁺ fluxes are determined in the presence and absence of agents that may block, inhibit, or enhance Na⁺. This permits determination of the actions of these agents on *NaN*.

### Example 20: In situ hybridization

### a. Probes

Probes are prepared as described above in Example 5.

### b. DRG Neuron Culture

Cultures of DRG neurons from adult rats were established as described previously [70]. Briefly, lumbar ganglia (L4, L5) from adult Sprague Dawley female rats were freed from their connective sheaths and incubated sequentially in enzyme solutions containing collagenase and then papain. The tissue was triturated in culture medium containing 1:1 Dulbecco's modified Eagle's medium (DMEM) and Hank's F12 medium and 10% fetal calf serum, 1.5 mg/ml trypsin inhibitor, 1.5 mg/ml bovine serum albumin, 100 U/ml penicillin and 0.1 mg/ml streptomycin and plated at a density of 500-1000 cells/mm² on polyornithine/laminin coated coverslips. The cells were maintained at 37°C in a humidified 95% air/5% CO₂ incubator overnight and then processed for *in situ* hybridization cytochemistry as described previously [56, 57]. Trigeminal ganglia can be cultured by a skilled artisan using similar methods.

### c. Tissue Preparation

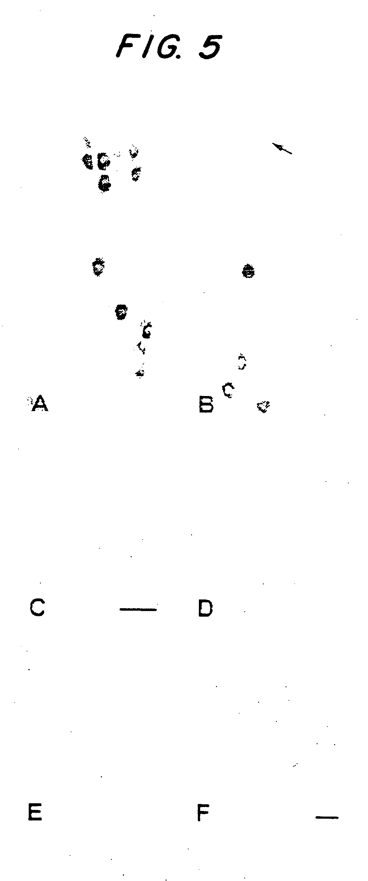

Adult female Sprague Dawley rats were deeply anesthetized, e.g., with chloral hydrate and perfused through the heart, first with a phosphate-buffered saline (PBS) solution and then with a 4% paraformaldehyde in 0.14 M Sorensen's phosphate buffer, pH 7.4, at 4°C. Following perfusion fixation, dorsal root ganglia at levels L4 and L5 and trigeminal ganglia were collected and placed in fresh fixative at 4°C. After 2-4 hours, the tissue was transferred to a solution containing 4% paraformaldehyde and 30% sucrose in 0.14 M phosphate buffer and stored overnight at 4°C. Fifteen µm sections were cut and placed on poly-L-lysine-coated slides. The slides were processed for *in situ* hybridization cytochemistry as previously described [24, 56]. Following *in situ* hybridization cytochemistry, the slides were dehydrated, cleared and mounted with Permount. The results are shown in Fig. 5.

Sections of DRG hybridized with *NaN* sense riboprobe showed no specific labeling (panel C, Fig. 5). In DRG (panel A, Fig. 5) and trigeminal (panel B) sections hybridized with a *NaN* antisense riboprobe, with the *NaN* signal present in most small (<30 mm diam.) neurons; in contrast, most large (>30 mm diam.) neurons did not exhibit *NaN* hybridization signal. Sections of spinal cord, cerebellum and liver hybridized with an antisense *NaN* riboprobe showed no specific signal (panels D, E and F respectively).

### Example 21: Microsatellite Sequences

The following are the murine intronic microsatellite sequences. These microsatellites may be polymorphic in the human SCN 11a gene and could be used as markers to screen for mutant alleles that are associated with a disease. Such screening methods, for instance, hybridization or amplification assays, are readily available. See Sambrook *et al*. or *Ausubel et al*.

Intron 4; microsatellite is dTdg

AGTTTAATGTTGAGTGAATTGTGGTGGTGATTTCCCACTTGAG
GCCTTTGTGTTAAAGCCCAATGTGTGTGTGTGTGTGTGTGTGTGTG
TGTGTGTGTGTGTGTGTGTGTGTGTGGTTGGGGGGTGGTGGCAGAGT
CTGGTATTGGTAAGGTGAGAGCAATCCCAGAACGTCCACCTGCTCTT
CCATTTTATTAATCAGGCAGGCCTCT

Intron 6; microsatellite is dCdA

TGTGCATGCTTGATTCCCAGCTCCTATGGTCTGATTACTCGGT
CCTTAGGAGCAAGGCCAGACTGTCCACCCTGACACACACACACACA
CACACACACACACACACACACACACACACACACACAGTGTAGAGAATT
ACCTCATTCTTGGAGTTTCTCTGGAAAAGGAATGTCTCAAAGCCAAG
TTCACAGAGCAACAGCTG

Intron 5; microsatellite is dCdTdG (dNdG2)x (X5-30)

GTAAGCCACTGGCTCTTAACTAAAATGCTCGTTGGCATTAGAA
CATTTCTGAGCTGGGGTGGTGGTGGTGGTGGTGGTGGTGGTGGT
GGTGGTGGTGGTGGTGGTGGTGATGGTGGTGGTGGAGGTGGNGGTG
GAGGTGGTGGCTGTGGTGGTGGNGGTGGTGGTGGTGGANGTGG
ANGTGGTGGCGTGGTGGTGGNGGTGGTGGTGGAGGTGGTGGCTGTG
GTGGTNGTGGTGGC

Intron 8; 5′ microsatellite is dTdC followed by a stretch of dT

TGTTAGAAACTCTAAGACAATGAAGCACCATGCTGGAAATAA
GAGCACAAACTCTTTCTTCATGCATTACCCACTGCTTGTGCTTTCACC
TTAGTGCTCGTGCTCTCTCTTTCTCTCTCTCTCTCTCTCTCTCTCTC
TCTCTCTCTCTCTCTCTCTCTCTCTCTCTCTTTTTTTTTTTTTT

Intron 8; 3′ microsatellite is dCdA

CACACACACACACACACACACACACACACACACACACACAG
AGAAACACTGTCGCAGTCATACATATAAAGATAAATACATCTTAAA
AAAAGAACCATGTGATTGAGTTATAAAATATTCCAACTTAT

Intron 10B; microsatellite is dCdA followed, three nucleotides
downstream by dCdA3.

AGGTCATTTCCTCTGCAGTGTGCTTGGCAGGAAAAACTTCCTG
GCTATTCAAGTCAGTGCCCTGCTTGATCATCCATGTATCACACACAC
ACAAAACAAACAAACAAACAAACAAAACCCTGGGGAAGAAGGAAG
AGGTTAAGCACATAGGCAGAGAGCAGCCAGGCTGACTCAGAGCAAA
CACCTGATCATTCTTCCAT

Intron 12; microsatellite is dpydG (dt/dCdG)

GTGCTGGGATCAAAGGCGTGCGCCGCCACCACGCCCGGCCCC
TTTTTATGTTTCAAATTTACTTTTATCATGTGCACGTGTGTGGGTGCG
TGCATGTGTGTGCGTGCGTGTGCGTGTGNGTGTGNGTGTGTGTGTGT
GTGTGTGTGTGTGTGTGTGTG

Intron 14; microsatellite is dCdA

CACACACACACACACACACACACACACACACACACACACACACACA
CACACACACACACACACTTGCATCTTTGAGTTAATTGGATAGGCTGAGT
CTTACACCGGAATCATACTGTTGC

Intron 15A; microsatellige is dCdA

CCAATGAGAGACTCTTGTCTCAAAAAAGCCATGGTGTCCAGA
TCCTGAGGAATAACACCTAAGAATGTGCTCTGGCCTGAAAACACAC
ACACACACACACACACACACACACACACACACACACAGTTTTATTTATTTA
TTTAAAAAAATATGTCTCTAGGCATTGCTGAAATGTCTCCTACAGGA
TTAAGTCAACCAGAGCCA

It should be understood that the foregoing discussion and examples merely present a detailed description of certain preferred embodiments. It will be apparent to those of ordinary skill in the art that various modification and equivalents can be made without departing from the spirit and scope of the invention.

### REFERENCES CITED

The following documents were cited and discussed above. These and any other documents referred to in this patent specification are hereby incorporated by reference in their entirety.
[1] Catterall, W.A. (1993) Trends in Neurosciences 16: 500-6.
[2] Isom, L.L., De Jongh, K.S. and Catterall, W.A. (1994) Neuron 12: 1183-94.
[3] Goldin, A.L. (1995) in: Handbook of receptors and channels, pp. 73-100 (North, R.A., Ed.) CRC press, Boca Raton, FL.
[4] Akopian, A.N., Sivilotti, L. and Wood, J.N. (1996) Nature 379: 257-62.
[5] Akopian, A.N., Souslova, V., Sivilotti, L. and Wood, J.N. (1997) FEBS Letters 400: 183-187.
[6] Sangameswaran, L. *et al.* (1996) J. Biol. Chem. 271: 5953-6.
[7] Beckh, S., Noda, M., Lubbert, H. and Numa, S. (1989) EMBO J. 8: 3611-6.
[8] Mandel, G. (1992) J. Membrane Biology 125: 193-205.
[9] Roden, D.M. and George, A.L., Jr. (1997) Am. J. Physiol. 273: H511-25.
[10] Ptacek, L.J. (1997) Neuromuscul. Disord. 7: 250-5.
[11] Cannon, S.C. (1997) Neuromuscul. Disord. 7:241-9.
[12] Cannon, S.C. (1996) Trends Neurosci. 19: 3-10.
[13] Rizzo, M.A., Kocsis, J.D. and Waxman, S.G. (1996) European Neurology 36: 3-12.
[14] Felipe, A., Knittle, T.J., Doyle, K.L. and Tamkun, M.M. (1994) J. Biol. Chem. 269: 30125-31.
[15] Eccles, J.C., Libet, B. and Young, R.R. (1958) J. Physiol. 143: 11-40.
[16] Gallego, R., Ivorra, I. and Morales, A. (1987) J. Physiol. (Lond) 391: 39-56.
[17] Kuno, M. and Llinas, R. (1970) J. Physiol. (Lond.) 210: 807-821.
[18] Dodge, F.A. and Cooley, J.W. (1973) IBM J. Res. Dev. 17: 219-229.
[19] Titmus, M.J. and Faber, D.S. (1986) J. Neurophysiol. 55: 1440-1454.
[20] Sernagor, E., Yarom, Y. and Werman, R. (1986) Proc. Natl. Acad. Sci. (USA) 83: 7966-70.
[21] Rizzo, M.A., Kocsis, J.D. and Waxman, S.G. (1995) Neurobiol. Dis. 2: 87-96.
[22] Cummins, T.R. and Waxman, S.G. (1997) J. Neurophysiology 17: 3503-3514.
[23] Dib-Hajj, S., Black, J.A., Felts, P. and Waxman, S.G. (1996) Proc. Natl. Acad. Sci. (USA) 93: 14950-4.
[24] Waxman, S.G., Kocsis, J.D. and Black, J.A. (1994) J. Neurophysiology 72: 466-70.
[25] Gold, M.S., Reichling, D.B., Shuster, M.J. and Levine, J.D. (1996) Proc. Natl. Acad. Sci. (USA) 93: 1108-12.
[26] England, S., Bevan, S. and Docherty, R.J. (1996) J. Physiology 495: 429-40.
[27] Ukomadu, C., Zhou, J., Sigworth, F.J. and Agnew, W.S. (1992) Neuron 8: 663-76.
[28] Dib-Hajj, S.D., Ishikawa, K., Cummins, T.R. and Waxman, S.G. (1997) FEBS Letters 416: 11-14.
[29] Hamill, O.P., Neher, M.A., Sakmann, B. and Sigworth, F.J. (1981) Pflügers Arch. 391: 85-100.
[30] Rizzo, M.A., Kocsis, J.D. and Waxman, S.G. (1994) J. Neurophysiology 72: 2796-815.
[31] Kimelberg, H.K. and Waltz, W. (1988) (Boulton, A., Baker, G. and Walz, W., Eds.).
[32] Sontheimer, H., Femandez-Marques, E., Ullrich, N., Pappas, C.A. and Waxman, S.G. (1994) J. Neuroscience 14: 2464-75.
[33] Harootunian, A., Kao, J.P.Y., Ecker, B.K. and Tsien, R.Y. (1989) J. Biol. Chem. 264: 19458-19467.
[34] Rose, C.R. and Ransom, B.R. (1996) J. Physiol. (Lond) 491: 291-305.
[35] Kimelberg, H.K. and Mayhew, E. (1975) J. Biol. Chem. 250: 100-104.
[36] Smith, P.K. *et al*. (1985) Anal. Biochem. 150: 76-85.
[37] Goldschmidt, R.C. and Kimelberg, H.K. (1989) Analytical Biochemistry 177: 41-45.
[38] Dubner, R. (1994) in: Textbook of Pain (Wall, P.D. and Melzack, R., Eds.) Churchill Livingstone Publishers.
[39] Dubuisson, D. and Dennis, S.G. (1977) Pain 4: 161-74.
[40] Iadarola, M.J., Brady, L.S., Draisci, G. and Dubner, R. (1988) Pain 35: 313-26.
[41] Bennett, G.J. and Xie, Y.K. (1988) Pain 33: 87-107.
[42] Kim, S.H. and Chung, J.M. (1992) Pain 50: 355-63.
[43] Seltzer, Z., Dubner, R. and Shir, Y. (1990) Pain 43: 205-18.
[44] Stys, P.K., Ransom, B.R. and Waxman, S.G. (1992) J. Neurophysiology 67: 236-40.
[45] Chomczynski, P.a.S., N (1987) Anal. Bioch. 162: 156-159.
[46] Dib-Hajj, S.D., Hinson, A.W., Black, J.A. and Waxman, S.G. (1996) FEBS Letters 384: 78-82.
[47] Gu, X.Q., Dibhajj, S., Rizzo, M.A. and Waxman, S.G. (1997)J. Neurophysiology 77: 236-246.
[48] Fjell, J., Dibhajj, S., Fried, K., Black, J.A. and Waxman, S.G. (1997) Molecular Brain Research 50: 197-204.
[49] Barnes, W.M. (1994) Proc. Natl. Acad. Sci. (USA) 91: 2216-2220.
[50] Cheng, S., Fockler, C. Barnes W. M. and Higuchi, R. (1994) Proc. Natl. Acad. Sci. (USA) 91: 5695-5699.
[51] George, A.L., Jr., Iyer, G.S., Kleinfield, R., Kallen, R.G. and Barchi, R.L. (1993) Genomics 15: 598-606.
[52] Souslova, V.A., Fox, M., Wood, J.N. and Akopian, A.N. (1997) Genomics 41: 201-209.
[53] McClatchey, A.I., Lin, C.S., Wang, J., Hoffinan, E.P., Rojas, C. and Gusella, J.F. (1992) Hum. Mol. Genet. 1: 521-7.
[54] Wang, Q., Li, Z., Shen, J. and Keating, M.T. (1996) Genomics 34: 9-16.
[55] Sontheimer, H. and Waxman, S.G. (1992) J. Neurophysiology 68: 1001-11.
[56] Black, J.A., Yokoyama, S., Waxman, S.G., Oh, Y., Zur, K.B., Sontheimer, H., Higashida, H. and Ransom, B.R. (1994) Brain Research. Molecular Brain Research 23: 235-45.
[57] Zur, K.B., Oh, Y., Waxman, S.G. and Black, J.A. (1995) Brain Research. Molecular Brain Research 30: 97-105.
[58] Tanaka *et al*., unpublished.
[59] Sambrook *et al., Molecular Cloning: A Laboratory Approach,* Cold Spring Harbor Press, NY, 1989.
[60] Ausubel *et al., Current Protocols in Molecular Biology,* Greene Publishing Co., NY, 1995.
[61] Cohen *et al., Proc Acad Sci USA* (1972) 69: 2110.
[62] Maniatis *et al., Molecular Cloning,* Cold Spring Harbor Laboratory, Cold Spring Harbor, N.Y. 1982.
[63] Graham *et al.,* Virol. (1973) 52: 456.
[64] Wigler *et al*., Proc. Natl. Acad. Sci. USA (1979) 76: 1373-76.
[65] Southern, *J. Mol. Biol*. (1975) 98: 503.
[66] Berent *et al*., *Biotech* (1985) 3: 208.
[67] Rose, *et al*., *J. Neurophysiology,* 1997 in press.
[68] Dib-Hajj and Waxman, unpublished.
[69] Saiki *et al.* (1985) *Science* 230: 1350.
[70] Rizzo, M.A., Kocsis, J.D., and Waxman, S.G. (1994). Slow sodium conductances of dorsal root ganglion neurons: intraneuronal homogeneity and interneuronal heterogeneity. Journal of Neurophysiology *72*, 2796-815.
[71] Dib-Hajj, S.D., Hinson, A.W., Black, J.A., and Waxman, S.G. (1996). Sodium channel mRNA in the B104 neuroblastoma cell line. FEBS Letters *384*, 78-82.
[72] Benham, C.D., and Tsien, R.W. (1987). A novel receptor-operated Ca2+-permeable channel activated by ATP in smooth muscle. Nature *328*, 275-8.
[73] Ercolani, L., Florence, B., Denaro, M., and Alexander, M. (1988). Isolation and complete sequence of a functional human glyceraldehyde-3-phosphate dehydrogenase gene. J Biol Chem *263*, 15335-41.

## Claims

1. An isolated nucleic acid molecule selected from the group consisting of a nucleic acid molecule comprising the nucleotide sequence shown in SEQ ID NO: 4 (Figure 7A), an isolated nucleic acid molecule comprising a nucleotide sequence that encodes a protein with the amino acid sequence shown in SEQ ID NO: 5 (Figure 7B), and a nucleic acid molecule that encodes a protein capable of producing a sodium current and which hybridizes to the sequence shown in SEQ ID NO: 4 (Figure 7A) under highly stringent conditions.

2. The isolated nucleic acid molecule of claim 1, wherein the nucleic acid molecule encodes a voltage-gated sodium channel.

3. The isolated nucleic acid molecule of claim 1, wherein the nucleic acid encodes a murine voltage-gated sodium channel.

4. An isolated nucleic acid molecule consisting of the nucleotide sequence shown in SEQ ID NO: 4 (Figure 7A).

5. An isolated nucleic acid molecule comprising the nucleotide sequence shown in SEQ ID NO: 6 (Figure 8A).

6. An isolated nucleic acid molecule comprising a nucleotide sequence that encodes a protein with the amino acid sequence shown in SEQ ID NO: 7 (Figure 8B).

7. The isolated nucleic acid molecule of any one of claims 1-6, wherein said nucleic acid molecule is operably linked to one or more expression control elements.

8. A vector comprising an isolated nucleic acid molecule of any one of claims 1-6.

9. A host cell transformed to contain the nucleic acid molecule of any one of claims 1-6.

10. A host cell comprising a vector of claim 8.

11. A host cell of claim 10, wherein said host is selected from the group consisting of prokaryotic hosts and eukaryotic hosts.

12. A method for producing a protein comprising the step of culturing a host cell of claim 9 under conditions in which the protein is expressed.

13. An isolated polypeptide produced by the method of claim 12.

14. An isolated polypeptide encoded by the nucleic acid molecule of any one of claims 1-6.

15. An isolated polypeptide selected from the group consisting of an isolated polypeptide comprising the amino acid sequence shown in SEQ ID NO: 5 (Figure 7B), an isolated polypeptide comprising a fragment of the amino acid sequence shown in SEQ ID NO: 5 (Figure 7B) and capable of transporting sodium ions across a cell membrane, an isolated polypeptide comprising the amino acid sequence shown in SEQ ID NO: 5 (Figure 7B) with one or more conservative amino acid substitutions, and an isolated polypeptide comprising the amino acid sequence shown in Figure 7B with one or more naturally occurring amino acid sequence variations.

16. An isolated polypeptide consisting of the amino acid sequence shown in SEQ ID NO: 5 (Figure 7B).

17. An isolated polypeptide selected from the group consisting of an isolated polypeptide comprising the amino acid sequence shown in SEQ ID NO: 7 (Figure 8B), an isolated polypeptide comprising the amino acid sequence shown in SEQ ID NO: 7 (Figure 8B) with one or more conservative amino acid substitutions, and an isolated polypeptide comprising the amino acid sequence shown in SEQ ID NO: 7 (Figure 8B) with one or more naturally occurring amino acid sequence variations.

18. A method of identifying an agent which modulates the expression of a nucleic acid encoding the polypeptide of any one of claims 13-17 comprising:
(a) exposing cells which express the nucleic acid to the agent; and
(b) determining whether the agent modulates expression of the nucleic acid.

19. A method of identifying an agent which modulates at least one activity of the polypeptide of any one of claims 13-17 comprising:
(a) exposing cells which express the polypeptide to the agent;
(b) determining whether the agent modulates at least one activity of the protein.

20. The method of claim 19, wherein the activity is determined by measurement of sodium ion transport across the cell membrane.

21. The method of claim 20, wherein the activity is determined by measurement of sodium ion influx into the cell.

22. The method of claim 20, wherein activity is determined by measuring intracellular sodium concentration.

23. The method of claim 21 or 22, wherein sodium ion influx or sodium concentration is measured using ²²Na or ⁸⁶Rb.

24. An method to treat pain or paraesthesia in a mammal comprising administering an effective amount of an agent capable of altering the expression of the nucleic acid of any of claims 1 to 6.

25. A method to treat pain or paraesthesia in a mammal comprising administering an effective amount of an agent capable of altering the activity of the polypeptide of any of claims 13-17.

26. The method of claim 25, wherein the activity is sodium ion influx.

27. The method of claim 24 or 25, wherein the mammal is a human.


SEQUENCE LISTING

<110> Yale University

<120> Dorsal Root Ganglia-Specific and Tetrodotoxin Resistant
      Voltage-Gated Sodium Channel, S. Dib-Hajj et al.

<130> 44574-5004-WO, Yale University

<140> PCT/US99/02008
<141> 1999-01-29

<150> US 60/072,990
<151> 1998-01-29

<150> US 60/109,402
<151> 1998-11-20

<160> 40

<170> PatentIn Ver. 2.0

<210> 1
<211> 5875
<212> DNA
<213> Rattus norvegicus

<220>
<221> CDS
<222> (41)..(5335)
<223> cDNA sequence for rat NaN

<220>
<221> unsure
<222> (1996), (4042)

<223> n = a or c or g or t

<400> 1
acggtgccct gatcctctgt accaggaaga cagggtgaag atg gag gag agg tac    55
                                            Met Glu Glu Arg Tyr
                                              1               5

tac ccg gtg atc ttc ccg gac gag cgg aat ttc cgc ccc ttc act tcc    103
Tyr Pro Val Ile Phe Pro Asp Glu Arg Asn Phe Arg Pro Phe Thr Ser
                 10                  15                  20

gac tct ctg gct gcc ata gag aag cgg att gct atc caa aag gag agg    151
Asp Ser Leu Ala Ala Ile Glu Lys Arg Ile Ala Ile Gln Lys Glu Arg
             25                  30                  35

aag aag tcc aaa gac aag gcg gca gct gag ccc cag cct cgg cct cag    199

Lys Lys Ser Lys Asp Lys Ala Ala Ala Glu Pro Gln Pro Arg Pro Gln
        40                  45                  50

ctt gac cta aag gcc tcc agg aag tta cct aag ctt tat ggt gac att 247
Leu Asp Leu Lys Ala Ser Arg Lys Leu Pro Lys Leu Tyr Gly Asp Ile
    55                  60                  65

ccc cct gag ctt gta gcg aag cct ctg gaa gac ctg gac cca ttc tac 295
Pro Pro Glu Leu Val Ala Lys Pro Leu Glu Asp Leu Asp Pro Phe Tyr
70                  75                  80                  85

aaa gac cat aag aca ttc atg gtg ttg aac aag aag aga aca att tat 343
Lys Asp His Lys Thr Phe Met Val Leu Asn Lys Lys Arg Thr Ile Tyr
                90                  95                  100

cgc ttc agc gcc aag cgg gcc ttg ttc att ctg ggg cct ttt aat ccc 391
Arg Phe Ser Ala Lys Arg Ala Leu Phe Ile Leu Gly Pro Phe Asn Pro
            105                 110                 115

ctc aga agc tta atg att cgt atc tct gtc cat tca gtc ttt agc atg 439
Leu Arg Ser Leu Met Ile Arg Ile Ser Val His Ser Val Phe Ser Met
        120                 125                 130

ttc atc atc tgc acg gtg atc atc aac tgt atg ttc atg gcg aat tct 487
Phe Ile Ile Cys Thr Val Ile Ile Asn Cys Met Phe Met Ala Asn Ser
    135                 140                 145

atg gag aga agt ttc gac aac gac att ccc gaa tac gtc ttc att ggg 535
Met Glu Arg Ser Phe Asp Asn Asp Ile Pro Glu Tyr Val Phe Ile Gly
150                 155                 160                 165

att tat att tta gaa gct gtg att aaa ata ttg gca aga ggc ttc att 583
Ile Tyr Ile Leu Glu Ala Val Ile Lys Ile Leu Ala Arg Gly Phe Ile
                170                 175                 180

gtg gat gag ttt tcc ttc ctc cga gat ccg tgg aac tgg ctg gac ttc 631
Val Asp Glu Phe Ser Phe Leu Arg Asp Pro Trp Asn Trp Leu Asp Phe
            185                 190                 195

att gtc att gga aca gcg atc gca act tgt ttt ccg ggc agc caa gtc 679
Ile Val Ile Gly Thr Ala Ile Ala Thr Cys Phe Pro Gly Ser Gln Val
        200                 205                 210

aat ctt tca gct ctt cgt acc ttc cga gtg ttc aga gct ctg aag gcg 727
Asn Leu Ser Ala Leu Arg Thr Phe Arg Val Phe Arg Ala Leu Lys Ala
    215                 220                 225

att tca gtt atc tca ggt ctg aag gtc atc gta ggt gcc ctg ctg cgc 775
Ile Ser Val Ile Ser Gly Leu Lys Val Ile Val Gly Ala Leu Leu Arg
230                 235                 240                 245

tcg gtg aag aag ctg gta gac gtg atg gtc ctc act ctc ttc tgc ctc 823

Ser Val Lys Lys Leu Val Asp Val Met Val Leu Thr Leu Phe Cys Leu
                250                 255                 260

agc atc ttt gcc ctg gtc ggt cag cag ctg ttc atg gga att ctg aac 871
Ser Ile Phe Ala Leu Val Gly Gln Gln Leu Phe Met Gly Ile Leu Asn
            265                 270                 275

cag aag tgt att aag cac aac tgt ggc ccc aac cct gca tcc aac aag 919
Gln Lys Cys Ile Lys His Asn Cys Gly Pro Asn Pro Ala Ser Asn Lys
        280                 285                 290

gat tgt ttt gaa aag gaa aaa gat agc gaa gac ttc ata atg tgt ggt 967
Asp Cys Phe Glu Lys Glu Lys Asp Ser Glu Asp Phe Ile Met Cys Gly
    295                 300                 305

acc tgg ctc ggc agc aga ccc tgt ccc aat ggt tct acg tgc gat aaa 1015
Thr Trp Leu Gly Ser Arg Pro Cys Pro Asn Gly Ser Thr Cys Asp Lys
310                 315                 320                 325

acc aca ttg aac cca gac aat aat tat aca aag ttt gac aac ttt ggc 1063
Thr Thr Leu Asn Pro Asp Asn Asn Tyr Thr Lys Phe Asp Asn Phe Gly
                330                 335                 340

tgg tcc ttt ctc gcc atg ttc cgg gtt atg act caa gac tcc tgg gag 1111
Trp Ser Phe Leu Ala Met Phe Arg Val Met Thr Gln Asp Ser Trp Glu
            345                 350                 355

agg ctt tac cga cag atc ctg cgg acc tct ggg atc tac ttt gtc ttc 1159
Arg Leu Tyr Arg Gln Ile Leu Arg Thr Ser Gly Ile Tyr Phe Val Phe
        360                 365                 370

ttc ttc gtg gtg gtc atc ttc ctg ggc tcc ttc tac ctg ctt aac cta 1207
Phe Phe Val Val Val Ile Phe Leu Gly Ser Phe Tyr Leu Leu Asn Leu
    375                 380                 385

acc ctg gct gtt gtc acc atg gct tat gaa gaa cag aac aga aat gta 1255
Thr Leu Ala Val Val Thr Met Ala Tyr Glu Glu Gln Asn Arg Asn Val
390                 395                 400                 405

gct gct gag aca gag gcc aag gag aaa atg ttt cag gaa gcc cag cag 1303
Ala Ala Glu Thr Glu Ala Lys Glu Lys Met Phe Gln Glu Ala Gln Gln
                410                 415                 420

ctg tta agg gag gag aag gag gct ctg gtt gcc atg gga att gac aga 1351
Leu Leu Arg Glu Glu Lys Glu Ala Leu Val Ala Met Gly Ile Asp Arg
            425                 430                 435

agt tcc ctt aat tcc ctt caa gct tca tcc ttt tcc ccg aag aag agg 1399
Ser Ser Leu Asn Ser Leu Gln Ala Ser Ser Phe Ser Pro Lys Lys Arg
        440                 445                 450

aag ttt ttc ggt agt aag aca aga aag tcc ttc ttt atg aga ggg tcc 1447

Lys Phe Phe Gly Ser Lys Thr Arg Lys Ser Phe Phe Met Arg Gly Ser
    455                 460                 465

aag acg gcc caa gcc tca gcg tct gat tca gag gac gat gcc tct aaa 1495
Lys Thr Ala Gln Ala Ser Ala Ser Asp Ser Glu Asp Asp Ala Ser Lys
470                 475                 480                 485

aat cca cag ctc ctt gag cag acc aaa cga ctg tcc cag aac ttg cca 1543
Asn Pro Gln Leu Leu Glu Gln Thr Lys Arg Leu Ser Gln Asn Leu Pro
                490                 495                 500

gtg gat ctc ttt gat gag cac gtg gac ccc ctc cac agg cag aga gcg 1591
Val Asp Leu Phe Asp Glu His Val Asp Pro Leu His Arg Gln Arg Ala
            505                 510                 515

ctg agc gct gtc agt atc tta acc atc acc atg cag gaa caa gaa aaa 1639
Leu Ser Ala Val Ser Ile Leu Thr Ile Thr Met Gln Glu Gln Glu Lys
        520                 525                 530

ttc cag gag cct tgt ttc cca tgt ggg aaa aat ttg gcc tct aag tac 1687
Phe Gln Glu Pro Cys Phe Pro Cys Gly Lys Asn Leu Ala Ser Lys Tyr
    535                 540                 545

ctg gtg tgg gac tgt agc cct caa tgg ctg tgc ata aag aag gtc ctg 1735
Leu Val Trp Asp Cys Ser Pro Gln Trp Leu Cys Ile Lys Lys Val Leu
550                 555                 560                 565

cgg acc atc atg acg gat ccc ttt act gag ctg gcc atc acc atc tgc 1783
Arg Thr Ile Met Thr Asp Pro Phe Thr Glu Leu Ala Ile Thr Ile Cys
                570                 575                 580

atc atc atc aat acc gtt ttc tta gcc gtg gag cac cac aac atg gat 1831
Ile Ile Ile Asn Thr Val Phe Leu Ala Val Glu His His Asn Met Asp
            585                 590                 595

gac aac tta aag acc ata ctg aaa ata gga aac tgg gtt ttc acg gga 1879
Asp Asn Leu Lys Thr Ile Leu Lys Ile Gly Asn Trp Val Phe Thr Gly
        600                 605                 610

att ttc ata gcg gaa atg tgt ctc aag atc atc gcg ctc gac cct tac 1927
Ile Phe Ile Ala Glu Met Cys Leu Lys Ile Ile Ala Leu Asp Pro Tyr
    615                 620                 625

cac tac ttc cgg cac ggc tgg aat gtt ttt gac agc atc gtg gcc ctc 1975
His Tyr Phe Arg His Gly Trp Asn Val Phe Asp Ser Ile Val Ala Leu
630                 635                 640                 645

ctg agt ctc gct gat gtg ctn tac aac aca ctg tct gat aac aat agg 2023
Leu Ser Leu Ala Asp Val Xaa Tyr Asn Thr Leu Ser Asp Asn Asn Arg
                650                 655                 660

tct ttc ttg gct tcc ctc aga gtg ctg agg gtc ttc aag tta gcc aaa 2071

Ser Phe Leu Ala Ser Leu Arg Val Leu Arg Val Phe Lys Leu Ala Lys
            665                 670                 675

tcc tgg ccc acg tta aac act ctc att aag atc atc ggc cac tcc gtg 2119
Ser Trp Pro Thr Leu Asn Thr Leu Ile Lys Ile Ile Gly His Ser Val
        680                 685                 690

ggc gcg ctt gga aac ctg act gtg gtc ctg act atc gtg gtc ttc atc 2167
Gly Ala Leu Gly Asn Leu Thr Val Val Leu Thr Ile Val Val Phe Ile
    695                 700                 705

ttt tct gtg gtg ggc atg cgg ctc ttc ggc acc aag ttt aac aag acc 2215
Phe Ser Val Val Gly Met Arg Leu Phe Gly Thr Lys Phe Asn Lys Thr
710                 715                 720                 725

gcc tac gcc acc cag gag cgg ccc agg cgg cgc tgg cac atg gat aat 2263
Ala Tyr Ala Thr Gln Glu Arg Pro Arg Arg Arg Trp His Met Asp Asn
                730                 735                 740

ttc tac cac tcc ttc ctg gtg gtg ttc cgc atc ctc tgt ggg gaa tgg 2311
Phe Tyr His Ser Phe Leu Val Val Phe Arg Ile Leu Cys Gly Glu Trp
            745                 750                 755

atc gag aac atg tgg ggc tgc atg cag gat atg gac ggc tcc ccg ttg 2359
Ile Glu Asn Met Trp Gly Cys Met Gln Asp Met Asp Gly Ser Pro Leu
        760                 765                 770

tgc atc att gtc ttt gtc ctg ata atg gtg atc ggg aag ctt gtg gtg 2407
Cys Ile Ile Val Phe Val Leu Ile Met Val Ile Gly Lys Leu Val Val
    775                 780                 785

ctt aac ctc ttc att gcc ttg ctg ctc aat tcc ttc agc aat gag gag 2455
Leu Asn Leu Phe Ile Ala Leu Leu Leu Asn Ser Phe Ser Asn Glu Glu
790                 795                 800                 805

aag gat ggg agc ctg gaa gga gag acc agg aaa acc aaa gtg cag cta 2503
Lys Asp Gly Ser Leu Glu Gly Glu Thr Arg Lys Thr Lys Val Gln Leu
                810                 815                 820

gcc ctg gat cgg ttc cgc cgg gcc ttc tcc ttc atg ctg cac gct ctt 2551
Ala Leu Asp Arg Phe Arg Arg Ala Phe Ser Phe Met Leu His Ala Leu
            825                 830                 835

cag agt ttt tgt tgc aag aaa tgc agg agg aaa aac tcg cca aag cca 2599
Gln Ser Phe Cys Cys Lys Lys Cys Arg Arg Lys Asn Ser Pro Lys Pro
        840                 845                 850

aaa gag aca aca gaa agc ttt gct ggt gag aat aaa gac tca atc ctc 2647
Lys Glu Thr Thr Glu Ser Phe Ala Gly Glu Asn Lys Asp Ser Ile Leu
    855                 860                 865

ccg gat gcg agg ccc tgg aag gag tat gat aca gac atg gct ttg tac 2695

Pro Asp Ala Arg Pro Trp Lys Glu Tyr Asp Thr Asp Met Ala Leu Tyr
870                 875                 880                 885

act gga cag gcc ggg gct ccg ctg gcc cca ctc gca gag gta gag gac     2743
Thr Gly Gln Ala Gly Ala Pro Leu Ala Pro Leu Ala Glu Val Glu Asp
                890                 895                 900

gat gtg gaa tat tgt ggt gaa ggc ggt gcc cta ccc acc tca caa cat     2791
Asp Val Glu Tyr Cys Gly Glu Gly Gly Ala Leu Pro Thr Ser Gln His
            905                 910                 915

agt gct gga gtt cag gcc ggt gac ctc cct cca gag acc aag cag ctc     2839
Ser Ala Gly Val Gln Ala Gly Asp Leu Pro Pro Glu Thr Lys Gln Leu
        920                 925                 930

act agc ccg gat gac caa ggg gtt gaa atg gaa gta ttt tct gaa gaa     2887
Thr Ser Pro Asp Asp Gln Gly Val Glu Met Glu Val Phe Ser Glu Glu
    935                 940                 945

gat ctg cat tta agc ata cag agt cct cga aag aag tct gac gca gtg     2935
Asp Leu His Leu Ser Ile Gln Ser Pro Arg Lys Lys Ser Asp Ala Val
950                 955                 960                 965

agc atg ctc tcg gaa tgc agc aca att gac ctg aat gat atc ttt aga     2983
Ser Met Leu Ser Glu Cys Ser Thr Ile Asp Leu Asn Asp Ile Phe Arg
                970                 975                 980

aat tta cag aaa aca gtt tcc ccc aaa aag cag cca gat aga tgc ttt     3031
Asn Leu Gln Lys Thr Val Ser Pro Lys Lys Gln Pro Asp Arg Cys Phe
            985                 990                 995

ccc aag ggc ctt agt tgt cac ttt cta tgc cac aaa aca gac aag aga     3079
Pro Lys Gly Leu Ser Cys His Phe Leu Cys His Lys Thr Asp Lys Arg
        1000                1005                1010

aag tcc ccc tgg gtc ctg tgg tgg aac att cgg aaa acc tgc tac caa     3127
Lys Ser Pro Trp Val Leu Trp Trp Asn Ile Arg Lys Thr Cys Tyr Gln
    1015                1020                1025

atc gtg aag cac agc tgg ttt gag agt ttc ata atc ttt gtt att ctg     3175
Ile Val Lys His Ser Trp Phe Glu Ser Phe Ile Ile Phe Val Ile Leu
1030                1035                1040                1045

ctg agc agt gga gcg ctg ata ttt gaa gat gtc aat ctc ccc agc cgg     3223
Leu Ser Ser Gly Ala Leu Ile Phe Glu Asp Val Asn Leu Pro Ser Arg
                1050                1055                1060

ccc caa gtt gag aaa tta cta agg tgt acc gat aat att ttc aca ttt     3271
Pro Gln Val Glu Lys Leu Leu Arg Cys Thr Asp Asn Ile Phe Thr Phe
            1065                1070                1075

att ttc ctc ctg gaa atg atc ctg aag tgg gtg gcc ttt gga ttc cgg     3319

Ile Phe Leu Leu Glu Met Ile Leu Lys Trp Val Ala Phe Gly Phe Arg
        1080                1085                1090

agg tat ttc acc agt gcc tgg tgc tgg ctt gat ttc ctc att gtg gtg 3367
Arg Tyr Phe Thr Ser Ala Trp Cys Trp Leu Asp Phe Leu Ile Val Val
    1095                1100                1105

gtg tct gtg ctc agt ctc atg aat cta cca agc ttg aag tcc ttc cgg 3415
Val Ser Val Leu Ser Leu Met Asn Leu Pro Ser Leu Lys Ser Phe Arg
1110                1115                1120                1125

act ctg cgg gcc ctg aga cct ctg cgg gcg ctg tcc cag ttt gaa gga 3463
Thr Leu Arg Ala Leu Arg Pro Leu Arg Ala Leu Ser Gln Phe Glu Gly
                1130                1135                1140

atg aag gtt gtc gtc tac gcc ctg atc agc gcc ata cct gcc att ctc 3511
Met Lys Val Val Val Tyr Ala Leu Ile Ser Ala Ile Pro Ala Ile Leu
            1145                1150                1155

aat gtc ttg ctg gtc tgc ctc att ttc tgg ctc gta ttt tgt atc ttg 3559
Asn Val Leu Leu Val Cys Leu Ile Phe Trp Leu Val Phe Cys Ile Leu
        1160                1165                1170

gga gta aat tta ttt tct ggg aag ttt gga agg tgc att aac ggg aca 3607
Gly Val Asn Leu Phe Ser Gly Lys Phe Gly Arg Cys Ile Asn Gly Thr
    1175                1180                1185

gac ata aat atg tat ttg gat ttt acc gaa gtt ccg aac cga agc caa 3655
Asp Ile Asn Met Tyr Leu Asp Phe Thr Glu Val Pro Asn Arg Ser Gln
1190                1195                1200                1205

tgt aac att agt aat tac tcg tgg aag gtc ccg cag gtc aac ttt gac 3703
Cys Asn Ile Ser Asn Tyr Ser Trp Lys Val Pro Gln Val Asn Phe Asp
                1210                1215                1220

aac gtg ggg aat gcc tat ctc gcc ctg ctg caa gtg gca acc tat aag 3751
Asn Val Gly Asn Ala Tyr Leu Ala Leu Leu Gln Val Ala Thr Tyr Lys
            1225                1230                1235

ggc tgg ctg gaa atc atg aat gct gct gtc gat tcc aga gag aaa gac 3799
Gly Trp Leu Glu Ile Met Asn Ala Ala Val Asp Ser Arg Glu Lys Asp
        1240                1245                1250

gag cag ccg gac ttt gag gcg aac ctc tac gcg tat ctc tac ttt gtg 3847
Glu Gln Pro Asp Phe Glu Ala Asn Leu Tyr Ala Tyr Leu Tyr Phe Val
    1255                1260                1265

gtt ttt atc atc ttc ggc tcc ttc ttt acc ctg aac ctc ttt atc ggt 3895
Val Phe Ile Ile Phe Gly Ser Phe Phe Thr Leu Asn Leu Phe Ile Gly
1270                1275                1280                1285

gtt att att gac aac ttc aat cag cag cag aaa aag tta ggt ggc caa 3943

Val Ile Ile Asp Asn Phe Asn Gln Gln Gln Lys Lys Leu Gly Gly Gln
                1290                1295                1300

gac att ttt atg aca gaa gaa cag aag aaa tat tac aat gca atg aaa 3991
Asp Ile Phe Met Thr Glu Glu Gln Lys Lys Tyr Tyr Asn Ala Met Lys
            1305                1310                1315

aag tta gga acc aag aaa cct caa aag ccc atc cca agg ccc ctg aac 4039
Lys Leu Gly Thr Lys Lys Pro Gln Lys Pro Ile Pro Arg Pro Leu Asn
        1320                1325                1330

aan tgt caa gcc ttt gtg ttc gac ctg gtc aca agc cat gtc ttt gac 4087
Xaa Cys Gln Ala Phe Val Phe Asp Leu Val Thr Ser His Val Phe Asp
    1335                1340                1345

gtc atc att ctg ggt ctt att gtc tta aat atg att atc atg atg gct 4135
Val Ile Ile Leu Gly Leu Ile Val Leu Asn Met Ile Ile Met Met Ala
1350                1355                1360                1365

gaa tct gcc gac cag ccc aaa gat gtg aag aaa acc ttt gat atc ctc 4183
Glu Ser Ala Asp Gln Pro Lys Asp Val Lys Lys Thr Phe Asp Ile Leu
                1370                1375                1380

aac ata gcc ttc gtg gtc atc ttt acc ata gag tgt ctc atc aaa gtc 4231
Asn Ile Ala Phe Val Val Ile Phe Thr Ile Glu Cys Leu Ile Lys Val
            1385                1390                1395

ttt gct ttg agg caa cac tac ttc acc aat ggc tgg aac tta ttt gat 4279
Phe Ala Leu Arg Gln His Tyr Phe Thr Asn Gly Trp Asn Leu Phe Asp
        1400                1405                1410

tgt gtg gtc gtg gtt ctt tct atc att agt acc ctg gtt tcc cgc ttg 4327
Cys Val Val Val Val Leu Ser Ile Ile Ser Thr Leu Val Ser Arg Leu
    1415                1420                1425

gag gac agt gac att tct ttc ccg ccc acg ctc ttc aga gtc gtc cgc 4375
Glu Asp Ser Asp Ile Ser Phe Pro Pro Thr Leu Phe Arg Val Val Arg
1430                1435                1440                1445

ttg gct cgg att ggt cga atc ctc agg ctg gtc cgg gct gcc cgg gga 4423
Leu Ala Arg Ile Gly Arg Ile Leu Arg Leu Val Arg Ala Ala Arg Gly
                1450                1455                1460

atc agg acc ctc ctc ttt gct ttg atg atg tct ctc ccc tct ctc ttc 4471
Ile Arg Thr Leu Leu Phe Ala Leu Met Met Ser Leu Pro Ser Leu Phe
            1465                1470                1475

aac atc ggt ctg ctg ctc ttc ctg gtg atg ttc att tac gcc atc ttt 4519
Asn Ile Gly Leu Leu Leu Phe Leu Val Met Phe Ile Tyr Ala Ile Phe
        1480                1485                1490

ggg atg agc tgg ttt tcc aaa gtg aag aag ggc tcc ggg atc gac gac 4567

Gly Met Ser Trp Phe Ser Lys Val Lys Lys Gly Ser Gly Ile Asp Asp
    1495                1500                1505

atc ttc aac ttc gag acc ttt acg ggc agc atg ctg tgc ctc ttc cag 4615
Ile Phe Asn Phe Glu Thr Phe Thr Gly Ser Met Leu Cys Leu Phe Gln
1510                1515                1520                1525

ata acc act tcg gct ggc tgg gat acc ctc ctc aac ccc atg ctg gag 4663
Ile Thr Thr Ser Ala Gly Trp Asp Thr Leu Leu Asn Pro Met Leu Glu
                1530                1535                1540

gca aaa gaa cac tgc aac tcc tcc tcc caa gac agc tgt cag cag ccg 4711
Ala Lys Glu His Cys Asn Ser Ser Ser Gln Asp Ser Cys Gln Gln Pro
            1545                1550                1555

cag ata gcc gtc gtc tac ttc gtc agt tac atc atc atc tcc ttc ctc 4759
Gln Ile Ala Val Val Tyr Phe Val Ser Tyr Ile Ile Ile Ser Phe Leu
        1560                1565                1570

atc gtg gtc aac atg tac atc gct gtg atc ctc gag aac ttc aac aca 4807
Ile Val Val Asn Met Tyr Ile Ala Val Ile Leu Glu Asn Phe Asn Thr
    1575                1580                1585

gcc acg gag gag agc gag gac cct ctg gga gag gac gac ttt gaa atc 4855
Ala Thr Glu Glu Ser Glu Asp Pro Leu Gly Glu Asp Asp Phe Glu Ile
1590                1595                1600                1605

ttc tat gag gtc tgg gag aag ttt gac ccc gag gcg tcg cag ttc atc 4903
Phe Tyr Glu Val Trp Glu Lys Phe Asp Pro Glu Ala Ser Gln Phe Ile
                1610                1615                1620

cag tat tcg gcc ctc tct gac ttt gcg gac gcc ctg ccg gag ccg ttg 4951
Gln Tyr Ser Ala Leu Ser Asp Phe Ala Asp Ala Leu Pro Glu Pro Leu
            1625                1630                1635

cgt gtg gcc aag ccg aat aag ttt cag ttt cta gtg atg gac ttg ccc 4999
Arg Val Ala Lys Pro Asn Lys Phe Gln Phe Leu Val Met Asp Leu Pro
        1640                1645                1650

atg gtg atg ggc gac cgc ctc cat tgc atg gat gtt ctc ttt gct ttc 5047
Met Val Met Gly Asp Arg Leu His Cys Met Asp Val Leu Phe Ala Phe
    1655                1660                1665

act acc agg gtc ctc ggg gac tcc agc ggc ttg gat acc atg aaa acc 5095
Thr Thr Arg Val Leu Gly Asp Ser Ser Gly Leu Asp Thr Met Lys Thr
1670                1675                1680                1685

atg atg gag gag aag ttt atg gag gcc aac cct ttt aag aag ctc tac 5143
Met Met Glu Glu Lys Phe Met Glu Ala Asn Pro Phe Lys Lys Leu Tyr
                1690                1695                1700

gag ccc ata gtc acc acc acc aag agg aag gag gag gag caa ggc gcc 5191

Glu Pro Ile Val Thr Thr Thr Lys Arg Lys Glu Glu Glu Gln Gly Ala
        1705                1710                1715

gcc gtc atc cag agg gcc tac cgg aaa cac atg gag aag atg gtc aaa    5239
Ala Val Ile Gln Arg Ala Tyr Arg Lys His Met Glu Lys Met Val Lys
    1720                1725                1730

ctg agg ctg aag gac agg tca agt tca tcg cac cag gtg ttt tgc aat    5287
Leu Arg Leu Lys Asp Arg Ser Ser Ser Ser His Gln Val Phe Cys Asn
  1735                1740                1745

gga gac ttg tcc agc ttg gat gtg gcc aag gtc aag gtt cac aat gac    5335
Gly Asp Leu Ser Ser Leu Asp Val Ala Lys Val Lys Val His Asn Asp
1750                1755                1760                1765

tgaaccctca tctccacccc tacctcactg cctcacagct tagcctccag cctctggcga 5395

gcaggcggca gactcactga acacaggccg ttcgatctgt gtttttggct gaacgaggtg 5455

acaggttggc gtccattttt aaatgactct tggaaagatt tcatgtagag agatgttaga 5515

agggactgca aaggacaccg accataacgg aaggcctgga ggacagtcca acttacataa 5575

agatgagaaa caagaaggaa agatcccagg aaaacttcag attgtgttct cagtacattc 5635

cccaatgtgt ctgttcggtg ttttgagtat gtgacctgcc acatgtagct ctttttgca 5695

tgtacgtcaa aaccctgcag taagttaata gcttgctacg ggtgttccta ccagcatcac 5755

agaattgggt gtatgactca aacctaaaag catgactctg acttgtcagt cagcaccccg 5815

actttcagac gctccaatct ctgtcccagg tgtctaacga ataaataggt aaaagaaaaa 5875

<210> 2
<211> 1765
<212> PRT
<213> Rattus norvegicus

<220>
<221> unsure
<222> (652), (1334)

<223> Xaa at 652 = Leu; Xaa at 1334 = Asn or Lys

<400> 2
Met Glu Glu Arg Tyr Tyr Pro Val Ile Phe Pro Asp Glu Arg Asn Phe
  1               5                  10                  15

Arg Pro Phe Thr Ser Asp Ser Leu Ala Ala Ile Glu Lys Arg Ile Ala

20                  25                  30

Ile Gln Lys Glu Arg Lys Lys Ser Lys Asp Lys Ala Ala Ala Glu Pro
        35                  40                  45

Gln Pro Arg Pro Gln Leu Asp Leu Lys Ala Ser Arg Lys Leu Pro Lys
    50                  55                  60

Leu Tyr Gly Asp Ile Pro Pro Glu Leu Val Ala Lys Pro Leu Glu Asp
65                  70                  75                  80

Leu Asp Pro Phe Tyr Lys Asp His Lys Thr Phe Met Val Leu Asn Lys
                85                  90                  95

Lys Arg Thr Ile Tyr Arg Phe Ser Ala Lys Arg Ala Leu Phe Ile Leu
            100                 105                 110

Gly Pro Phe Asn Pro Leu Arg Ser Leu Met Ile Arg Ile Ser Val His
        115                 120                 125

Ser Val Phe Ser Met Phe Ile Ile Cys Thr Val Ile Ile Asn Cys Met
    130                 135                 140

Phe Met Ala Asn Ser Met Glu Arg Ser Phe Asp Asn Asp Ile Pro Glu
145                 150                 155                 160

Tyr Val Phe Ile Gly Ile Tyr Ile Leu Glu Ala Val Ile Lys Ile Leu
                165                 170                 175

Ala Arg Gly Phe Ile Val Asp Glu Phe Ser Phe Leu Arg Asp Pro Trp
            180                 185                 190

Asn Trp Leu Asp Phe Ile Val Ile Gly Thr Ala Ile Ala Thr Cys Phe
        195                 200                 205

Pro Gly Ser Gln Val Asn Leu Ser Ala Leu Arg Thr Phe Arg Val Phe
    210                 215                 220

Arg Ala Leu Lys Ala Ile Ser Val Ile Ser Gly Leu Lys Val Ile Val
225                 230                 235                 240

Gly Ala Leu Leu Arg Ser Val Lys Lys Leu Val Asp Val Met Val Leu
                245                 250                 255

Thr Leu Phe Cys Leu Ser Ile Phe Ala Leu Val Gly Gln Gln Leu Phe
            260                 265                 270

Met Gly Ile Leu Asn Gln Lys Cys Ile Lys His Asn Cys Gly Pro Asn
        275                 280                 285

Pro Ala Ser Asn Lys Asp Cys Phe Glu Lys Glu Lys Asp Ser Glu Asp
    290                 295                 300

Phe Ile Met Cys Gly Thr Trp Leu Gly Ser Arg Pro Cys Pro Asn Gly
305                 310                 315                 320

Ser Thr Cys Asp Lys Thr Thr Leu Asn Pro Asp Asn Asn Tyr Thr Lys
                325                 330                 335

Phe Asp Asn Phe Gly Trp Ser Phe Leu Ala Met Phe Arg Val Met Thr
            340                 345                 350

Gln Asp Ser Trp Glu Arg Leu Tyr Arg Gln Ile Leu Arg Thr Ser Gly
        355                 360                 365

Ile Tyr Phe Val Phe Phe Phe Val Val Val Ile Phe Leu Gly Ser Phe
    370                 375                 380

Tyr Leu Leu Asn Leu Thr Leu Ala Val Val Thr Met Ala Tyr Glu Glu
385                 390                 395                 400

Gln Asn Arg Asn Val Ala Ala Glu Thr Glu Ala Lys Glu Lys Met Phe
                405                 410                 415

Gln Glu Ala Gln Gln Leu Leu Arg Glu Glu Lys Glu Ala Leu Val Ala
            420                 425                 430

Met Gly Ile Asp Arg Ser Ser Leu Asn Ser Leu Gln Ala Ser Ser Phe
        435                 440                 445

Ser Pro Lys Lys Arg Lys Phe Phe Gly Ser Lys Thr Arg Lys Ser Phe
    450                 455                 460

Phe Met Arg Gly Ser Lys Thr Ala Gln Ala Ser Ala Ser Asp Ser Glu
465                 470                 475                 480

Asp Asp Ala Ser Lys Asn Pro Gln Leu Leu Glu Gln Thr Lys Arg Leu
                485                 490                 495

Ser Gln Asn Leu Pro Val Asp Leu Phe Asp Glu His Val Asp Pro Leu
            500                 505                 510

His Arg Gln Arg Ala Leu Ser Ala Val Ser Ile Leu Thr Ile Thr Met
        515                 520                 525

Gln Glu Gln Glu Lys Phe Gln Glu Pro Cys Phe Pro Cys Gly Lys Asn
    530                 535                 540

Leu Ala Ser Lys Tyr Leu Val Trp Asp Cys Ser Pro Gln Trp Leu Cys
545                 550                 555                 560

Ile Lys Lys Val Leu Arg Thr Ile Met Thr Asp Pro Phe Thr Glu Leu
                565                 570                 575

Ala Ile Thr Ile Cys Ile Ile Ile Asn Thr Val Phe Leu Ala Val Glu

            580                 585                 590

His His Asn Met Asp Asp Asn Leu Lys Thr Ile Leu Lys Ile Gly Asn
        595                 600                 605

Trp Val Phe Thr Gly Ile Phe Ile Ala Glu Met Cys Leu Lys Ile Ile
    610                 615                 620

Ala Leu Asp Pro Tyr His Tyr Phe Arg His Gly Trp Asn Val Phe Asp
625                 630                 635                 640

Ser Ile Val Ala Leu Leu Ser Leu Ala Asp Val Xaa Tyr Asn Thr Leu
                645                 650                 655

Ser Asp Asn Asn Arg Ser Phe Leu Ala Ser Leu Arg Val Leu Arg Val
            660                 665                 670

Phe Lys Leu Ala Lys Ser Trp Pro Thr Leu Asn Thr Leu Ile Lys Ile
        675                 680                 685

Ile Gly His Ser Val Gly Ala Leu Gly Asn Leu Thr Val Val Leu Thr
    690                 695                 700

Ile Val Val Phe Ile Phe Ser Val Val Gly Met Arg Leu Phe Gly Thr
705                 710                 715                 720

Lys Phe Asn Lys Thr Ala Tyr Ala Thr Gln Glu Arg Pro Arg Arg Arg
                725                 730                 735

Trp His Met Asp Asn Phe Tyr His Ser Phe Leu Val Val Phe Arg Ile
            740                 745                 750

Leu Cys Gly Glu Trp Ile Glu Asn Met Trp Gly Cys Met Gln Asp Met
        755                 760                 765

Asp Gly Ser Pro Leu Cys Ile Ile Val Phe Val Leu Ile Met Val Ile
    770                 775                 780

Gly Lys Leu Val Val Leu Asn Leu Phe Ile Ala Leu Leu Leu Asn Ser
785                 790                 795                 800

Phe Ser Asn Glu Glu Lys Asp Gly Ser Leu Glu Gly Glu Thr Arg Lys
                805                 810                 815

Thr Lys Val Gln Leu Ala Leu Asp Arg Phe Arg Arg Ala Phe Ser Phe
            820                 825                 830

Met Leu His Ala Leu Gln Ser Phe Cys Cys Lys Lys Cys Arg Arg Lys
        835                 840                 845

Asn Ser Pro Lys Pro Lys Glu Thr Thr Glu Ser Phe Ala Gly Glu Asn
    850                 855                 860

Lys Asp Ser Ile Leu Pro Asp Ala Arg Pro Trp Lys Glu Tyr Asp Thr
865                 870                 875                 880

Asp Met Ala Leu Tyr Thr Gly Gln Ala Gly Ala Pro Leu Ala Pro Leu
                885                 890                 895

Ala Glu Val Glu Asp Asp Val Glu Tyr Cys Gly Glu Gly Gly Ala Leu
            900                 905                 910

Pro Thr Ser Gln His Ser Ala Gly Val Gln Ala Gly Asp Leu Pro Pro
        915                 920                 925

Glu Thr Lys Gln Leu Thr Ser Pro Asp Asp Gln Gly Val Glu Met Glu
    930                 935                 940

Val Phe Ser Glu Glu Asp Leu His Leu Ser Ile Gln Ser Pro Arg Lys
945                 950                 955                 960

Lys Ser Asp Ala Val Ser Met Leu Ser Glu Cys Ser Thr Ile Asp Leu
                965                 970                 975

Asn Asp Ile Phe Arg Asn Leu Gln Lys Thr Val Ser Pro Lys Lys Gln
            980                 985                 990

Pro Asp Arg Cys Phe Pro Lys Gly Leu Ser Cys His Phe Leu Cys His
        995                 1000                1005

Lys Thr Asp Lys Arg Lys Ser Pro Trp Val Leu Trp Trp Asn Ile Arg
    1010                1015                1020

Lys Thr Cys Tyr Gln Ile Val Lys His Ser Trp Phe Glu Ser Phe Ile
025 1030 1035 1040

Ile Phe Val Ile Leu Leu Ser Ser Gly Ala Leu Ile Phe Glu Asp Val
                1045                1050                1055

Asn Leu Pro Ser Arg Pro Gln Val Glu Lys Leu Leu Arg Cys Thr Asp
            1060                1065                1070

Asn Ile Phe Thr Phe Ile Phe Leu Leu Glu Met Ile Leu Lys Trp Val
        1075                1080                1085

Ala Phe Gly Phe Arg Arg Tyr Phe Thr Ser Ala Trp Cys Trp Leu Asp
    1090                1095                1100

Phe Leu Ile Val Val Val Ser Val Leu Ser Leu Met Asn Leu Pro Ser
105 1110 1115 1120

Leu Lys Ser Phe Arg Thr Leu Arg Ala Leu Arg Pro Leu Arg Ala Leu
                1125                1130                1135

Ser Gln Phe Glu Gly Met Lys Val Val Val Tyr Ala Leu Ile Ser Ala

1140                    1145                    1150

Ile Pro Ala Ile Leu Asn Val Leu Leu Val Cys Leu Ile Phe Trp Leu
        1155                    1160                    1165

Val Phe Cys Ile Leu Gly Val Asn Leu Phe Ser Gly Lys Phe Gly Arg
    1170                    1175                    1180

Cys Ile Asn Gly Thr Asp Ile Asn Met Tyr Leu Asp Phe Thr Glu Val
185                     1190                    1195                    1200

Pro Asn Arg Ser Gln Cys Asn Ile Ser Asn Tyr Ser Trp Lys Val Pro
                1205                    1210                    1215

Gln Val Asn Phe Asp Asn Val Gly Asn Ala Tyr Leu Ala Leu Leu Gln
            1220                    1225                    1230

Val Ala Thr Tyr Lys Gly Trp Leu Glu Ile Met Asn Ala Ala Val Asp
        1235                    1240                    1245

Ser Arg Glu Lys Asp Glu Gln Pro Asp Phe Glu Ala Asn Leu Tyr Ala
    1250                    1255                    1260

Tyr Leu Tyr Phe Val Val Phe Ile Ile Phe Gly Ser Phe Phe Thr Leu
265                     1270                    1275                    1280

Asn Leu Phe Ile Gly Val Ile Ile Asp Asn Phe Asn Gln Gln Gln Lys
                1285                    1290                    1295

Lys Leu Gly Gly Gln Asp Ile Phe Met Thr Glu Glu Gln Lys Lys Tyr
            1300                    1305                    1310

Tyr Asn Ala Met Lys Lys Leu Gly Thr Lys Lys Pro Gln Lys Pro Ile
        1315                    1320                    1325

Pro Arg Pro Leu Asn Xaa Cys Gln Ala Phe Val Phe Asp Leu Val Thr
    1330                    1335                    1340

Ser His Val Phe Asp Val Ile Ile Leu Gly Leu Ile Val Leu Asn Met
345                     1350                    1355                    1360

Ile Ile Met Met Ala Glu Ser Ala Asp Gln Pro Lys Asp Val Lys Lys
                1365                    1370                    1375

Thr Phe Asp Ile Leu Asn Ile Ala Phe Val Val Ile Phe Thr Ile Glu
            1380                    1385                    1390

Cys Leu Ile Lys Val Phe Ala Leu Arg Gln His Tyr Phe Thr Asn Gly
        1395                    1400                    1405

Trp Asn Leu Phe Asp Cys Val Val Val Leu Ser Ile Ile Ser Thr
    1410                    1415                    1420

Leu Val Ser Arg Leu Glu Asp Ser Asp Ile Ser Phe Pro Pro Thr Leu
425                 1430                1435                1440

Phe Arg Val Val Arg Leu Ala Arg Ile Gly Arg Ile Leu Arg Leu Val
                1445                1450                1455

Arg Ala Ala Arg Gly Ile Arg Thr Leu Leu Phe Ala Leu Met Met Ser
            1460                1465                1470

Leu Pro Ser Leu Phe Asn Ile Gly Leu Leu Leu Phe Leu Val Met Phe
        1475                1480                1485

Ile Tyr Ala Ile Phe Gly Met Ser Trp Phe Ser Lys Val Lys Lys Gly
    1490                1495                1500

Ser Gly Ile Asp Asp Ile Phe Asn Phe Glu Thr Phe Thr Gly Ser Met
505                 1510                1515                1520

Leu Cys Leu Phe Gln Ile Thr Thr Ser Ala Gly Trp Asp Thr Leu Leu
                1525                1530                1535

Asn Pro Met Leu Glu Ala Lys Glu His Cys Asn Ser Ser Ser Gln Asp
            1540                1545                1550

Ser Cys Gln Gln Pro Gln Ile Ala Val Val Tyr Phe Val Ser Tyr Ile
        1555                1560                1565

Ile Ile Ser Phe Leu Ile Val Val Asn Met Tyr Ile Ala Val Ile Leu
    1570                1575                1580

Glu Asn Phe Asn Thr Ala Thr Glu Glu Ser Glu Asp Pro Leu Gly Glu
585                 1590                1595                1600

Asp Asp Phe Glu Ile Phe Tyr Glu Val Trp Glu Lys Phe Asp Pro Glu
                1605                1610                1615

Ala Ser Gln Phe Ile Gln Tyr Ser Ala Leu Ser Asp Phe Ala Asp Ala
            1620                1625                1630

Leu Pro Glu Pro Leu Arg Val Ala Lys Pro Asn Lys Phe Gln Phe Leu
        1635                1640                1645

Val Met Asp Leu Pro Met Val Met Gly Asp Arg Leu His Cys Met Asp
    1650                1655                1660

Val Leu Phe Ala Phe Thr Thr Arg Val Leu Gly Asp Ser Ser Gly Leu
665                 1670                1675                1680

Asp Thr Met Lys Thr Met Met Glu Glu Lys Phe Met Glu Ala Asn Pro
                1685                1690                1695

Phe Lys Lys Leu Tyr Glu Pro Ile Val Thr Thr Thr Lys Arg Lys Glu

1700                    1705                    1710

Glu Glu Gln Gly Ala Ala Val Ile Gln Arg Ala Tyr Arg Lys His Met
        1715                    1720                    1725

Glu Lys Met Val Lys Leu Arg Leu Lys Asp Arg Ser Ser Ser Ser His
    1730                    1735                    1740

Gln Val Phe Cys Asn Gly Asp Leu Ser Ser Leu Asp Val Ala Lys Val
745                     1750                    1755                    1760

Lys Val His Asn Asp
                1765


<210> 3
<211> 1765
<212> PRT
<213> Rattus norvegicus

<220>
<223> putative amino acid seq. of rat NaN

<400> 3
   Met Glu Glu Arg Tyr Tyr Pro Val Ile Phe Pro Asp Glu Arg Asn Phe
     1               5                  10                  15
   Arg Pro Phe Thr Ser Asp Ser Leu Ala Ala Ile Glu Lys Arg Ile Ala
                20                  25                  30
   Ile Gln Lys Glu Arg Lys Lys Ser Lys Asp Lys Ala Ala Ala Glu Pro
            35                  40                  45
   Gln Pro Arg Pro Gln Leu Asp Leu Lys Ala Ser Arg Lys Leu Pro Lys
        50                  55                  60
   Leu Tyr Gly Asp Ile Pro Pro Glu Leu Val Ala Lys Pro Leu Glu Asp
    65                  70                  75                  80
   Leu Asp Pro Phe Tyr Lys Asp His Lys Thr Phe Met Val Leu Asn Lys
                    85                  90                  95
   Lys Arg Thr Ile Tyr Arg Phe Ser Ala Lys Arg Ala Leu Phe Ile Leu
               100                 105                 110
   Gly Pro Phe Asn Pro Leu Arg Ser Leu Met Ile Arg Ile Ser Val His
           115                 120                 125
   Ser Val Phe Ser Met Phe Ile Ile Cys Thr Val Ile Ile Asn Cys Met
       130                 135                 140
   Phe Met Ala Asn Ser Met Glu Arg Ser Phe Asp Asn Asp Ile Pro Glu
   145                 150                 155                 160
   Tyr Val Phe Ile Gly Ile Tyr Ile Leu Glu Ala Val Ile Lys Ile Leu
                   165                 170                 175
   Ala Arg Gly Phe Ile Val Asp Glu Phe Ser Phe Leu Arg Asp Pro Trp
               180                 185                 190
   Asn Trp Leu Asp Phe Ile Val Ile Gly Thr Ala Ile Ala Thr Cys Phe
           195                 200                 205
   Pro Gly Ser Gln Val Asn Leu Ser Ala Leu Arg Thr Phe Arg Val Phe
       210                 215                 220
   Arg Ala Leu Lys Ala Ile Ser Val Ile Ser Gly Leu Lys Val Ile Val
   225                 230                 235                 240
   Gly Ala Leu Leu Arg Ser Val Lys Lys Leu Val Asp Val Met Val Leu
                   245                 250                 255
   Thr Leu Phe Cys Leu Ser Ile Phe Ala Leu Val Gly Gln Gln Leu Phe
               260                 265                 270
   Met Gly Ile Leu Asn Gln Lys Cys Ile Lys His Asn Cys Gly Pro Asn
           275                 280                 285
   Pro Ala Ser Asn Lys Asp Cys Phe Glu Lys Glu Lys Asp Ser Glu Asp
       290                 295                 300
   Phe Ile Met Cys Gly Thr Trp Leu Gly Ser Arg Pro Cys Pro Asn Gly
   305                 310                 315                 320
   Ser Thr Cys Asp Lys Thr Thr Leu Asn Pro Asp Asn Asn Tyr Thr Lys
                   325                 330                 335
   Phe Asp Asn Phe Gly Trp Ser Phe Leu Ala Met Phe Arg Val Met Thr
               340                 345                 350
   Gln Asp Ser Trp Glu Arg Leu Tyr Arg Gln Ile Leu Arg Thr Ser Gly
           355                 360                 365
   Ile Tyr Phe Val Phe Phe Phe Val Val Val Ile Phe Leu Gly Ser Phe
       370                 375                 380
   Tyr Leu Leu Asn Leu Thr Leu Ala Val Val Thr Met Ala Tyr Glu Glu
   385                 390                 395                 400
   Gln Asn Arg Asn Val Ala Ala Glu Thr Glu Ala Lys Glu Lys Met Phe
                   405                 410                 415
   Gln Glu Ala Gln Gln Leu Leu Arg Glu Glu Lys Glu Ala Leu Val Ala
               420                 425                 430
   Met Gly Ile Asp Arg Ser Ser Leu Asn Ser Leu Gln Ala Ser Ser Phe
           435                 440                 445
   Ser Pro Lys Lys Arg Lys Phe Phe Gly Ser Lys Thr Arg Lys Ser Phe
       450                 455                 460
   Phe Met Arg Gly Ser Lys Thr Ala Gln Ala Ser Ala Ser Asp Ser Glu
   465                 470                 475                 480
   Asp Asp Ala Ser Lys Asn Pro Gln Leu Leu Glu Gln Thr Lys Arg Leu
                   485                 490                 495
   Ser Gln Asn Leu Pro Val Asp Leu Phe Asp Glu His Val Asp Pro Leu
               500                 505                 510
   His Arg Gln Arg Ala Leu Ser Ala Val Ser Ile Leu Thr Ile Thr Met
           515                 520                 525
   Gln Glu Gln Glu Lys Phe Gln Glu Pro Cys Phe Pro Cys Gly Lys Asn
       530                 535                 540
   Leu Ala Ser Lys Tyr Leu Val Trp Asp Cys Ser Pro Gln Trp Leu Cys
   545                 550                 555                 560
   Ile Lys Lys Val Leu Arg Thr Ile Met Thr Asp Pro Phe Thr Glu Leu
                   565                 570                 575
   Ala Ile Thr Ile Cys Ile Ile Ile Asn Thr Val Phe Leu Ala Val Glu
               580                 585                 590
   His His Asn Met Asp Asp Asn Leu Lys Thr Ile Leu Lys Ile Gly Asn
           595                 600                 605
   Trp Val Phe Thr Gly Ile Phe Ile Ala Glu Met Cys Leu Lys Ile Ile
       610                 615                 620
   Ala Leu Asp Pro Tyr His Tyr Phe Arg His Gly Trp Asn Val Phe Asp
   625                 630                 635                 640
   Ser Ile Val Ala Leu Leu Ser Leu Ala Asp Val Leu Tyr Asn Thr Leu
                   645                 650                 655
   Ser Asp Asn Asn Arg Ser Phe Leu Ala Ser Leu Arg Val Leu Arg Val
               660                 665                 670
   Phe Lys Leu Ala Lys Ser Trp Pro Thr Leu Asn Thr Leu Ile Lys Ile
           675                 680                 685
   Ile Gly His Ser Val Gly Ala Leu Gly Asn Leu Thr Val Val Leu Thr
       690                 695                 700
   Ile Val Val Phe Ile Phe Ser Val Val Gly Met Arg Leu Phe Gly Thr
   705                 710                 715                 720
   Lys Phe Asn Lys Thr Ala Tyr Ala Thr Gln Glu Arg Pro Arg Arg Arg
                   725                 730                 735
   Trp His Met Asp Asn Phe Tyr His Ser Phe Leu Val Val Phe Arg Ile
               740                 745                 750
   Leu Cys Gly Glu Trp Ile Glu Asn Met Trp Gly Cys Met Gln Asp Met
           755                 760                 765
   Asp Gly Ser Pro Leu Cys Ile Ile Val Phe Val Leu Ile Met Val Ile
       770                 775                 780
   Gly Lys Leu Val Val Leu Asn Leu Phe Ile Ala Leu Leu Leu Asn Ser
   785                 790                 795                 800
   Phe Ser Asn Glu Glu Lys Asp Gly Ser Leu Glu Gly Glu Thr Arg Lys
                   805                 810                 815
   Thr Lys Val Gln Leu Ala Leu Asp Arg Phe Arg Arg Ala Phe Ser Phe
               820                 825                 830
   Met Leu His Ala Leu Gln Ser Phe Cys Cys Lys Lys Cys Arg Arg Lys
           835                 840                 845
   Asn Ser Pro Lys Pro Lys Glu Thr Thr Glu Ser Phe Ala Gly Glu Asn
       850                 855                 860
   Lys Asp Ser Ile Leu Pro Asp Ala Arg Pro Trp Lys Glu Tyr Asp Thr
   865                 870                 875                 880
   Asp Met Ala Leu Tyr Thr Gly Gln Ala Gly Ala Pro Leu Ala Pro Leu
                   885                 890                 895
   Ala Glu Val Glu Asp Asp Val Glu Tyr Cys Gly Glu Gly Gly Ala Leu
               900                 905                 910
   Pro Thr Ser Gln His Ser Ala Gly Val Gln Ala Gly Asp Leu Pro Pro
           915                 920                 925
   Glu Thr Lys Gln Leu Thr Ser Pro Asp Asp Gln Gly Val Glu Met Glu
       930                 935                 940
   Val Phe Ser Glu Glu Asp Leu His Leu Ser Ile Gln Ser Pro Arg Lys
   945                 950                 955                 960
   Lys Ser Asp Ala Val Ser Met Leu Ser Glu Cys Ser Thr Ile Asp Leu
                   965                 970                 975
   Asn Asp Ile Phe Arg Asn Leu Gln Lys Thr Val Ser Pro Lys Lys Gln
   980                 985                 990
   Pro Asp Arg Cys Phe Pro Lys Gly Leu Ser Cys His Phe Leu Cys His
           995                 1000                1005
   Lys Thr Asp Lys Arg Lys Ser Pro Trp Val Leu Trp Trp Asn Ile Arg
       1010                1015                1020
   Lys Thr Cys Tyr Gln Ile Val Lys His Ser Trp Phe Glu Ser Phe Ile
   1025                1030                1035                1040
   Ile Phe Val Ile Leu Leu Ser Ser Gly Ala Leu Ile Phe Glu Asp Val
                   1045                1050                1055
   Asn Leu Pro Ser Arg Pro Gln Val Glu Lys Leu Leu Arg Cys Thr Asp
               1060                1065                1070
   Asn Ile Phe Thr Phe Ile Phe Leu Leu Glu Met Ile Leu Lys Trp Val
           1075                1080                1085
   Ala Phe Gly Phe Arg Arg Tyr Phe Thr Ser Ala Trp Cys Trp Leu Asp
       1090                1095                1100
   Phe Leu Ile Val Val Val Ser Val Leu Ser Leu Met Asn Leu Pro Ser
   1105                1110                1115                1120
   Leu Lys Ser Phe Arg Thr Leu Arg Ala Leu Arg Pro Leu Arg Ala Leu
                   1125                1130                1135
   Ser Gln Phe Glu Gly Met Lys Val Val Val Tyr Ala Leu Ile Ser Ala
               1140                1145                1150
   Ile Pro Ala Ile Leu Asn Val Leu Leu Val Cys Leu Ile Phe Trp Leu
           1155                1160                1165
   Val Phe Cys Ile Leu Gly Val Asn Leu Phe Ser Gly Lys Phe Gly Arg
       1170                1175                1180
   Cys Ile Asn Gly Thr Asp Ile Asn Met Tyr Leu Asp Phe Thr Glu Val
   1185                1190                1195                1200
   Pro Asn Arg Ser Gln Cys Asn Ile Ser Asn Tyr Ser Trp Lys Val Pro
                   1205                1210                1215
   Gln Val Asn Phe Asp Asn Val Gly Asn Ala Tyr Leu Ala Leu Leu Gln
               1220                1225                1230
   Val Ala Thr Tyr Lys Gly Trp Leu Glu Ile Met Asn Ala Ala Val Asp
           1235                1240                1245
   Ser Arg Glu Lys Asp Glu Gln Pro Asp Phe Glu Ala Asn Leu Tyr Ala
       1250                1255                1260
   Tyr Leu Tyr Phe Val Val Phe Ile Ile Phe Gly Ser Phe Phe Thr Leu
   1265                1270                1275                1280
   Asn Leu Phe Ile Gly Val Ile Ile Asp Asn Phe Asn Gln Gln Gln Lys
                   1285                1290                1295
   Lys Leu Gly Gly Gln Asp Ile Phe Met Thr Glu Glu Gln Lys Lys Tyr
               1300                1305                1310
   Tyr Asn Ala Met Lys Lys Leu Gly Thr Lys Lys Pro Gln Lys Pro Ile
           1315                1320                1325
   Pro Arg Pro Leu Asn Arg Cys Gln Ala Phe Val Phe Asp Leu Val Thr
       1330                1335                1340
   Ser His Val Phe Asp Val Ile Ile Leu Gly Leu Ile Val Leu Asn Met
   1345                1350                1355                1360
   Ile Ile Met Met Ala Glu Ser Ala Asp Gln Pro Lys Asp Val Lys Lys
                   1365                1370                1375
   Thr Phe Asp Ile Leu Asn Ile Ala Phe Val Val Ile Phe Thr Ile Glu
               1380                1385                1390
   Cys Leu Ile Lys Val Phe Ala Leu Arg Gln His Tyr Phe Thr Asn Gly
           1395                1400                1405
   Trp Asn Leu Phe Asp Cys Val Val Val Val Leu Ser Ile Ile Ser Thr
       1410                1415                1420
   Leu Val Ser Arg Leu Glu Asp Ser Asp Ile Ser Phe Pro Pro Thr Leu
   1425                1430                1435                1440
   Phe Arg Val Val Arg Leu Ala Arg Ile Gly Arg Ile Leu Arg Leu Val
                   1445                1450                1455
   Arg Ala Ala Arg Gly Ile Arg Thr Leu Leu Phe Ala Leu Met Met Ser
               1460                1465                1470
   Leu Pro Ser Leu Phe Asn Ile Gly Leu Leu Leu Phe Leu Val Met Phe
           1475                1480                1485
   Ile Tyr Ala Ile Phe Gly Met Ser Trp Phe Ser Lys Val Lys Lys Gly
       1490                1495                1500
   Ser Gly Ile Asp Asp Ile Phe Asn Phe Glu Thr Phe Thr Gly Ser Met
   1505                1510                1515                1520
   Leu Cys Leu Phe Gln Ile Thr Thr Ser Ala Gly Trp Asp Thr Leu Leu
                   1525                1530                1535
   Asn Pro Met Leu Glu Ala Lys Glu His Cys Asn Ser Ser Ser Gln Asp
           1540                1545                1550
   Ser Cys Gln Gln Pro Gln Ile Ala Val Val Tyr Phe Val Ser Tyr Ile
       1555                1560                1565
   Ile Ile Ser Phe Leu Ile Val Val Asn Met Tyr Ile Ala Val Ile Leu
     1570                1575                1580
   Glu Asn Phe Asn Thr Ala Thr Glu Glu Ser Glu Asp Pro Leu Gly Glu
   1585                1590                1595                1600
   Asp Asp Phe Glu Ile Phe Tyr Glu Val Trp Glu Lys Phe Asp Pro Glu
                   1605                1610                1615
   Ala Ser Gln Phe Ile Gln Tyr Ser Ala Leu Ser Asp Phe Ala Asp Ala
               1620                1625                1630
   Leu Pro Glu Pro Leu Arg Val Ala Lys Pro Asn Lys Phe Gln Phe Leu
       1635                1640                1645
   Val Met Asp Leu Pro Met Val Met Gly Asp Arg Leu His Cys Met Asp
     1650                1655                1660
   Val Leu Phe Ala Phe Thr Thr Arg Val Leu Gly Asp Ser Ser Gly Leu
   1665                1670                1675                1680
   Asp Thr Met Lys Thr Met Met Glu Glu Lys Phe Met Glu Ala Asn Pro
                   1685                1690                1695
   Phe Lys Lys Leu Tyr Glu Pro Ile Val Thr Thr Thr Lys Arg Lys Glu
               1700                1705                1710
   Glu Glu Gln Gly Ala Ala Val Ile Gln Arg Ala Tyr Arg Lys His Met
       1715                1720                1725
   Glu Lys Met Val Lys Leu Arg Leu Lys Asp Arg Ser Ser Ser Ser His
     1730                1735                1740
   Gln Val Phe Cys Asn Gly Asp Leu Ser Ser Leu Asp Val Ala Lys Val
   1745                1750                1755                1760
   Lys Val His Asn Asp
                   1765

<210> 4
<211> 5822
<212> DNA
<213> Mus musculus

<220>

<221> CDS
<222> (19)..(5313)

<220>
<221> unsure
<222> (5804)
<223> cDNA sequence of mouse NaN, n = a or c or g or t

<400> 4
tctgagccaa gggtgaag atg gag gag agg tac tat cca gtg atc ttc cca 51
Met Glu Glu Arg Tyr Tyr Pro Val Ile Phe Pro
1               5                   10

gac gag agg aat ttc cgc ccc ttc act ttc gac tct ttg gct gca ata 99
Asp Glu Arg Asn Phe Arg Pro Phe Thr Phe Asp Ser Leu Ala Ala Ile
            15                  20                  25

gag aag cgg atc acc atc caa aag gag aag aag aaa tcc aaa gac aag 147
Glu Lys Arg Ile Thr Ile Gln Lys Glu Lys Lys Lys Ser Lys Asp Lys
        30                  35                  40

gca gca act gag ccc cag cct cgg cct cag ctc gac cta aag gcc tcc 195
Ala Ala Thr Glu Pro Gln Pro Arg Pro Gln Leu Asp Leu Lys Ala Ser
    45                  50                  55

agg aag tta cct aag ctc tat ggc gac gtt ccc cct gac ctt ata gcg 243
Arg Lys Leu Pro Lys Leu Tyr Gly Asp Val Pro Pro Asp Leu Ile Ala
60                  65                  70                  75

aag ccc ctg gaa gat ctg gac cca ttt tac aaa gac cat aag aca ttc 291
Lys Pro Leu Glu Asp Leu Asp Pro Phe Tyr Lys Asp His Lys Thr Phe
                80                  85                  90

atg gta ttg aac aag aag aga aca atc tat cgc ttc agc gcc aag agg 339
Met Val Leu Asn Lys Lys Arg Thr Ile Tyr Arg Phe Ser Ala Lys Arg
            95                  100                 105

gcc ttg ttc att ctg ggg cct ttt aat ccc atc aga agc ttc atg att 387
Ala Leu Phe Ile Leu Gly Pro Phe Asn Pro Ile Arg Ser Phe Met Ile
        110                 115                 120

cgc atc tct gtc cat tca gtc ttc agc atg ttc att atc tgc aca gtg 435
Arg Ile Ser Val His Ser Val Phe Ser Met Phe Ile Ile Cys Thr Val
    125                 130                 135

atc atc aac tgt atg ttc atg gct aat aat tct tct gtg gac agt cgt 483
Ile Ile Asn Cys Met Phe Met Ala Asn Asn Ser Ser Val Asp Ser Arg
140                 145                 150                 155

cct agc agt aac att ccc gaa tac gtc ttc att ggg att tat gtt tta 531
Pro Ser Ser Asn Ile Pro Glu Tyr Val Phe Ile Gly Ile Tyr Val Leu
                160                 165                 170

gaa gct gtg att aaa ata ttg gca aga ggc ttc att gtg gat gag ttt 579
Glu Ala Val Ile Lys Ile Leu Ala Arg Gly Phe Ile Val Asp Glu Phe
            175                 180                 185

tcc tac ctc cga gat cct tgg aac tgg ctg gac ttc att gtc atc gga 627
Ser Tyr Leu Arg Asp Pro Trp Asn Trp Leu Asp Phe Ile Val Ile Gly
        190                 195                 200

aca gcg ata gcg cct tgt ttt ctc ggt aac aaa gtc aat aat ctt tcc 675
Thr Ala Ile Ala Pro Cys Phe Leu Gly Asn Lys Val Asn Asn Leu Ser
    205                 210                 215

act cta cgt acc ttc cga gtg ttg aga gct ctg aaa gcc att tct gta 723
Thr Leu Arg Thr Phe Arg Val Leu Arg Ala Leu Lys Ala Ile Ser Val
220                 225                 230                 235

atc tca ggt ctg aag gtc atc gtg ggt gcc ctg ctg cgc tcc gtg aag 771
Ile Ser Gly Leu Lys Val Ile Val Gly Ala Leu Leu Arg Ser Val Lys
                240                 245                 250

aag cta gtg gac gtg atg gtc ctc act ctc ttt tgc ctc agc atc ttt 819
Lys Leu Val Asp Val Met Val Leu Thr Leu Phe Cys Leu Ser Ile Phe
            255                 260                 265

gcc ctg gtt ggt cag cag ctc ttc atg gga att ctg agc cag aaa tgt 867
Ala Leu Val Gly Gln Gln Leu Phe Met Gly Ile Leu Ser Gln Lys Cys
        270                 275                 280

att aag gac gac tgt ggc cct aac gct ttt tcc aac aag gat tgc ttt 915
Ile Lys Asp Asp Cys Gly Pro Asn Ala Phe Ser Asn Lys Asp Cys Phe
    285                 290                 295

gta aaa gaa aat gat agc gag gac ttc ata atg tgt ggc aac tgg ctc 963
Val Lys Glu Asn Asp Ser Glu Asp Phe Ile Met Cys Gly Asn Trp Leu
300                 305                 310                 315

ggc aga aga tcc tgc ccc gat ggt tcc acg tgc aat aaa acc aca ttt 1011
Gly Arg Arg Ser Cys Pro Asp Gly Ser Thr Cys Asn Lys Thr Thr Phe
                320                 325                 330

aac cca gat tat aat tat aca aac ttt gac agc ttt ggc tgg tct ttt 1059
Asn Pro Asp Tyr Asn Tyr Thr Asn Phe Asp Ser Phe Gly Trp Ser Phe
            335                 340                 345

ctc gcc atg ttc cgg gtt atg act caa gac tcc tgg gag aag ctt tat 1107
Leu Ala Met Phe Arg Val Met Thr Gln Asp Ser Trp Glu Lys Leu Tyr
        350                 355                 360

cga cag atc ctt cgc acc tcc ggg atc tac ttt gtc ttc ttc ttc gtg 1155
Arg Gln Ile Leu Arg Thr Ser Gly Ile Tyr Phe Val Phe Phe Phe Val
    365                 370                 375

gtc gtc atc ttc ctg ggc tct ttc tac ctg ctt aac tta acc ctg gct 1203
Val Val Ile Phe Leu Gly Ser Phe Tyr Leu Leu Asn Leu Thr Leu Ala
380                 385                 390                 395

gtc gtc acc atg gct tac gag gaa cag aac aga aat gtc gct gcc gag 1251
Val Val Thr Met Ala Tyr Glu Glu Gln Asn Arg Asn Val Ala Ala Glu
                400                 405                 410

aca gag gcc aag gag aag atg ttt cag gaa gcc cag cag ctg ttg agg 1299
Thr Glu Ala Lys Glu Lys Met Phe Gln Glu Ala Gln Gln Leu Leu Arg
            415                 420                 425

gag gaa aag gag gct ctg gtt gcc atg gga att gac aga act tcc ctt 1347
Glu Glu Lys Glu Ala Leu Val Ala Met Gly Ile Asp Arg Thr Ser Leu
        430                 435                 440

aat tcc ctc caa gct tcg tcc ttt tcc cca aag aag agg aag ttt ttt 1395
Asn Ser Leu Gln Ala Ser Ser Phe Ser Pro Lys Lys Arg Lys Phe Phe
    445                 450                 455

ggc agt aag aca aga aag tcc ttc ttt atg aga ggg tcc aag aca gcc 1443
Gly Ser Lys Thr Arg Lys Ser Phe Phe Met Arg Gly Ser Lys Thr Ala
460                 465                 470                 475

cga gcc tca gcg tcc gat tca gag gac gat gcc tct aaa aac cca caa 1491
Arg Ala Ser Ala Ser Asp Ser Glu Asp Asp Ala Ser Lys Asn Pro Gln
                480                 485                 490

ctc ctt gag caa aca aaa cga cta tcc cag aac ttg ccc gta gaa ctc 1539
Leu Leu Glu Gln Thr Lys Arg Leu Ser Gln Asn Leu Pro Val Glu Leu
            495                 500                 505

ttt gat gag cac gtg gac ccc ctc cat agg cag aga gcg ctg agt gcc 1587
Phe Asp Glu His Val Asp Pro Leu His Arg Gln Arg Ala Leu Ser Ala
        510                 515                 520

gtc agt atc tta acc atc acc atg cag gaa caa gaa aaa tcc cag gag 1635
Val Ser Ile Leu Thr Ile Thr Met Gln Glu Gln Glu Lys Ser Gln Glu
    525                 530                 535

cct tgt ttc ccg tgt ggg aaa aac ttg gca tcc aag tac ctg gtg tgg 1683
Pro Cys Phe Pro Cys Gly Lys Asn Leu Ala Ser Lys Tyr Leu Val Trp
540                 545                 550                 555

gaa tgt agc cct ccg tgg ctg tgc ata aag aag gtc ctg cag act atc 1731
Glu Cys Ser Pro Pro Trp Leu Cys Ile Lys Lys Val Leu Gln Thr Ile

                560                 565                 570

atg aca gac ccc ttc act gag ctg gcc atc acc atc tgc atc atc gtc 1779
Met Thr Asp Pro Phe Thr Glu Leu Ala Ile Thr Ile Cys Ile Ile Val
            575                 580                 585

aat act gtc ttc ttg gcc atg gaa cac cac aat atg gat aac tct tta 1827
Asn Thr Val Phe Leu Ala Met Glu His His Asn Met Asp Asn Ser Leu
        590                 595                 600

aaa gac ata ctg aaa ata gga aac tgg gtt ttc act gga att ttc ata 1875
Lys Asp Ile Leu Lys Ile Gly Asn Trp Val Phe Thr Gly Ile Phe Ile
    605                 610                 615

gcg gaa atg tgt ctc aag atc att gcg cta gac cct tac cac tac ttc 1923
Ala Glu Met Cys Leu Lys Ile Ile Ala Leu Asp Pro Tyr His Tyr Phe
620                 625                 630                 635

cgg cac ggc tgg aac atc ttt gac agc att gtg gcc ctt gtg agt ctc 1971
Arg His Gly Trp Asn Ile Phe Asp Ser Ile Val Ala Leu Val Ser Leu
                640                 645                 650

gct gac gtg ctc ttc cac aaa ctg tct aaa aac ctc tcc ttc ttg gct 2019
Ala Asp Val Leu Phe His Lys Leu Ser Lys Asn Leu Ser Phe Leu Ala
            655                 660                 665

tcc ctc aga gtg ctg agg gtc ttc aag tta gcc aaa tcc tgg ccc aca 2067
Ser Leu Arg Val Leu Arg Val Phe Lys Leu Ala Lys Ser Trp Pro Thr
        670                 675                 680

tta aac act ctc att aag atc atc ggc cac tcc gtg ggt gcg ctc gga 2115
Leu Asn Thr Leu Ile Lys Ile Ile Gly His Ser Val Gly Ala Leu Gly
    685                 690                 695

aac ctg act gtg gtc cta acg atc gtg gtc ttc atc ttt tcc gtg gtt 2163
Asn Leu Thr Val Val Leu Thr Ile Val Val Phe Ile Phe Ser Val Val
700                 705                 710                 715

ggc atg cgg ctc ttt ggt gcc aag ttt aac aag act tgc tcc acc tct 2211
Gly Met Arg Leu Phe Gly Ala Lys Phe Asn Lys Thr Cys Ser Thr Ser
                720                 725                 730

ccg gag tcc ctc cgg cgc tgg cac atg ggt gat ttc tac cat tcc ttc 2259
Pro Glu Ser Leu Arg Arg Trp His Met Gly Asp Phe Tyr His Ser Phe
            735                 740                 745

ctg gtg gtg ttc cgc atc ctc tgt ggg gag tgg atc gag aac atg tgg 2307
Leu Val Val Phe Arg Ile Leu Cys Gly Glu Trp Ile Glu Asn Met Trp
        750                 755                 760

gaa tgc atg cag gag atg gaa ggc tcc ccg ctg tgt gtc atc gtc ttt 2355
Glu Cys Met Gln Glu Met Glu Gly Ser Pro Leu Cys Val Ile Val Phe

765                 770                 775

gtg ctg atc atg gtg gtc ggg aag ctc gtg gtg ctt aac ctc ttc att     2403
Val Leu Ile Met Val Val Gly Lys Leu Val Val Leu Asn Leu Phe Ile
780                 785                 790                 795

gcc ttg ctg ctc aat tcc ttc agc aat gag gaa aag gat ggg aac cca     2451
Ala Leu Leu Leu Asn Ser Phe Ser Asn Glu Glu Lys Asp Gly Asn Pro
                800                 805                 810

gaa gga gag acc agg aaa acc aaa gtg cag cta gcc ctg gat cgg ttc     2499
Glu Gly Glu Thr Arg Lys Thr Lys Val Gln Leu Ala Leu Asp Arg Phe
            815                 820                 825

agc cga gcg ttc tac ttc atg gcg cgc gct ctt cag aat ttc tgt tgc     2547
Ser Arg Ala Phe Tyr Phe Met Ala Arg Ala Leu Gln Asn Phe Cys Cys
        830                 835                 840

aag aga tgc agg agg caa aac tcg cca aag cca aat gag gca aca gaa     2595
Lys Arg Cys Arg Arg Gln Asn Ser Pro Lys Pro Asn Glu Ala Thr Glu
    845                 850                 855

agc ttt gct ggt gag agt aga gac aca gcc acc ctg gat aca agg tcc     2643
Ser Phe Ala Gly Glu Ser Arg Asp Thr Ala Thr Leu Asp Thr Arg Ser
860                 865                 870                 875

tgg aag gag tat gat tca gaa atg act ctg tac act ggg cag gcc ggg     2691
Trp Lys Glu Tyr Asp Ser Glu Met Thr Leu Tyr Thr Gly Gln Ala Gly
                880                 885                 890

gct cca ctg gcc cca ctg gca aaa gaa gag gac gat atg gaa tgt tgt     2739
Ala Pro Leu Ala Pro Leu Ala Lys Glu Glu Asp Asp Met Glu Cys Cys
            895                 900                 905

ggt gaa tgt gat gcc tca cct acc tca cag cct agt gag gaa gct cag     2787
Gly Glu Cys Asp Ala Ser Pro Thr Ser Gln Pro Ser Glu Glu Ala Gln
        910                 915                 920

gcc tgt gac ctc cct ctg aag acc aag cgg ctc ccc agc cca gat gac     2835
Ala Cys Asp Leu Pro Leu Lys Thr Lys Arg Leu Pro Ser Pro Asp Asp
    925                 930                 935

cac ggg gtt gaa atg gaa gtg ttt tcc gaa gaa gat ccg aat tta acc     2883
His Gly Val Glu Met Glu Val Phe Ser Glu Glu Asp Pro Asn Leu Thr
940                 945                 950                 955

ata cag agt gct cga aag aag tct gat gcg gca agc atg ctc tca gaa     2931
Ile Gln Ser Ala Arg Lys Lys Ser Asp Ala Ala Ser Met Leu Ser Glu
                960                 965                 970

tgc agc aca ata gac ctg aat gat atc ttt aga aat tta cag aaa aca     2979
Cys Ser Thr Ile Asp Leu Asn Asp Ile Phe Arg Asn Leu Gln Lys Thr

            975                 980                 985

gtt tcc ccc caa aag caa cca gat cga tgc ttt ccc aag ggc ctc agt 3027
Val Ser Pro Gln Lys Gln Pro Asp Arg Cys Phe Pro Lys Gly Leu Ser
        990                 995                 1000

tgt atc ttt cta tgt tgc aaa aca atc aaa aaa aag tcc ccc tgg gtc 3075
Cys Ile Phe Leu Cys Cys Lys Thr Ile Lys Lys Lys Ser Pro Trp Val
    1005                1010                1015

ctg tgg tgg aat ctt cgg aaa acc tgc tac caa atc gtg aag cat agc 3123
Leu Trp Trp Asn Leu Arg Lys Thr Cys Tyr Gln Ile Val Lys His Ser
1020                1025                1030                1035

tgg ttt gag agc ttc ata att ttt gtc atc ctg ctg agc agc gga gca 3171
Trp Phe Glu Ser Phe Ile Ile Phe Val Ile Leu Leu Ser Ser Gly Ala
                1040                1045                1050

ctg ata ttc gaa gat gtc aat ctt ccc agc cgg ccc caa gtt gaa aaa 3219
Leu Ile Phe Glu Asp Val Asn Leu Pro Ser Arg Pro Gln Val Glu Lys
            1055                1060                1065

tta ctg aag tgt acc gat aat att ttc aca ttt att ttt ctc ctg gaa 3267
Leu Leu Lys Cys Thr Asp Asn Ile Phe Thr Phe Ile Phe Leu Leu Glu
        1070                1075                1080

atg att ttg aag tgg gtg gcc ttt gga ttc cgg aag tat ttc acc agt 3315
Met Ile Leu Lys Trp Val Ala Phe Gly Phe Arg Lys Tyr Phe Thr Ser
    1085                1090                1095

gcc tgg tgc tgg ctc gat ttc ctc att gtg gtg gtg tct gtg ctc agc 3363
Ala Trp Cys Trp Leu Asp Phe Leu Ile Val Val Val Ser Val Leu Ser
1100                1105                1110                1115

ctc acg aac tta cca aac ttg aag tcc ttc cgg aat ctg cga gcg ctg 3411
Leu Thr Asn Leu Pro Asn Leu Lys Ser Phe Arg Asn Leu Arg Ala Leu
                1120                1125                1130

aga cct ctg cgg gca ctg tct cag ttt gaa gga atg aag gtt gtt gtc 3459
Arg Pro Leu Arg Ala Leu Ser Gln Phe Glu Gly Met Lys Val Val Val
            1135                1140                1145

aat gcc ctc atg agt gcc ata cct gcc atc ctc aat gtc ttg ctg gtc 3507
Asn Ala Leu Met Ser Ala Ile Pro Ala Ile Leu Asn Val Leu Leu Val
        1150                1155                1160

tgc ctc att ttc tgg ctc ata ttt tgt atc ctg gga gta aat ttt ttt 3555
Cys Leu Ile Phe Trp Leu Ile Phe Cys Ile Leu Gly Val Asn Phe Phe
    1165                1170                1175

tct ggg aag ttt gga aga tgc att aat gga aca gac ata aat aaa tat 3603
Ser Gly Lys Phe Gly Arg Cys Ile Asn Gly Thr Asp Ile Asn Lys Tyr

1180                1185                1190                1195

ttc aac gct tcc aat gtt cca aac caa agc caa tgt tta gtt agt aat    3651
Phe Asn Ala Ser Asn Val Pro Asn Gln Ser Gln Cys Leu Val Ser Asn
            1200                1205                1210

tac acg tgg aaa gtc ccg aat gtc aac ttt gac aac gtg ggg aat gcc    3699
Tyr Thr Trp Lys Val Pro Asn Val Asn Phe Asp Asn Val Gly Asn Ala
        1215                1220                1225

tac ctt gcc ctg ctg caa gtg gcg acc tat aag ggc tgg ctg gac att    3747
Tyr Leu Ala Leu Leu Gln Val Ala Thr Tyr Lys Gly Trp Leu Asp Ile
    1230                1235                1240

atg aat gca gct gtt gat tcc aga ggg aaa gat gag cag ccg gcc ttt    3795
Met Asn Ala Ala Val Asp Ser Arg Gly Lys Asp Glu Gln Pro Ala Phe
1245                1250                1255

gag gcg aat cta tac gca tac ctt tac ttc gtg gtt ttt atc atc ttc    3843
Glu Ala Asn Leu Tyr Ala Tyr Leu Tyr Phe Val Val Phe Ile Ile Phe
1260                1265                1270                1275

ggc tca ttc ttt acc ctg aac ctc ttt atc ggt gtt att att gac aac    3891
Gly Ser Phe Phe Thr Leu Asn Leu Phe Ile Gly Val Ile Ile Asp Asn
            1280                1285                1290

ttc aat cag cag cag aaa aag tta ggt ggc caa gac att ttt atg aca    3939
Phe Asn Gln Gln Gln Lys Lys Leu Gly Gly Gln Asp Ile Phe Met Thr
        1295                1300                1305

gaa gaa cag aag aaa tat tac aat gca atg aaa aag tta gga acc aag    3987
Glu Glu Gln Lys Lys Tyr Tyr Asn Ala Met Lys Lys Leu Gly Thr Lys
    1310                1315                1320

aag cct caa aag ccc atc cca agg ccc ctg aac aaa tgt caa gcc ttc    4035
Lys Pro Gln Lys Pro Ile Pro Arg Pro Leu Asn Lys Cys Gln Ala Phe
1325                1330                1335

gtg ttc gat ttg gtc aca agc cag gtc ttt gac gtc atc att ctg ggt    4083
Val Phe Asp Leu Val Thr Ser Gln Val Phe Asp Val Ile Ile Leu Gly
1340                1345                1350                1355

ctt att gtc aca aac atg att atc atg atg gct gaa tct gaa ggc cag    4131
Leu Ile Val Thr Asn Met Ile Ile Met Met Ala Glu Ser Glu Gly Gln
            1360                1365                1370

ccc aac gaa gtg aag aaa atc ttt gat att ctc aac ata gtc ttc gtg    4179
Pro Asn Glu Val Lys Lys Ile Phe Asp Ile Leu Asn Ile Val Phe Val
        1375                1380                1385

gtc atc ttt acc gta gag tgt ctc atc aaa gtc ttt gct ttg agg caa    4227
Val Ile Phe Thr Val Glu Cys Leu Ile Lys Val Phe Ala Leu Arg Gln

1390                1395                1400

cac tac ttc acc aat ggc tgg aac tta ttt gat tgt gtg gtc gtg gtt    4275
His Tyr Phe Thr Asn Gly Trp Asn Leu Phe Asp Cys Val Val Val Val
    1405                1410                1415

ctt tcc atc att agt acc ttg gtt tct ggc ttg gag aac agc aac gtc    4323
Leu Ser Ile Ile Ser Thr Leu Val Ser Gly Leu Glu Asn Ser Asn Val
1420                1425                1430                1435

ttc ccg ccc aca ctc ttc agg att gtc cgc ttg gct cgg atc ggt cga    4371
Phe Pro Pro Thr Leu Phe Arg Ile Val Arg Leu Ala Arg Ile Gly Arg
                1440                1445                1450

atc ctc aga ctg gtc cgg gcg gct cga gga atc agg aca ctc ctt ttc    4419
Ile Leu Arg Leu Val Arg Ala Ala Arg Gly Ile Arg Thr Leu Leu Phe
            1455                1460                1465

gcg ttg atg atg tct ctc ccc tct ctc ttc aac att ggt ctg ctt ctc    4467
Ala Leu Met Met Ser Leu Pro Ser Leu Phe Asn Ile Gly Leu Leu Leu
        1470                1475                1480

ttt ctg gtg atg ttc att tat gcc atc ttt ggg atg aac tgg ttt tcc    4515
Phe Leu Val Met Phe Ile Tyr Ala Ile Phe Gly Met Asn Trp Phe Ser
    1485                1490                1495

aaa gtg aag aga ggc tct ggg att gat gac atc ttc aac ttt gac act    4563
Lys Val Lys Arg Gly Ser Gly Ile Asp Asp Ile Phe Asn Phe Asp Thr
1500                1505                1510                1515

ttc tcg ggc agc atg ctc tgc ctc ttc cag ata acc act tca gcc ggc    4611
Phe Ser Gly Ser Met Leu Cys Leu Phe Gln Ile Thr Thr Ser Ala Gly
                1520                1525                1530

tgg gat gct ctc ctc aac ccc atg ctg gaa tca aaa gcc tct tgc aat    4659
Trp Asp Ala Leu Leu Asn Pro Met Leu Glu Ser Lys Ala Ser Cys Asn
            1535                1540                1545

tcc tcc tcc caa gag agc tgt cag cag ccg cag ata gcc ata gtc tac    4707
Ser Ser Ser Gln Glu Ser Cys Gln Gln Pro Gln Ile Ala Ile Val Tyr
        1550                1555                1560

ttc gtc agc tac atc atc atc tcc ttt ctc att gtg gtt aac atg tac    4755
Phe Val Ser Tyr Ile Ile Ile Ser Phe Leu Ile Val Val Asn Met Tyr
    1565                1570                1575

ata gct gtg att cta gag aac ttc aac aca gcc aca gag gag agc gag    4803
Ile Ala Val Ile Leu Glu Asn Phe Asn Thr Ala Thr Glu Glu Ser Glu
1580                1585                1590                1595

gac ccc ctg ggc gaa gac gac ttt gag atc ttc tat gag atc tgg gag    4851
Asp Pro Leu Gly Glu Asp Asp Phe Glu Ile Phe Tyr Glu Ile Trp Glu

1600                1605                1610

aag ttt gac ccc gaa gca aca cag ttc atc cag tac tca tcc ctc tct    4899
Lys Phe Asp Pro Glu Ala Thr Gln Phe Ile Gln Tyr Ser Ser Leu Ser
        1615                1620                1625

gac ttc gcc gac gcc ctg ccc gag ccg ttg cgt gtg gcc aag ccc aac    4947
Asp Phe Ala Asp Ala Leu Pro Glu Pro Leu Arg Val Ala Lys Pro Asn
    1630                1635                1640

agg ttt cag ttt ctc atg atg gac ttg ccc atg gtg atg ggt gat cgc    4995
Arg Phe Gln Phe Leu Met Met Asp Leu Pro Met Val Met Gly Asp Arg
  1645                1650                1655

ctc cat tgc atg gat gtt ctc ttt gct ttc acc acc agg gtc ctc ggg    5043
Leu His Cys Met Asp Val Leu Phe Ala Phe Thr Thr Arg Val Leu Gly
1660                1665                1670                1675

aac tcc agc ggc ttg gat acc atg aaa gcc atg atg gag gag aag ttc    5091
Asn Ser Ser Gly Leu Asp Thr Met Lys Ala Met Met Glu Glu Lys Phe
                1680                1685                1690

atg gag gcc aat cct ttc aag aag ttg tac gag ccc att gtc acc acc    5139
Met Glu Ala Asn Pro Phe Lys Lys Leu Tyr Glu Pro Ile Val Thr Thr
            1695                1700                1705

aca aag agg aag gag gag gag gaa tgt gcc gct gtc atc cag agg gcc    5187
Thr Lys Arg Lys Glu Glu Glu Glu Cys Ala Ala Val Ile Gln Arg Ala
        1710                1715                1720

tac cgg aga cac atg gag aag atg atc aag ctg aag ctg aaa ggc agg    5235
Tyr Arg Arg His Met Glu Lys Met Ile Lys Leu Lys Leu Lys Gly Arg
    1725                1730                1735

tca agt tca tcg ctc cag gtg ttt tgc aat gga gac ttg tct agc ttg    5283
Ser Ser Ser Ser Leu Gln Val Phe Cys Asn Gly Asp Leu Ser Ser Leu
1740                1745                1750                1755

gat gtg ccc aag atc aag gtt cat tgt gac tgaaaccccc acctgcacgc    5333
Asp Val Pro Lys Ile Lys Val His Cys Asp
                1760                1765

ctacctcaca gcctcacagc tcagccccca gcctctggcg aacaagcggc ggactcaccg 5393

aacaggccgt tcaacttgtt tttttgggtg aaagaggtga taggttggtg tccattttta 5453

aatgattctt ggaaagattg aacgtcggaa catgttagaa aggactgcca aggacatcca 5513

cagtaacgga aggcctgaag gacagttcaa attatgtaaa gaaacgagaa ggaaaggtca 5573

catgtctgtt cagttttaag tatgtgacct gccacatgta gctcctttgc atgttaagtg 5633

agaagtcaaa accctgccat aagtaaatag ctttgttgca ggtgtttcta ccagtgctgc 5693

ggatttgggt gtatggctca aacctgaaag catgactctg acttgtcagc accccaactt 5753

tcagaagctc tgatctctgt cctaggtgtt tgacaaataa atacataaaa naaaaaaaaa 5813

aaaaaaaaa 5822

<210> 5
<211> 1765
<212> PRT
<213> Mus musculus

<220>
<223> putative protein seq. of mouse NaN

<400> 5
Met Glu Glu Arg Tyr Tyr Pro Val Ile Phe Pro Asp Glu Arg Asn Phe
1               5                   10                  15

Arg Pro Phe Thr Phe Asp Ser Leu Ala Ala Ile Glu Lys Arg Ile Thr
            20                  25                  30

Ile Gln Lys Glu Lys Lys Lys Ser Lys Asp Lys Ala Ala Thr Glu Pro
        35                  40                  45

Gln Pro Arg Pro Gln Leu Asp Leu Lys Ala Ser Arg Lys Leu Pro Lys
    50                  55                  60

Leu Tyr Gly Asp Val Pro Pro Asp Leu Ile Ala Lys Pro Leu Glu Asp
65                  70                  75                  80

Leu Asp Pro Phe Tyr Lys Asp His Lys Thr Phe Met Val Leu Asn Lys
                85                  90                  95

Lys Arg Thr Ile Tyr Arg Phe Ser Ala Lys Arg Ala Leu Phe Ile Leu
            100                 105                 110

Gly Pro Phe Asn Pro Ile Arg Ser Phe Met Ile Arg Ile Ser Val His
        115                 120                 125

Ser Val Phe Ser Met Phe Ile Ile Cys Thr Val Ile Ile Asn Cys Met
    130                 135                 140

Phe Met Ala Asn Asn Ser Ser Val Asp Ser Arg Pro Ser Ser Asn Ile
145                 150                 155                 160

Pro Glu Tyr Val Phe Ile Gly Ile Tyr Val Leu Glu Ala Val Ile Lys
                165                 170                 175

Ile Leu Ala Arg Gly Phe Ile Val Asp Glu Phe Ser Tyr Leu Arg Asp
            180                 185                 190

Pro Trp Asn Trp Leu Asp Phe Ile Val Ile Gly Thr Ala Ile Ala Pro
        195                 200                 205

Cys Phe Leu Gly Asn Lys Val Asn Asn Leu Ser Thr Leu Arg Thr Phe
    210                 215                 220

Arg Val Leu Arg Ala Leu Lys Ala Ile Ser Val Ile Ser Gly Leu Lys
225                 230                 235                 240

Val Ile Val Gly Ala Leu Leu Arg Ser Val Lys Lys Leu Val Asp Val
                245                 250                 255

Met Val Leu Thr Leu Phe Cys Leu Ser Ile Phe Ala Leu Val Gly Gln
            260                 265                 270

Gln Leu Phe Met Gly Ile Leu Ser Gln Lys Cys Ile Lys Asp Asp Cys
        275                 280                 285

Gly Pro Asn Ala Phe Ser Asn Lys Asp Cys Phe Val Lys Glu Asn Asp
    290                 295                 300

Ser Glu Asp Phe Ile Met Cys Gly Asn Trp Leu Gly Arg Arg Ser Cys
305                 310                 315                 320

Pro Asp Gly Ser Thr Cys Asn Lys Thr Thr Phe Asn Pro Asp Tyr Asn
                325                 330                 335

Tyr Thr Asn Phe Asp Ser Phe Gly Trp Ser Phe Leu Ala Met Phe Arg
            340                 345                 350

Val Met Thr Gln Asp Ser Trp Glu Lys Leu Tyr Arg Gln Ile Leu Arg
        355                 360                 365

Thr Ser Gly Ile Tyr Phe Val Phe Phe Phe Val Val Val Ile Phe Leu
    370                 375                 380

Gly Ser Phe Tyr Leu Leu Asn Leu Thr Leu Ala Val Val Thr Met Ala
385                 390                 395                 400

Tyr Glu Glu Gln Asn Arg Asn Val Ala Ala Glu Thr Glu Ala Lys Glu
                405                 410                 415

Lys Met Phe Gln Glu Ala Gln Gln Leu Leu Arg Glu Glu Lys Glu Ala
            420                 425                 430

Leu Val Ala Met Gly Ile Asp Arg Thr Ser Leu Asn Ser Leu Gln Ala
        435                 440                 445

Ser Ser Phe Ser Pro Lys Lys Arg Lys Phe Phe Gly Ser Lys Thr Arg
    450                 455                 460

Lys Ser Phe Phe Met Arg Gly Ser Lys Thr Ala Arg Ala Ser Ala Ser
465                 470                 475                 480

Asp Ser Glu Asp Asp Ala Ser Lys Asn Pro Gln Leu Leu Glu Gln Thr
                485                 490                 495

Lys Arg Leu Ser Gln Asn Leu Pro Val Glu Leu Phe Asp Glu His Val
            500                 505                 510

Asp Pro Leu His Arg Gln Arg Ala Leu Ser Ala Val Ser Ile Leu Thr
        515                 520                 525

Ile Thr Met Gln Glu Gln Glu Lys Ser Gln Glu Pro Cys Phe Pro Cys
    530                 535                 540

Gly Lys Asn Leu Ala Ser Lys Tyr Leu Val Trp Glu Cys Ser Pro Pro
545                 550                 555                 560

Trp Leu Cys Ile Lys Lys Val Leu Gln Thr Ile Met Thr Asp Pro Phe
                565                 570                 575

Thr Glu Leu Ala Ile Thr Ile Cys Ile Ile Val Asn Thr Val Phe Leu
            580                 585                 590

Ala Met Glu His His Asn Met Asp Asn Ser Leu Lys Asp Ile Leu Lys
        595                 600                 605

Ile Gly Asn Trp Val Phe Thr Gly Ile Phe Ile Ala Glu Met Cys Leu
    610                 615                 620

Lys Ile Ile Ala Leu Asp Pro Tyr His Tyr Phe Arg His Gly Trp Asn
625                 630                 635                 640

Ile Phe Asp Ser Ile Val Ala Leu Val Ser Leu Ala Asp Val Leu Phe
                645                 650                 655

His Lys Leu Ser Lys Asn Leu Ser Phe Leu Ala Ser Leu Arg Val Leu
            660                 665                 670

Arg Val Phe Lys Leu Ala Lys Ser Trp Pro Thr Leu Asn Thr Leu Ile
        675                 680                 685

Lys Ile Ile Gly His Ser Val Gly Ala Leu Gly Asn Leu Thr Val Val
    690                 695                 700

Leu Thr Ile Val Val Phe Ile Phe Ser Val Val Gly Met Arg Leu Phe
705                 710                 715                 720

Gly Ala Lys Phe Asn Lys Thr Cys Ser Thr Ser Pro Glu Ser Leu Arg

                725                 730                 735

Arg Trp His Met Gly Asp Phe Tyr His Ser Phe Leu Val Val Phe Arg
            740                 745                 750

Ile Leu Cys Gly Glu Trp Ile Glu Asn Met Trp Glu Cys Met Gln Glu
        755                 760                 765

Met Glu Gly Ser Pro Leu Cys Val Ile Val Phe Val Leu Ile Met Val
    770                 775                 780

Val Gly Lys Leu Val Val Leu Asn Leu Phe Ile Ala Leu Leu Leu Asn
785                 790                 795                 800

Ser Phe Ser Asn Glu Glu Lys Asp Gly Asn Pro Glu Gly Glu Thr Arg
                805                 810                 815

Lys Thr Lys Val Gln Leu Ala Leu Asp Arg Phe Ser Arg Ala Phe Tyr
            820                 825                 830

Phe Met Ala Arg Ala Leu Gln Asn Phe Cys Cys Lys Arg Cys Arg Arg
        835                 840                 845

Gln Asn Ser Pro Lys Pro Asn Glu Ala Thr Glu Ser Phe Ala Gly Glu
    850                 855                 860

Ser Arg Asp Thr Ala Thr Leu Asp Thr Arg Ser Trp Lys Glu Tyr Asp
865                 870                 875                 880

Ser Glu Met Thr Leu Tyr Thr Gly Gln Ala Gly Ala Pro Leu Ala Pro
                885                 890                 895

Leu Ala Lys Glu Glu Asp Asp Met Glu Cys Cys Gly Glu Cys Asp Ala
            900                 905                 910

Ser Pro Thr Ser Gln Pro Ser Glu Glu Ala Gln Ala Cys Asp Leu Pro
        915                 920                 925

Leu Lys Thr Lys Arg Leu Pro Ser Pro Asp Asp His Gly Val Glu Met
    930                 935                 940

Glu Val Phe Ser Glu Glu Asp Pro Asn Leu Thr Ile Gln Ser Ala Arg
945                 950                 955                 960

Lys Lys Ser Asp Ala Ala Ser Met Leu Ser Glu Cys Ser Thr Ile Asp
                965                 970                 975

Leu Asn Asp Ile Phe Arg Asn Leu Gln Lys Thr Val Ser Pro Gln Lys
            980                 985                 990

Gln Pro Asp Arg Cys Phe Pro Lys Gly Leu Ser Cys Ile Phe Leu Cys
        995                 1000                1005

Cys Lys Thr Ile Lys Lys Lys Ser Pro Trp Val Leu Trp Trp Asn Leu
    1010                1015                1020

Arg Lys Thr Cys Tyr Gln Ile Val Lys His Ser Trp Phe Glu Ser Phe
1025                1030                1035                1040

Ile Ile Phe Val Ile Leu Leu Ser Ser Gly Ala Leu Ile Phe Glu Asp
                1045                1050                1055

Val Asn Leu Pro Ser Arg Pro Gln Val Glu Lys Leu Leu Lys Cys Thr
            1060                1065                1070

Asp Asn Ile Phe Thr Phe Ile Phe Leu Leu Glu Met Ile Leu Lys Trp
        1075                1080                1085

Val Ala Phe Gly Phe Arg Lys Tyr Phe Thr Ser Ala Trp Cys Trp Leu
    1090                1095                1100

Asp Phe Leu Ile Val Val Val Ser Val Leu Ser Leu Thr Asn Leu Pro
1105                1110                1115                1120

Asn Leu Lys Ser Phe Arg Asn Leu Arg Ala Leu Arg Pro Leu Arg Ala
                1125                1130                1135

Leu Ser Gln Phe Glu Gly Met Lys Val Val Val Asn Ala Leu Met Ser
            1140                1145                1150

Ala Ile Pro Ala Ile Leu Asn Val Leu Leu Val Cys Leu Ile Phe Trp
        1155                1160                1165

Leu Ile Phe Cys Ile Leu Gly Val Asn Phe Phe Ser Gly Lys Phe Gly
    1170                1175                1180

Arg Cys Ile Asn Gly Thr Asp Ile Asn Lys Tyr Phe Asn Ala Ser Asn
1185                1190                1195                1200

Val Pro Asn Gln Ser Gln Cys Leu Val Ser Asn Tyr Thr Trp Lys Val
                1205                1210                1215

Pro Asn Val Asn Phe Asp Asn Val Gly Asn Ala Tyr Leu Ala Leu Leu
            1220                1225                1230

Gln Val Ala Thr Tyr Lys Gly Trp Leu Asp Ile Met Asn Ala Ala Val
        1235                1240                1245

Asp Ser Arg Gly Lys Asp Glu Gln Pro Ala Phe Glu Ala Asn Leu Tyr
    1250                1255                1260

Ala Tyr Leu Tyr Phe Val Val Phe Ile Ile Phe Gly Ser Phe Phe Thr
1265                1270                1275                1280

Leu Asn Leu Phe Ile Gly Val Ile Ile Asp Asn Phe Asn Gln Gln Gln
                1285                1290                1295

Lys Lys Leu Gly Gly Gln Asp Ile Phe Met Thr Glu Glu Gln Lys Lys
            1300                1305                1310

Tyr Tyr Asn Ala Met Lys Lys Leu Gly Thr Lys Lys Pro Gln Lys Pro
        1315                1320                1325

Ile Pro Arg Pro Leu Asn Lys Cys Gln Ala Phe Val Phe Asp Leu Val
    1330                1335                1340

Thr Ser Gln Val Phe Asp Val Ile Ile Leu Gly Leu Ile Val Thr Asn
1345                1350                1355                1360

Met Ile Ile Met Met Ala Glu Ser Glu Gly Gln Pro Asn Glu Val Lys
                1365                1370                1375

Lys Ile Phe Asp Ile Leu Asn Ile Val Phe Val Val Ile Phe Thr Val
            1380                1385                1390

Glu Cys Leu Ile Lys Val Phe Ala Leu Arg Gln His Tyr Phe Thr Asn
        1395                1400                1405

Gly Trp Asn Leu Phe Asp Cys Val Val Val Val Leu Ser Ile Ile Ser
    1410                1415                1420

Thr Leu Val Ser Gly Leu Glu Asn Ser Asn Val Phe Pro Pro Thr Leu
1425                1430                1435                1440

Phe Arg Ile Val Arg Leu Ala Arg Ile Gly Arg Ile Leu Arg Leu Val
                1445                1450                1455

Arg Ala Ala Arg Gly Ile Arg Thr Leu Leu Phe Ala Leu Met Met Ser
            1460                1465                1470

Leu Pro Ser Leu Phe Asn Ile Gly Leu Leu Leu Phe Leu Val Met Phe
        1475                1480                1485

Ile Tyr Ala Ile Phe Gly Met Asn Trp Phe Ser Lys Val Lys Arg Gly
    1490                1495                1500

Ser Gly Ile Asp Asp Ile Phe Asn Phe Asp Thr Phe Ser Gly Ser Met
1505                1510                1515                1520

Leu Cys Leu Phe Gln Ile Thr Thr Ser Ala Gly Trp Asp Ala Leu Leu
                1525                1530                1535

Asn Pro Met Leu Glu Ser Lys Ala Ser Cys Asn Ser Ser Ser Gln Glu
            1540                1545                1550

Ser Cys Gln Gln Pro Gln Ile Ala Ile Val Tyr Phe Val Ser Tyr Ile

1555                1560                1565

Ile Ile Ser Phe Leu Ile Val Val Asn Met Tyr Ile Ala Val Ile Leu
    1570                1575                1580

Glu Asn Phe Asn Thr Ala Thr Glu Glu Ser Glu Asp Pro Leu Gly Glu
1585                1590                1595                1600

Asp Asp Phe Glu Ile Phe Tyr Glu Ile Trp Glu Lys Phe Asp Pro Glu
                1605                1610                1615

Ala Thr Gln Phe Ile Gln Tyr Ser Ser Leu Ser Asp Phe Ala Asp Ala
            1620                1625                1630

Leu Pro Glu Pro Leu Arg Val Ala Lys Pro Asn Arg Phe Gln Phe Leu
        1635                1640                1645

Met Met Asp Leu Pro Met Val Met Gly Asp Arg Leu His Cys Met Asp
    1650                1655                1660

Val Leu Phe Ala Phe Thr Thr Arg Val Leu Gly Asn Ser Ser Gly Leu
1665                1670                1675                1680

Asp Thr Met Lys Ala Met Met Glu Glu Lys Phe Met Glu Ala Asn Pro
                1685                1690                1695

Phe Lys Lys Leu Tyr Glu Pro Ile Val Thr Thr Thr Lys Arg Lys Glu
            1700                1705                1710

Glu Glu Glu Cys Ala Ala Val Ile Gln Arg Ala Tyr Arg Arg His Met
        1715                1720                1725

Glu Lys Met Ile Lys Leu Lys Leu Lys Gly Arg Ser Ser Ser Ser Leu
    1730                1735                1740

Gln Val Phe Cys Asn Gly Asp Leu Ser Ser Leu Asp Val Pro Lys Ile
1745                1750                1755                1760

Lys Val His Cys Asp
                1765

<210> 6
<211> 3701
<212> DNA
<213> Homo sapiens

<220>
<221> CDS
<222> (1)..(3699)
<223> partial human NaN cDNA sequence

<220>
<221> unsure
<222> (922)
<223> y = c or t

<400> 6
tcc att gtc att gga ata gcg att gtg tca tat att cca gga atc acc      48
Ser Ile Val Ile Gly Ile Ala Ile Val Ser Tyr Ile Pro Gly Ile Thr
1               5                   10                  15

atc aaa cta ttg ccc ctg cgt acc ttc cgt gtg ttc aga gct ttg aaa      96
Ile Lys Leu Leu Pro Leu Arg Thr Phe Arg Val Phe Arg Ala Leu Lys
            20                  25                  30

gca att tca gta gtt tca cgt ctg aag gtc atc gtg ggg gcc ttg cta      144
Ala Ile Ser Val Val Ser Arg Leu Lys Val Ile Val Gly Ala Leu Leu
        35                  40                  45

cgc tct gtg aag aag ctg gtc aac gtg att atc ctc acc ttc ttt tgc      192
Arg Ser Val Lys Lys Leu Val Asn Val Ile Ile Leu Thr Phe Phe Cys
    50                  55                  60

ctc agc atc ttt gcc ctg gta ggt cag cag ctc ttc atg gga agt ctg      240
Leu Ser Ile Phe Ala Leu Val Gly Gln Gln Leu Phe Met Gly Ser Leu
65                  70                  75                  80

aac ctg aaa tgc atc tcg agg gac tgt aaa aat atc agt aac ccg gaa      288
Asn Leu Lys Cys Ile Ser Arg Asp Cys Lys Asn Ile Ser Asn Pro Glu
                85                  90                  95

gct tat gac cat tgc ttt gaa aag aaa gaa aat tca cct gaa ttc aaa      336
Ala Tyr Asp His Cys Phe Glu Lys Lys Glu Asn Ser Pro Glu Phe Lys
            100                 105                 110

atg tgt ggc atc tgg atg ggt aac agt gcc tgt tcc ata caa tat gaa      384
Met Cys Gly Ile Trp Met Gly Asn Ser Ala Cys Ser Ile Gln Tyr Glu
        115                 120                 125

tgt aag cac acc aaa att aat cct gac tat aat tat acg aat ttt gac      432
Cys Lys His Thr Lys Ile Asn Pro Asp Tyr Asn Tyr Thr Asn Phe Asp
    130                 135                 140

aac ttt ggc tgg tct ttt ctt gcc atg ttc cgg ctg atg acc caa gat      480
Asn Phe Gly Trp Ser Phe Leu Ala Met Phe Arg Leu Met Thr Gln Asp
145                 150                 155                 160

tcc tgg gag aag ctt tat caa cag acc ctg cgt act act ggg ctc tac      528
Ser Trp Glu Lys Leu Tyr Gln Gln Thr Leu Arg Thr Thr Gly Leu Tyr
                165                 170                 175

tca gtc ttc ttc ttc att gtg gtc att ttc ctg ggc tcc ttc tac ctg      576
Ser Val Phe Phe Phe Ile Val Val Ile Phe Leu Gly Ser Phe Tyr Leu

180                 185                 190

att aac tta acc ctg gct gtt gtt acc atg gca tat gag gag cag aac     624
Ile Asn Leu Thr Leu Ala Val Val Thr Met Ala Tyr Glu Glu Gln Asn
        195                 200                 205

aag aat gta gct gca gag ata gag gcc aag gaa aag atg ttt cag gaa     672
Lys Asn Val Ala Ala Glu Ile Glu Ala Lys Glu Lys Met Phe Gln Glu
    210                 215                 220

gcc cag cag ctg tta aag gag gaa aag gag gct ctg gtt gcc atg gga     720
Ala Gln Gln Leu Leu Lys Glu Glu Lys Glu Ala Leu Val Ala Met Gly
225                 230                 235                 240

att gac aga agt tca ctt act tcc ctt gaa aca tca tat ttt acc cca     768
Ile Asp Arg Ser Ser Leu Thr Ser Leu Glu Thr Ser Tyr Phe Thr Pro
                245                 250                 255

aaa aag aga aag ctc ttt ggt aat aag aaa agg aag tcc ttc ttt ttg     816
Lys Lys Arg Lys Leu Phe Gly Asn Lys Lys Arg Lys Ser Phe Phe Leu
            260                 265                 270

aga gag tct ggg aaa gac cag cct cct ggg tca gat tct gat gaa gat     864
Arg Glu Ser Gly Lys Asp Gln Pro Pro Gly Ser Asp Ser Asp Glu Asp
        275                 280                 285

tgc caa aaa aag cca cag ctc cta gag caa acc aaa cga ctg tcc cag     912
Cys Gln Lys Lys Pro Gln Leu Leu Glu Gln Thr Lys Arg Leu Ser Gln
    290                 295                 300

aat cta tca ytg gac cac ttt gat gag cat gga gat cct ctc caa agg     960
Asn Leu Ser Leu Asp His Phe Asp Glu His Gly Asp Pro Leu Gln Arg
305                 310                 315                 320

cag aga gca ctg agt gct gtc agc atc ctc acc atc acc atg aag gaa     1008
Gln Arg Ala Leu Ser Ala Val Ser Ile Leu Thr Ile Thr Met Lys Glu
                325                 330                 335

caa gaa aaa tca caa gag cct tgt ctc cct tgt gga gaa aac ctg gca     1056
Gln Glu Lys Ser Gln Glu Pro Cys Leu Pro Cys Gly Glu Asn Leu Ala
            340                 345                 350

tcc aag tac ctc gtg tgg aac tgt tgc ccc cag tgg ctg tgc gtt aag     1104
Ser Lys Tyr Leu Val Trp Asn Cys Cys Pro Gln Trp Leu Cys Val Lys
        355                 360                 365

aag gtc ctg aga act gtg atg act gac ccg ttt act gag ctg gcc atc     1152
Lys Val Leu Arg Thr Val Met Thr Asp Pro Phe Thr Glu Leu Ala Ile
    370                 375                 380

acc atc tgc atc atc atc aac act gtc ttc ttg gcc atg gag cat cac     1200
Thr Ile Cys Ile Ile Ile Asn Thr Val Phe Leu Ala Met Glu His His

385                 390                 395                 400

aag atg gag gcc agt ttt gag aag atg ttg aat ata ggg aat ttg gtt      1248
Lys Met Glu Ala Ser Phe Glu Lys Met Leu Asn Ile Gly Asn Leu Val
                405                 410                 415

ttc act agc att ttt ata gca gaa atg tgc cta aaa atc att gcg ctc      1296
Phe Thr Ser Ile Phe Ile Ala Glu Met Cys Leu Lys Ile Ile Ala Leu
            420                 425                 430

gat ccc tac cac tac ttt cgc cga ggc tgg aac att ttt gac agc att      1344
Asp Pro Tyr His Tyr Phe Arg Arg Gly Trp Asn Ile Phe Asp Ser Ile
        435                 440                 445

gtt gct ctt ctg agt ttt gca gat gta atg aac tgt gta ctt caa aag      1392
Val Ala Leu Leu Ser Phe Ala Asp Val Met Asn Cys Val Leu Gln Lys
    450                 455                 460

aga agc tgg cca ttc ttg cgt tcc ttc aga gtg ctc agg gtc ttc aag      1440
Arg Ser Trp Pro Phe Leu Arg Ser Phe Arg Val Leu Arg Val Phe Lys
465                 470                 475                 480

tta gcc aaa tcc tgg cca act ttg aac aca cta att aag ata atc ggc      1488
Leu Ala Lys Ser Trp Pro Thr Leu Asn Thr Leu Ile Lys Ile Ile Gly
                485                 490                 495

aac tct gtc gga gcc ctt gga agc ctg act gtg gtc ctg gtc att gtg      1536
Asn Ser Val Gly Ala Leu Gly Ser Leu Thr Val Val Leu Val Ile Val
            500                 505                 510

atc ttt att ttc tca gta gtt ggc atg cag ctt ttt ggc cgt agc ttc      1584
Ile Phe Ile Phe Ser Val Val Gly Met Gln Leu Phe Gly Arg Ser Phe
        515                 520                 525

aat tcc caa aag agt cca aaa ctc tgt aac ccg aca ggc ccg aca gtc      1632
Asn Ser Gln Lys Ser Pro Lys Leu Cys Asn Pro Thr Gly Pro Thr Val
    530                 535                 540

tca tgt tta cgg cac tgg cac atg ggg gat ttc tgg cac tcc ttc cta      1680
Ser Cys Leu Arg His Trp His Met Gly Asp Phe Trp His Ser Phe Leu
545                 550                 555                 560

gtg gta ttc cgc atc ctc tgc ggg gaa tgg atc gaa aat atg tgg gaa      1728
Val Val Phe Arg Ile Leu Cys Gly Glu Trp Ile Glu Asn Met Trp Glu
                565                 570                 575

tgt atg caa gaa gcg aat gca tca tca tca ttg tgt gtt att gtc ttc      1776
Cys Met Gln Glu Ala Asn Ala Ser Ser Ser Leu Cys Val Ile Val Phe
            580                 585                 590

ata ttg atc acg gtg ata gga aaa ctt gtg gtg ctc aac ctc ttc att      1824
Ile Leu Ile Thr Val Ile Gly Lys Leu Val Val Leu Asn Leu Phe Ile

595                    600                    605

gcc tta ctg ctc aat tcc ttt agc aat gag gaa aga aat gga aac tta    1872
Ala Leu Leu Leu Asn Ser Phe Ser Asn Glu Glu Arg Asn Gly Asn Leu
    610                 615                 620

gaa gga gag gcc agg aaa act aaa gtc cag tta gca ctg gat cga ttc    1920
Glu Gly Glu Ala Arg Lys Thr Lys Val Gln Leu Ala Leu Asp Arg Phe
625                 630                 635                 640

cgc cgg gct ttt tgt ttt gtg aga cac act ctt gag cat ttc tgt cac    1968
Arg Arg Ala Phe Cys Phe Val Arg His Thr Leu Glu His Phe Cys His
                645                 650                 655

aag tgg tgc agg aag caa aac tta cca cag caa aaa gag gtg gca gga    2016
Lys Trp Cys Arg Lys Gln Asn Leu Pro Gln Gln Lys Glu Val Ala Gly
            660                 665                 670

ggc tgt gct gca caa agc aaa gac atc att ccc ctg gtc atg gag atg    2064
Gly Cys Ala Ala Gln Ser Lys Asp Ile Ile Pro Leu Val Met Glu Met
        675                 680                 685

aaa agg ggc tca gag acc cag gag gag ctt ggt ata cta acc tct gta    2112
Lys Arg Gly Ser Glu Thr Gln Glu Glu Leu Gly Ile Leu Thr Ser Val
    690                 695                 700

cca aag acc ctg ggc gtc agg cat gat tgg act tgg ttg gca cca ctt    2160
Pro Lys Thr Leu Gly Val Arg His Asp Trp Thr Trp Leu Ala Pro Leu
705                 710                 715                 720

gcg gag gag gaa gat gac gtt gaa ttt tct ggt gaa gat aat gca cag    2208
Ala Glu Glu Glu Asp Asp Val Glu Phe Ser Gly Glu Asp Asn Ala Gln
                725                 730                 735

cgc atc aca caa cct gag cct gaa caa cag gcc tat gag ctc cat cag    2256
Arg Ile Thr Gln Pro Glu Pro Glu Gln Gln Ala Tyr Glu Leu His Gln
            740                 745                 750

gag aac aag aag ccc acg agc cag aga gtt caa agt gtg gaa att gac    2304
Glu Asn Lys Lys Pro Thr Ser Gln Arg Val Gln Ser Val Glu Ile Asp
        755                 760                 765

atg ttc tct gaa gat gag cct cat ctg acc ata cag gat ccc cga aag    2352
Met Phe Ser Glu Asp Glu Pro His Leu Thr Ile Gln Asp Pro Arg Lys
    770                 775                 780

aag tct gat gtt acc agt ata cta tca gaa tgt agc acc att gat ctt    2400
Lys Ser Asp Val Thr Ser Ile Leu Ser Glu Cys Ser Thr Ile Asp Leu
785                 790                 795                 800

cag gat ggc ttt gga tgg tta cct gag atg gtt ccc aaa aag caa cca    2448
Gln Asp Gly Phe Gly Trp Leu Pro Glu Met Val Pro Lys Lys Gln Pro

805                 810                 815

gag aga tgt ttg ccc aaa ggc ttt ggt tgc tgc ttt cca tgc tgt agc     2496
Glu Arg Cys Leu Pro Lys Gly Phe Gly Cys Cys Phe Pro Cys Cys Ser
            820                 825                 830

gtg gac aag aga aag cct ccc tgg gtc att tgg tgg aac ctg cgg aaa     2544
Val Asp Lys Arg Lys Pro Pro Trp Val Ile Trp Trp Asn Leu Arg Lys
        835                 840                 845

acc tgc tac caa ata gtg aaa cac agc tgg ttt gag agc ttt att atc     2592
Thr Cys Tyr Gln Ile Val Lys His Ser Trp Phe Glu Ser Phe Ile Ile
    850                 855                 860

ttt gtg att ctg ctg agc agt ggg gca ctg ata ttt gaa gat gtt cac     2640
Phe Val Ile Leu Leu Ser Ser Gly Ala Leu Ile Phe Glu Asp Val His
865                 870                 875                 880

ctt gag aac caa ccc aaa atc caa gaa tta cta aat tgt act gac att     2688
Leu Glu Asn Gln Pro Lys Ile Gln Glu Leu Leu Asn Cys Thr Asp Ile
                885                 890                 895

att ttt aca cat atc ttt atc ctg gag atg gta cta aaa tgg gta gcc     2736
Ile Phe Thr His Ile [?]he Ile Leu Glu Met Val [?]eu [?]ys Trp Val Ala
            900                 905                 910

ttc gga ttt gga aag tat ttc acc agt gcc tgg tgc tgc ctt gat ttc     2784
Phe Gly Phe Gly Lys Tyr Phe Thr Ser Ala Trp Cys Cys Leu Asp Phe
        915                 920                 925

atc att gtg att gtc tct gtg acc acc ctc att aac tta atg gaa ttg     2832
Ile Ile Val Ile Val Ser Val Thr Thr Leu Ile Asn Leu Met Glu Leu
    930                 935                 940

aag tcc ttc cgg act cta cga gca ctg agg cct ctt cgt gcg ctg tcc     2880
Lys Ser Phe Arg Thr Leu Arg Ala Leu Arg Pro Leu Arg Ala Leu Ser
945                 950                 955                 960

cag ttt gaa gga atg aag gtg gtg gtc aat gct ctc ata ggt gcc ata     2928
Gln Phe Glu Gly Met Lys Val Val Val Asn Ala Leu Ile Gly Ala Ile
                965                 970                 975

cct gcc att ctg aat gtt ttg ctt gtc tgc ctc att ttc tgg ctc gta     2976
Pro Ala Ile Leu Asn Val Leu Leu Val Cys Leu Ile Phe Trp Leu Val
            980                 985                 990

ttt tgt att ctg gga gta tac ttc ttt tct gga aaa ttt ggg aaa tgc     3024
Phe Cys Ile Leu Gly Val Tyr Phe Phe Ser Gly Lys Phe Gly Lys Cys
        995                 1000                1005

att aat gga aca gac tca gtt ata aat tat acc atc att aca aat aaa     3072
Ile Asn Gly Thr Asp Ser Val Ile Asn Tyr Thr Ile Ile Thr Asn Lys

1010                1015                1020

agt caa tgt gaa agt ggc aat ttc tct tgg atc aac cag aaa gtc aac    3120
Ser Gln Cys Glu Ser Gly Asn Phe Ser Trp Ile Asn Gln Lys Val Asn
1025                1030                1035                1040

ttt gac aat gtg gga aat gct tac ctc gct ctg ctg caa gtg gca aca    3168
Phe Asp Asn Val Gly Asn Ala Tyr Leu Ala Leu Leu Gln Val Ala Thr
                1045                1050                1055

ttt aag ggc tgg atg gat att ata tat gca gct gtt gat tcc aca gag    3216
Phe Lys Gly Trp Met Asp Ile Ile Tyr Ala Ala Val Asp Ser Thr Glu
            1060                1065                1070

aaa gaa caa cag cca gag ttt gag agc aat tca ctc ggt tac att tac    3264
Lys Glu Gln Gln Pro Glu Phe Glu Ser Asn Ser Leu Gly Tyr Ile Tyr
        1075                1080                1085

ttc gta gtc ttt atc atc ttt ggc tca ttc ttc act ctg aat ctc ttc    3312
Phe Val Val Phe Ile Ile Phe Gly Ser Phe Phe Thr Leu Asn Leu Phe
    1090                1095                1100

att ggc gtt atc att gac aac ttc aac caa cag cag aaa aag tta ggt    3360
Ile Gly Val Ile Ile Asp Asn Phe Asn Gln Gln Gln Lys Lys Leu Gly
1105                1110                1115                1120

ggc caa gac att ttt atg aca gaa gaa cag aag aaa tac tat aat gca    3408
Gly Gln Asp Ile Phe Met Thr Glu Glu Gln Lys Lys Tyr Tyr Asn Ala
                1125                1130                1135

atg aaa aaa tta gga tcc aaa aaa cct caa aaa ccc att cca cgg cct    3456
Met Lys Lys Leu Gly Ser Lys Lys Pro Gln Lys Pro Ile Pro Arg Pro
            1140                1145                1150

ctg aac aaa tgt caa ggt ctc gtg ttc gac ata gtc aca agc cag atc    3504
Leu Asn Lys Cys Gln Gly Leu Val Phe Asp Ile Val Thr Ser Gln Ile
        1155                1160                1165

ttt gac atc atc atc ata agt ctc att atc cta aac atg att agc atg    3552
Phe Asp Ile Ile Ile Ile Ser Leu Ile Ile Leu Asn Met Ile Ser Met
    1170                1175                1180

atg gct gaa tca tac aac caa ccc aaa gcc atg aaa tcc atc ctt gac    3600
Met Ala Glu Ser Tyr Asn Gln Pro Lys Ala Met Lys Ser Ile Leu Asp
1185                1190                1195                1200

cat ctc aac tgg gtc ttt gtg gtc atc ttt acg tta gaa tgt ctc atc    3648
His Leu Asn Trp Val Phe Val Val Ile Phe Thr Leu Glu Cys Leu Ile
                1205                1210                1215

aaa atc ttt gct ttg agg caa tac tac ttc acc aat ggc tgg aat tta    3696
Lys Ile Phe Ala Leu Arg Gln Tyr Tyr Phe Thr Asn Gly Trp Asn Leu

1220                    1225                    1230

ttt ga                                                                    3701
Phe

<210> 7
<211> 1233
<212> PRT
<213> Homo sapiens

<220>
<223> partial human NaN amino acid seq.

<400> 7
Ser Ile Val Ile Gly Ile Ala Ile Val Ser Tyr Ile Pro Gly Ile Thr
1               5                   10                  15

Ile Lys Leu Leu Pro Leu Arg Thr Phe Arg Val Phe Arg Ala Leu Lys
            20                  25                  30

Ala Ile Ser Val Val Ser Arg Leu Lys Val Ile Val Gly Ala Leu Leu
        35                  40                  45

Arg Ser Val Lys Lys Leu Val Asn Val Ile Ile Leu Thr Phe Phe Cys
    50                  55                  60

Leu Ser Ile Phe Ala Leu Val Gly Gln Gln Leu Phe Met Gly Ser Leu
65                  70                  75                  80

Asn Leu Lys Cys Ile Ser Arg Asp Cys Lys Asn Ile Ser Asn Pro Glu
                85                  90                  95

Ala Tyr Asp His Cys Phe Glu Lys Lys Glu Asn Ser Pro Glu Phe Lys
            100                 105                 110

Met Cys Gly Ile Trp Met Gly Asn Ser Ala Cys Ser Ile Gln Tyr Glu
        115                 120                 125

Cys Lys His Thr Lys Ile Asn Pro Asp Tyr Asn Tyr Thr Asn Phe Asp
    130                 135                 140

Asn Phe Gly Trp Ser Phe Leu Ala Met Phe Arg Leu Met Thr Gln Asp
145                 150                 155                 160

Ser Trp Glu Lys Leu Tyr Gln Gln Thr Leu Arg Thr Thr Gly Leu Tyr
                165                 170                 175

Ser Val Phe Phe Phe Ile Val Val Ile Phe Leu Gly Ser Phe Tyr Leu
            180                 185                 190

Ile Asn Leu Thr Leu Ala Val Val Thr Met Ala Tyr Glu Glu Gln Asn
        195                 200                 205

Lys Asn Val Ala Ala Glu Ile Glu Ala Lys Glu Lys Met Phe Gln Glu
    210                 215                 220

Ala Gln Gln Leu Leu Lys Glu Glu Lys Glu Ala Leu Val Ala Met Gly
225                 230                 235                 240

Ile Asp Arg Ser Ser Leu Thr Ser Leu Glu Thr Ser Tyr Phe Thr Pro
                245                 250                 255

Lys Lys Arg Lys Leu Phe Gly Asn Lys Lys Arg Lys Ser Phe Phe Leu
            260                 265                 270

Arg Glu Ser Gly Lys Asp Gln Pro Pro Gly Ser Asp Ser Asp Glu Asp
        275                 280                 285

Cys Gln Lys Lys Pro Gln Leu Leu Glu Gln Thr Lys Arg Leu Ser Gln
    290                 295                 300

Asn Leu Ser Leu Asp His Phe Asp Glu His Gly Asp Pro Leu Gln Arg
305                 310                 315                 320

Gln Arg Ala Leu Ser Ala Val Ser Ile Leu Thr Ile Thr Met Lys Glu
                325                 330                 335

Gln Glu Lys Ser Gln Glu Pro Cys Leu Pro Cys Gly Glu Asn Leu Ala
            340                 345                 350

Ser Lys Tyr Leu Val Trp Asn Cys Cys Pro Gln Trp Leu Cys Val Lys
        355                 360                 365

Lys Val Leu Arg Thr Val Met Thr Asp Pro Phe Thr Glu Leu Ala Ile
    370                 375                 380

Thr Ile Cys Ile Ile Ile Asn Thr Val Phe Leu Ala Met Glu His His
385                 390                 395                 400

Lys Met Glu Ala Ser Phe Glu Lys Met Leu Asn Ile Gly Asn Leu Val
                405                 410                 415

Phe Thr Ser Ile Phe Ile Ala Glu Met Cys Leu Lys Ile Ile Ala Leu
            420                 425                 430

Asp Pro Tyr His Tyr Phe Arg Arg Gly Trp Asn Ile Phe Asp Ser Ile
        435                 440                 445

Val Ala Leu Leu Ser Phe Ala Asp Val Met Asn Cys Val Leu Gln Lys
    450                 455                 460

Arg Ser Trp Pro Phe Leu Arg Ser Phe Arg Val Leu Arg Val Phe Lys

465                 470                 475                 480

Leu Ala Lys Ser Trp Pro Thr Leu Asn Thr Leu Ile Lys Ile Ile Gly
                485                 490                 495

Asn Ser Val Gly Ala Leu Gly Ser Leu Thr Val Val Leu Val Ile Val
            500                 505                 510

Ile Phe Ile Phe Ser Val Val Gly Met Gln Leu Phe Gly Arg Ser Phe
        515                 520                 525

Asn Ser Gln Lys Ser Pro Lys Leu Cys Asn Pro Thr Gly Pro Thr Val
    530                 535                 540

Ser Cys Leu Arg His Trp His Met Gly Asp Phe Trp His Ser Phe Leu
545                 550                 555                 560

Val Val Phe Arg Ile Leu Cys Gly Glu Trp Ile Glu Asn Met Trp Glu
                565                 570                 575

Cys Met Gln Glu Ala Asn Ala Ser Ser Ser Leu Cys Val Ile Val Phe
            580                 585                 590

Ile Leu Ile Thr Val Ile Gly Lys Leu Val Val Leu Asn Leu Phe Ile
        595                 600                 605

Ala Leu Leu Leu Asn Ser Phe Ser Asn Glu Glu Arg Asn Gly Asn Leu
    610                 615                 620

Glu Gly Glu Ala Arg Lys Thr Lys Val Gln Leu Ala Leu Asp Arg Phe
625                 630                 635                 640

Arg Arg Ala Phe Cys Phe Val Arg His Thr Leu Glu His Phe Cys His
                645                 650                 655

Lys Trp Cys Arg Lys Gln Asn Leu Pro Gln Gln Lys Glu Val Ala Gly
            660                 665                 670

Gly Cys Ala Ala Gln Ser Lys Asp Ile Ile Pro Leu Val Met Glu Met
        675                 680                 685

Lys Arg Gly Ser Glu Thr Gln Glu Glu Leu Gly Ile Leu Thr Ser Val
    690                 695                 700

Pro Lys Thr Leu Gly Val Arg His Asp Trp Thr Trp Leu Ala Pro Leu
705                 710                 715                 720

Ala Glu Glu Glu Asp Asp Val Glu Phe Ser Gly Glu Asp Asn Ala Gln
                725                 730                 735

Arg Ile Thr Gln Pro Glu Pro Glu Gln Gln Ala Tyr Glu Leu His Gln
            740                 745                 750

Glu Asn Lys Lys Pro Thr Ser Gln Arg Val Gln Ser Val Glu Ile Asp
        755                 760                 765

Met Phe Ser Glu Asp Glu Pro His Leu Thr Ile Gln Asp Pro Arg Lys
    770                 775                 780

Lys Ser Asp Val Thr Ser Ile Leu Ser Glu Cys Ser Thr Ile Asp Leu
785                 790                 795                 800

Gln Asp Gly Phe Gly Trp Leu Pro Glu Met Val Pro Lys Lys Gln Pro
                805                 810                 815

Glu Arg Cys Leu Pro Lys Gly Phe Gly Cys Cys Phe Pro Cys Cys Ser
            820                 825                 830

Val Asp Lys Arg Lys Pro Pro Trp Val Ile Trp Trp Asn Leu Arg Lys
        835                 840                 845

Thr Cys Tyr Gln Ile Val Lys His Ser Trp Phe Glu Ser Phe Ile Ile
    850                 855                 860

Phe Val Ile Leu Leu Ser Ser Gly Ala Leu Ile Phe Glu Asp Val His
865                 870                 875                 880

Leu Glu Asn Gln Pro Lys Ile Gln Glu Leu Leu Asn Cys Thr Asp Ile
                885                 890                 895

Ile Phe Thr His Ile Phe Ile Leu Glu Met Val Leu Lys Trp Val Ala
            900                 905                 910

Phe Gly Phe Gly Lys Tyr Phe Thr Ser Ala Trp Cys Cys Leu Asp Phe
        915                 920                 925

Ile Ile Val Ile Val Ser Val Thr Thr Leu Ile Asn Leu Met Glu Leu
    930                 935                 940

Lys Ser Phe Arg Thr Leu Arg Ala Leu Arg Pro Leu Arg Ala Leu Ser
945                 950                 955                 960

Gln Phe Glu Gly Met Lys Val Val Val Asn Ala Leu Ile Gly Ala Ile
                965                 970                 975

Pro Ala Ile Leu Asn Val Leu Leu Val Cys Leu Ile Phe Trp Leu Val
            980                 985                 990

Phe Cys Ile Leu Gly Val Tyr Phe Phe Ser Gly Lys Phe Gly Lys Cys
        995                 1000                1005

Ile Asn Gly Thr Asp Ser Val Ile Asn Tyr Thr Ile Ile Thr Asn Lys
    1010                1015                1020

Ser Gln Cys Glu Ser Gly Asn Phe Ser Trp Ile Asn Gln Lys Val Asn
1025                1030                1035                1040

Phe Asp Asn Val Gly Asn Ala Tyr Leu Ala Leu Leu Gln Val Ala Thr
                1045                1050                1055

Phe Lys Gly Trp Met Asp Ile Ile Tyr Ala Ala Val Asp Ser Thr Glu
            1060                1065                1070

Lys Glu Gln Gln Pro Glu Phe Glu Ser Asn Ser Leu Gly Tyr Ile Tyr
        1075                1080                1085

Phe Val Val Phe Ile Ile Phe Gly Ser Phe Phe Thr Leu Asn Leu Phe
    1090                1095                1100

Ile Gly Val Ile Ile Asp Asn Phe Asn Gln Gln Gln Lys Lys Leu Gly
1105                1110                1115                1120

Gly Gln Asp Ile Phe Met Thr Glu Glu Gln Lys Lys Tyr Tyr Asn Ala
                1125                1130                1135

Met Lys Lys Leu Gly Ser Lys Lys Pro Gln Lys Pro Ile Pro Arg Pro
            1140                1145                1150

Leu Asn Lys Cys Gln Gly Leu Val Phe Asp Ile Val Thr Ser Gln Ile
        1155                1160                1165

Phe Asp Ile Ile Ile Ile Ser Leu Ile Ile Leu Asn Met Ile Ser Met
    1170                1175                1180

Met Ala Glu Ser Tyr Asn Gln Pro Lys Ala Met Lys Ser Ile Leu Asp
1185                1190                1195                1200

His Leu Asn Trp Val Phe Val Val Ile Phe Thr Leu Glu Cys Leu Ile
                1205                1210                1215

Lys Ile Phe Ala Leu Arg Gln Tyr Tyr Phe Thr Asn Gly Trp Asn Leu
            1220                1225                1230

Phe

<210> 8
<211> 1243
<212> PRT
<213> Homo sapiens

<220>
<223> partial human NaN amino acid seq.

<400> 8

Ser Ile Val Ile Gly Ile Ala Ile Val Ser Tyr Ile Pro Gly Ile Thr
1               5                   10                  15

Ile Lys Leu Leu Pro Leu Arg Thr Phe Arg Val Phe Arg Ala Leu Lys
            20                  25                  30

Ala Ile Ser Val Val Ser Arg Leu Lys Val Ile Val Gly Ala Leu Leu
        35                  40                  45

Arg Ser Val Lys Lys Leu Val Asn Val Ile Ile Leu Thr Phe Phe Cys
    50                  55                  60

Leu Ser Ile Phe Ala Leu Val Gly Gln Gln Leu Phe Met Gly Ser Leu
65                  70                  75                  80

Asn Leu Lys Cys Ile Ser Arg Asp Cys Lys Asn Ile Ser Asn Pro Glu
                85                  90                  95

Ala Tyr Asp His Cys Phe Glu Lys Lys Glu Asn Ser Pro Glu Phe Lys
            100                 105                 110

Met Cys Gly Ile Trp Met Gly Asn Ser Ala Cys Ser Ile Gln Tyr Glu
        115                 120                 125

Cys Lys His Thr Lys Ile Asn Pro Asp Tyr Asn Tyr Thr Asn Phe Asp
    130                 135                 140

Asn Phe Gly Trp Ser Phe Leu Ala Met Phe Arg Leu Met Thr Gln Asp
145                 150                 155                 160

Ser Trp Glu Lys Leu Tyr Gln Gln Thr Leu Arg Thr Thr Gly Leu Tyr
                165                 170                 175

Ser Val Phe Phe Phe Ile Val Val Ile Phe Leu Gly Ser Phe Tyr Leu
            180                 185                 190

Ile Asn Leu Thr Leu Ala Val Val Thr Met Ala Tyr Glu Glu Gln Asn
        195                 200                 205

Lys Asn Val Ala Ala Glu Ile Glu Ala Lys Glu Lys Met Phe Gln Glu
    210                 215                 220

Ala Gln Gln Leu Leu Lys Glu Glu Lys Glu Ala Leu Val Ala Met Gly
225                 230                 235                 240

Ile Asp Arg Ser Ser Leu Thr Ser Leu Glu Thr Ser Tyr Phe Thr Pro
                245                 250                 255

Lys Lys Arg Lys Leu Phe Gly Asn Lys Lys Arg Lys Ser Phe Phe Leu
            260                 265                 270

Arg Glu Ser Gly Lys Asp Gln Pro Pro Gly Ser Asp Ser Asp Glu Asp
        275                 280                 285

Cys Gln Lys Lys Pro Gln Leu Leu Glu Gln Thr Lys Arg Leu Ser Gln
    290                 295                 300

Asn Leu Ser Leu Asp His Phe Asp Glu His Gly Asp Pro Leu Gln Arg
305                 310                 315                 320

Gln Arg Ala Leu Ser Ala Val Ser Ile Leu Thr Ile Thr Met Lys Glu
                325                 330                 335

Gln Glu Lys Ser Gln Glu Pro Cys Leu Pro Cys Gly Glu Asn Leu Ala
            340                 345                 350

Ser Lys Tyr Leu Val Trp Asn Cys Cys Pro Gln Trp Leu Cys Val Lys
        355                 360                 365

Lys Val Leu Arg Thr Val Met Thr Asp Pro Phe Thr Glu Leu Ala Ile
    370                 375                 380

Thr Ile Cys Ile Ile Ile Asn Thr Val Phe Leu Ala Met Glu His His
385                 390                 395                 400

Lys Met Glu Ala Ser Phe Glu Lys Met Leu Asn Ile Gly Asn Leu Val
                405                 410                 415

Phe Thr Ser Ile Phe Ile Ala Glu Met Cys Leu Lys Ile Ile Ala Leu
            420                 425                 430

Asp Pro Tyr His Tyr Phe Arg Arg Gly Trp Asn Ile Phe Asp Ser Ile
        435                 440                 445

Val Ala Leu Leu Ser Phe Ala Asp Val Met Asn Cys Val Leu Gln Lys
    450                 455                 460

Arg Ser Trp Pro Phe Leu Arg Ser Phe Arg Val Leu Arg Val Phe Lys
465                 470                 475                 480

Leu Ala Lys Ser Trp Pro Thr Leu Asn Thr Leu Ile Lys Ile Ile Gly
                485                 490                 495

Asn Ser Val Gly Ala Leu Gly Ser Leu Thr Val Val Leu Val Ile Val
            500                 505                 510

Ile Phe Ile Phe Ser Val Val Gly Met Gln Leu Phe Gly Arg Ser Phe
        515                 520                 525

Asn Ser Gln Lys Ser Pro Lys Leu Cys Asn Pro Thr Gly Pro Thr Val
    530                 535                 540

Ser Cys Leu Arg His Trp His Met Gly Asp Phe Trp His Ser Phe Leu

545                 550                 555                 560

Val Val Phe Arg Ile Leu Cys Gly Glu Trp Ile Glu Asn Met Trp Glu
                565                 570                 575

Cys Met Gln Glu Ala Asn Ala Ser Ser Ser Leu Cys Val Ile Val Phe
            580                 585                 590

Ile Leu Ile Thr Val Ile Gly Lys Leu Val Val Leu Asn Leu Phe Ile
        595                 600                 605

Ala Leu Leu Leu Asn Ser Phe Ser Asn Glu Glu Arg Asn Gly Asn Leu
    610                 615                 620

Glu Gly Glu Ala Arg Lys Thr Lys Val Gln Leu Ala Leu Asp Arg Phe
625                 630                 635                 640

Arg Arg Ala Phe Cys Phe Val Arg His Thr Leu Glu His Phe Cys His
                645                 650                 655

Lys Trp Cys Arg Lys Gln Asn Leu Pro Gln Gln Lys Glu Val Ala Gly
            660                 665                 670

Gly Cys Ala Ala Gln Ser Lys Asp Ile Ile Pro Leu Val Met Glu Met
        675                 680                 685

Lys Arg Gly Ser Glu Thr Gln Glu Glu Leu Gly Ile Leu Thr Ser Val
    690                 695                 700

Pro Lys Thr Leu Gly Val Arg His Asp Trp Thr Trp Leu Ala Pro Leu
705                 710                 715                 720

Ala Glu Glu Glu Asp Asp Val Glu Phe Ser Gly Glu Asp Asn Ala Gln
                725                 730                 735

Arg Ile Thr Gln Pro Glu Pro Glu Gln Gln Ala Tyr Glu Leu His Gln
            740                 745                 750

Glu Asn Lys Lys Pro Thr Ser Gln Arg Val Gln Ser Val Glu Ile Asp
        755                 760                 765

Met Phe Ser Glu Asp Glu Pro His Leu Thr Ile Gln Asp Pro Arg Lys
    770                 775                 780

Lys Ser Asp Val Thr Ser Ile Leu Ser Glu Cys Ser Thr Ile Asp Leu
785                 790                 795                 800

Gln Asp Gly Phe Gly Trp Leu Pro Glu Met Val Pro Lys Lys Gln Pro
                805                 810                 815

Glu Arg Cys Leu Pro Lys Gly Phe Gly Cys Cys Phe Pro Cys Cys Ser
            820                 825                 830

Val Asp Lys Arg Lys Pro Pro Trp Val Ile Trp Trp Asn Leu Arg Lys
        835                 840                 845

Thr Cys Tyr Gln Ile Val Lys His Ser Trp Phe Glu Ser Phe Ile Ile
    850                 855                 860

Phe Val Ile Leu Leu Ser Ser Gly Ala Leu Ile Phe Glu Asp Val His
865                 870                 875                 880

Leu Glu Asn Gln Pro Lys Ile Gln Glu Leu Leu Asn Cys Thr Asp Ile
                885                 890                 895

Ile Phe Thr His Ile Phe Ile Leu Glu Met Val Leu Lys Trp Val Ala
            900                 905                 910

Phe Gly Phe Gly Lys Tyr Phe Thr Ser Ala Trp Cys Cys Leu Asp Phe
        915                 920                 925

Ile Ile Val Ile Val Ser Val Thr Thr Leu Ile Asn Leu Met Glu Leu
    930                 935                 940

Lys Ser Phe Arg Thr Leu Arg Ala Leu Arg Pro Leu Arg Ala Leu Ser
945                 950                 955                 960

Gln Phe Glu Gly Met Lys Val Val Val Asn Ala Leu Ile Gly Ala Ile
                965                 970                 975

Pro Ala Ile Leu Asn Val Leu Leu Val Cys Leu Ile Phe Trp Leu Val
            980                 985                 990

Phe Cys Ile Leu Gly Val Tyr Phe Phe Ser Gly Lys Phe Gly Lys Cys
        995                 1000                1005

Ile Asn Gly Thr Asp Ser Val Ile Asn Tyr Thr Ile Ile Thr Asn Lys
    1010                1015                1020

Ser Gln Cys Glu Ser Gly Asn Phe Ser Trp Ile Asn Gln Lys Val Asn
1025                1030                1035                1040

Phe Asp Asn Val Gly Asn Ala Tyr Leu Ala Leu Leu Gln Val Ala Thr
                1045                1050                1055

Phe Lys Gly Trp Met Asp Ile Ile Tyr Ala Ala Val Asp Ser Thr Glu
            1060                1065                1070

Lys Glu Gln Gln Pro Glu Phe Glu Ser Asn Ser Leu Gly Tyr Ile Tyr
        1075                1080                1085

Phe Val Val Phe Ile Ile Phe Gly Ser Phe Phe Thr Leu Asn Leu Phe
    1090                1095                1100

Ile Gly Val Ile Ile Asp Asn Phe Asn Gln Gln Gln Lys Lys Leu Gly
1105                1110                1115                1120

Gly Gln Asp Ile Phe Met Thr Glu Glu Gln Lys Lys Tyr Tyr Asn Ala
                1125                1130                1135

Met Lys Lys Leu Gly Ser Lys Lys Pro Gln Lys Pro Ile Pro Arg Pro
            1140                1145                1150

Leu Asn Lys Cys Gln Gly Leu Val Phe Asp Ile Val Thr Ser Gln Ile
        1155                1160                1165

Phe Asp Ile Ile Ile Ile Ser Leu Ile Ile Leu Asn Met Ile Ser Met
    1170                1175                1180

Met Ala Glu Ser Tyr Asn Gln Pro Lys Ala Met Lys Ser Ile Leu Asp
1185                1190                1195                1200

His Leu Asn Trp Val Phe Val Val Ile Phe Thr Leu Glu Cys Leu Ile
                1205                1210                1215

Lys Ile Phe Ala Leu Arg Gln Tyr Tyr Phe Thr Asn Gly Trp Asn Leu
            1220                1225                1230

Phe Asp Cys Val Val Val Leu Leu Ser Ile Val
        1235                1240

<210> 9
<211> 20
<212> DNA
<213> Artificial Sequence

<220>
<221> variation
<222> (6)
<223> r = a or g

<220>
<223> Description of Artificial Sequence:  rat NaN
      forward primer no. 1

<400> 9
gacccrtgga attggttgga                                                20

<210> 10
<211> 20
<212> DNA
<213> Artificial Sequence

<220>
<223> Description of Artificial Sequence: rat NaN
      forward primer no. 2

<400> 10
aatccctgga attggttgga                                                    20


<210> 11
<211> 20
<212> DNA
<213> Artificial Sequence

<220>
<223> Description of Artificial Sequence: rat NaN
      forward primer no. 3

<400> 11
gacccgtgga actggttaga                                                    20


<210> 12
<211> 20
<212> DNA
<213> Artificial Sequence

<220>
<223> Description of Artificial Sequence: rat NaN
      forward primer no. 4

<400> 12
gatctttgga actggcttga                                                    20


<210> 13
<211> 21
<212> DNA
<213> Artificial Sequence

<220>
<223> Description of Artificial Sequence: rat Nan
      forward primer no. 5

<400> 13
aacatagtgc tggagttcag g                                                  21


<210> 14
<211> 21
<212> DNA
<213> Artificial Sequence

<220>
<223> Description of Artificial Sequence:  rat NaN
      forward primer no. 6

<400> 14
gtggcctttg gattccggag g                                                21


<210> 15
<211> 24
<212> DNA
<213> Artificial Sequence

<220>
<223> Description of Artificial Sequence:  rat NaN
      reverse primer no. 1

<400> 15
caagaaggcc cagctgaagg tgtc                                             24


<210> 16
<211> 24
<212> DNA
<213> Artificial Sequence

<220>
<223> Description of Artificial Sequence:  rat NaN
      reverse primer no. 2

<400> 16
gaggaatgcc cacgcaaagg aatc                                             24


<210> 17
<211> 24
<212> DNA
<213> Artificial Sequence

<220>
<223> Description of Artificial Sequence:  rat NaN
      reverse primer no. 3

<400> 17
aagaagggac cagccaaagt tgtc                                             24


<210> 18
<211> 20
<212> DNA
<213> Artificial Sequence

<220>
<221> variation
<222> (3), (9), (12), (13)
<223> y = c or t; r = a or g; n = a or c or g or t; w = a or t

<220>
<223> Description of Artificial Sequence: rat NaN
      reverse primer no. 4

<400> 18
acytccatrc anwcccacat                                                    20

<210> 19
<211> 20
<212> DNA
<213> Artificial Sequence

<220>
<221> variation
<222> (3), (6), (9), (15)
<223> r = a or g; n = a or c or g or t

<220>
<223> Description of Artificial Sequence: rat NaN
      reverse primer no. 5

<400> 19
agraartcna gccarcacca                                                    20

<210> 20
<211> 19
<212> DNA
<213> Artificial Sequence

<220>
<223> Description of Artificial Sequence: rat NaN
      reverse primer no. 6

<400> 20
tctgctgccg agccaggta                                                     19

<210> 21
<211> 20
<212> DNA
<213> Artificial Sequence

<220>
<223> Description of Artificial Sequence: rat NaN

reverse primer no. 7

<400> 21
ctgagataac tgaaatcgcc                                                    20


<210> 22
<211> 27
<212> DNA
<213> Artificial Sequence

<220>
<223> Description of Artificial Sequence: primer,
      Marathon AP-1

<400> 22
ccatcctaat acgactcact atagggc                                            27


<210> 23
<211> 23
<212> DNA
<213> Artificial Sequence

<220>
<223> Description of Artificial Sequence: primer,
      Marathon AP-2

<400> 23
actcactata gggctcgagc ggc                                                23


<210> 24
<211> 23
<212> DNA
<213> Artificial Sequence

<220>
<223> Description of Artificial Sequence: mouse NaN
      forward primer

<400> 24
ccctgctgcg ctcggtgaag aag                                                23


<210> 25
<211> 20
<212> DNA
<213> Artificial Sequence

<220>
<223> Description of Artificial Sequence: mouse NaN

reverse primer

<400> 25
gacaaagtag atcccagagg                                                    20


<210> 26
<211> 17
<212> DNA
<213> Artificial Sequence

<220>
<223> Description of Artificial Sequence:  human NaN
      forward primer

<400> 26
ctcagtagtt ggcatgc                                                       17


<210> 27
<211> 24
<212> DNA
<213> Artificial Sequence

<220>
<223> Description of Artificial Sequence:  human NaN
      reverse primer

<400> 27
ggaaagaagc acgaccacac agtc                                               24


<210> 28
<211> 94
<212> PRT
<213> Rattus norvegicus

<220>
<223> C-terminal truncated rat NaN

<400> 28
Ala Ala Gly Gln Ala Met Arg Lys Gln Gly Asp Ile Leu Gly Pro Asn
  1               5                  10                  15

Ile His Gln Phe Ser Gln Ser Ser Glu Thr Pro Phe Leu Gly Cys Pro
             20                  25                  30

Gln Gln Arg Thr Cys Val Ser Phe Val Arg Pro Gln Arg Val Leu Arg
         35                  40                  45

Val Pro Trp Phe Pro Ala Trp Arg Thr Val Thr Phe Leu Ser Arg Pro
     50                  55                  60

Arg Ser Ser Glu Ser Ser Ala Trp Leu Gly Leu Val Glu Ser Ser Gly
65                  70                  75                  80

Trp Ser Gly Leu Pro Gly Glu Ser Gly Pro Ser Ser Leu Leu
                85                  90

<210> 29
<211> 211
<212> DNA
<213> Mus musculus

<400> 29
agtttaatgt tgagtgaatt gtggtggtga tttcccactt gaggcctttg tgttaaagcc 60

caatgtgtgt gtgtgtgtgt gtgtgtgtgt gtgtgtgtgt gtgtgtgtgt gtgtgtggtt 120

gggggtggt ggcagagtct ggtattggta aggtgagagc aatcccagaa cgtccacctg 180

ctcttccatt ttattaatca ggcaggcctc t                                211

<210> 30
<211> 242
<212> DNA
<213> Mus musculus

<220>
<221> unsure
<222> (132), (159), (178), (184), (203), (234)
<223> n = a or c or g or t

<400> 30
gtaagccact ggctcttaac taaaatgctc gttggcatta gaacatttct gagctggggt 60

ggtggtggtg gtggtggtgg tggtggtggt ggtggtggtg gtggtggtgg tgatggtggt 120

ggtggaggtg gnggtggagg tggtggctgt ggtggtggng gtggtggtgg tggtggangt 180

ggangtggtg gcgtggtggt ggnggtggtg gtggaggtgg tggctgtggt ggtngtggtg 240

gc                                                                242

<210> 31
<211> 200
<212> DNA
<213> Mus musculus

<400> 31
tgtgcatgct tgattcccag ctcctatggt ctgattactc ggtccttagg agcaaggcca 60

gactgtccac cctgacacac acacacacac acacacacac acacacacac acacacacac 120

acagtgtaga gaattacctc attcttggag tttctctgga aaaggaatgt ctcaaagcca 180

agttcacaga gcaacagctg 200

<210> 32
<211> 181
<212> DNA
<213> Mus musculus

<400> 32
tgttagaaac tctaagacaa tgaagcacca tgctggaaat aagagcacaa actctttctt 60

catgcattac ccactgcttg tgctttcacc ttagtgctcg tgctctctct ttctctctct 120

ctctctctct ctctctctct ctctctctct ctctctctct ctctctcttt tttttttttt 180

t 181

<210> 33
<211> 128
<212> DNA
<213> Mus musculus

<400> 33
cacacacaca cacacacaca cacacacaca cacacacaca gagaaacact gtcgcagtca 60

tacatataaa gataaataca tcttaaaaaa agaaccatgt gattgagtta taaaatattc 120

caacttat 128

<210> 34
<211> 200
<212> DNA
<213> Mus musculus

<400> 34
aggtcatttc ctctgcagtg tgcttggcag gaaaaacttc ctggctattc aagtcagtgc 60

cctgcttgat catccatgta tcacacacac acaaaacaaa caaacaaaca aacaaaaccc 120

tggggaagaa ggaagaggtt aagcacatag gcagagagca gccaggctga ctcagagcaa 180

acacctgatc attcttccat 200

<210> 35

<211> 158
<212> DNA
<213> Mus musculus

<220>
<221> unsure
<222> (118), (125)
<223> n= a or c or g or t

<400> 35
   gtgctgggat caaaggcgtg cgccgccacc acgcccggcc cctttttatg tttcaaattt 60
   acttttatca tgtgcacgtg tgtgggtgcg tgcatgtgtg tgcgtgcgtg tgcgtgtgng 120
   tgtgngtgtg tgtgtgtgtg tgtgtgtgtg tgtgtgtg 158


<210> 36
<211> 113
<212> DNA
<213> Mus musculus

<400> 36
cacacacaca cacacacaca cacacacaca cacacacaca cacacacaca cacacacttg 60

catctttgag ttaattggat aggctgagtc ttacaccgga atcatactgt tgc 113


<210> 37
<211> 200
<212> DNA
<213> Mus musculus

<400> 37
ccaatgagag actcttgtct caaaaaagcc atggtgtcca gatcctgagg aataacacct 60

aagaatgtgc tctggcctga aaacacacac acacacacac acacacacac acacacacac 120

agttttattt atttatttaa aaaaatatgt ctctaggcat tgctgaaatg tctcctacag 180

gattaagtca accagagcca 200


<210> 38
<211> 7
<212> PRT
<213> Artificial Sequence

<220>
<223> Description of Artificial Sequence: protein seq.
      basis for rat NaN reverse primer no. 4

<400> 38
Met Trp Val Cys Met Glu Val
1               5


<210> 39
<211> 7
<212> PRT
<213> Artificial Sequence

<220>
<223> Description of Artificial Sequence:  protein seq.
      basis for rat NaN reverse primer no. 4

<400> 39
Met Trp Asp Cys Met Glu Val
1               5


<210> 40
<211> 20
<212> PRT
<213> Artificial Sequence

<220>
<223> Description of Artificial Sequence:  a.a. seq.
      used to derive epitope for polyclonal antibody

<400> 40
Cys Gly Pro Asn Pro Ala Ser Asn Lys Asp Cys Phe Glu Lys Glu Lys
1               5                   10                  15

Asp Ser Glu Asp
            20